# EUROPEAN PATENT APPLICATION

(11) **EP 4 491 131 A1**
(43) Date of publication of application: **15.01.2025**
(21) Application number: 22930988.5
(22) Date of filing: 04.10.2022
(51) Int. Cl.: A61B 10/00, A61B 5/00, G16H 40/63, A61B 5/16

(54) **INFORMATION PROCESSING DEVICE, INFORMATION PROCESSING SYSTEM, ODOR GENERATION SYSTEM, AND PROGRAM**

(30) Priority: 11.03.2022 JP 2022037978
(71) Applicant: Sony Group Corporation, Tokyo 108-0075 (JP)
(72) Inventor: TAKAKI, Kazutaka, Tokyo 108-0075 (JP); FUJITA,Shuji, Tokyo 108-0075 (JP); INOUE, Yukito, Tokyo 108-0075 (JP); MITSUOKA, Sayaka, Tokyo 108-0075 (JP); TAKAHASHI, Kazuma, Tokyo 108-0075 (JP); SAMUKAWA, Tsunetoshi, Tokyo 108-0075 (JP)
(74) Representative: D Young & Co LLP
(86) International application number: PCT/JP2022/037168
(87) International publication number: WO 2023/171011

(57) **Abstract**

Provided is a technology for automatically conducting a test.

In the present technology, provided is an information processing device including a processing unit that identifies information regarding an odor component to be released and controls release of the odor component on the basis of information associated with a user. Further provided is an information processing system including an information processing device including a processing unit that identifies information regarding an odor component to be released and controls release of the odor component on the basis of information associated with a user, and a display device that displays the information regarding the odor component to be released.

## Description

### TECHNICAL FIELD

The present technology relates to an information processing device, an information processing system, an odor generation system, and a program.

### BACKGROUND ART

There has been proposed a technology for presenting, when conducting olfactometry or the like, an odor to a user by supplying air to an odor holder that holds the odor to release the odor along with the flow of the air. When the olfactory sense decreases, eating habits and safety are adversely affected, such as a decrease in ability to identify rotten food or a decrease in ability to notice gas leakage or smoke, leading to a decrease in Quality of Life (QOL). Furthermore, it has been recently known that a specific neurodegenerative disease causes olfactory dysfunction, prior to a decline in cognitive function.

Here, typical examples of a kit used to conduct olfactometry include a kit referred to as "T&T olfactometer" and a kit referred to as "OSIT-J". The T&T olfactometer is also referred to as standard olfactory acuity test in Japan, and determines an olfactory function of a subject by putting odor paper into a bottle containing various types of liquids (multiple concentrations × multiple types of scents) to impregnate the odor paper with odors and causing the subject to sniff the odor paper. On the other hand, the OSIT-J is also referred to as odor stick, and determines the olfactory function of the subject by rubbing multiple odor sticks with paper for wrapping powdered medicine and causing the subject to sniff the paper for wrapping powdered medicine. Moreover, there is a so-called "Alinamin test" where Alinamin (registered trademark) is intravenously injected to make a determination of anosmia.

Furthermore, for example, Patent Literature 1 discloses an olfactory testing device including an odor presentation button tool and an odor identification display, in which the odor identification display includes a board having a plurality of recesses formed on a surface of the board and an information display board detachably fitted to each of the plurality of recesses of the board, at least one of a picture or a character of an object that generates an odor presented by the odor presentation button tool described above is displayed on one of the plurality of information display boards, and at least one of a picture or a character of an object that does not generate an odor presented by the odor presentation button tool described above is displayed on the remaining information display board.

### CITATION LIST

### PATENT DOCUMENT

Patent Document 1: Japanese Utility Model Registration No. 3098464

### SUMMARY OF THE INVENTION

### PROBLEMS TO BE SOLVED BY THE INVENTION

There, however, has been a lack of a technology to automatically conduct an odor-related test, and there is a fact that a technology with less complexity and the like in the test and less burden on a user is desired.

It is therefore a main object of the present technology to provide a technology that enables automatic conduction of a test.

### SOLUTIONS TO PROBLEMS

In the present technology, first provided is an information processing device including a processing unit that identifies information regarding an odor component to be released and controls release of the odor component on the basis of information associated with a user.

Furthermore, in the present technology, further provided is an information processing system including an information processing device including a processing unit that identifies information regarding an odor component to be released and controls release of the odor component on the basis of information associated with a user, and a display device that displays the information regarding the odor component to be released.

Moreover, in the present technology, further provided is an odor generation system including an odor generation device including a cartridge holding portion that holds a cartridge holding an odor component, and a releasing portion that releases the odor component to outside, and an information processing device including a processing unit that identifies information regarding an odor component to be released and controls release of the odor component on the basis of information associated with a user.

In addition, further provided is a program for executing a processing function, the processing function including identifying information regarding an odor component to be released and controlling release of the odor component on the basis of information associated with a user.

### BRIEF DESCRIPTION OF DRAWINGS

Fig. 1 is a block diagram illustrating an embodiment of an information processing device 3 according to a first embodiment.
Figs. 2A and 2B are diagrams for describing information displayed on a user interface unit 33.
Figs. 3A and 3B are diagrams for describing information displayed on the user interface unit 33.
Fig. 4 is a diagram for describing information displayed on the user interface unit 33.

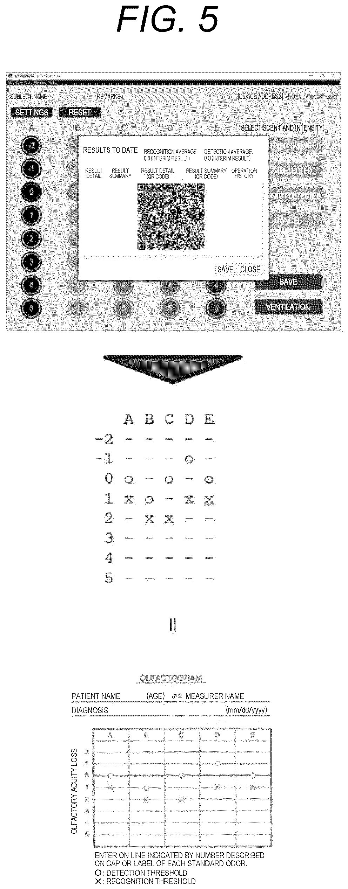

Fig. 5 is a diagram for describing information output from an output unit 34.
Fig. 6 is a flowchart illustrating an operation example of the information processing device 3.
Fig. 7 is a diagram for describing a specific example of the information processing device 3.
Fig. 8 is a diagram for describing a specific example of the information processing device 3.
Fig. 9 is a diagram for describing a specific example of the information processing device 3.
Fig. 10 is a diagram for describing a specific example of the information processing device 3.
Fig. 11 is a diagram for describing a specific example of the information processing device 3.
Fig. 12 is a diagram for describing a specific example of the information processing device 3.
Fig. 13 is a block diagram illustrating an embodiment of an information processing system 200 according to a second embodiment.
Fig. 14 is a diagram illustrating an embodiment of an odor generation system 100 according to a third embodiment.
Fig. 15 is a six-sided view of an embodiment of a cartridge 10.
Fig. 16 is a cross-sectional view taken along line P-P of the cartridge 10.
Fig. 17 is a cross-sectional view taken along line Q-Q of the cartridge 10.
Fig. 18 is a perspective view of an example of an embodiment of a movable portion 12.
Fig. 19 is a perspective view of an example of an embodiment of a cartridge unit 20.
Fig. 20 is a perspective view of the cartridge unit 20, illustrating a surface (back surface in Fig. 19) of a retaining portion 205 without a plurality of the cartridges 10.
Fig. 21 is a flowchart illustrating an operation example of the odor generation system 100.
Fig. 22 is a diagram illustrating an embodiment of the odor generation system 100 according to a fourth embodiment.
Fig. 23 is a diagram illustrating an embodiment of the user interface unit 33.
Fig. 24 is a diagram for describing a state where a non-volatile memory is incorporated in the cartridge 10 or the cartridge unit 20.
Fig. 25 is a diagram illustrating transfer of data regarding an olfactory sense.
Figs. 26A to 26C are diagrams each illustrating an example of an ASCII art graph.
Fig. 27 is a diagram illustrating a neurodegenerative disease prediction model based analysis of olfactory sense data.
Fig. 28 is a diagram illustrating an example of an embodiment of a UI of the user interface unit 33.
Fig. 29 is a diagram illustrating transition of screens displayed on a tablet terminal 301.
Figs. 30A and 30B are diagrams each illustrating an example of an embodiment of a measurement preparation screen.
Fig. 31 is a diagram for describing behavior when selecting a measurer.
Fig. 32 is a diagram illustrating an example of the embodiment of the measurement preparation screen.
Fig. 33 is a diagram illustrating an example of an embodiment of a free measurement screen.
Figs. 34A and 34B are diagrams each illustrating an example of the embodiment of the free measurement screen.
Figs. 35A and 35B are diagrams each illustrating an example of the embodiment of the free measurement screen.
Figs. 36A and 36B are diagrams each illustrating an example of the embodiment of the free measurement screen.
Figs. 37A and 37B are diagrams each illustrating an example of the embodiment of the free measurement screen.
Figs. 38A and 38B are diagrams each illustrating an example of the embodiment of the free measurement screen.
Figs. 39A and 39B are diagrams each illustrating an example of the embodiment of the free measurement screen.
Fig. 40 is a diagram illustrating an example of the embodiment of the free measurement screen.
Fig. 41 is a diagram illustrating an example of an embodiment of a result display screen.
Fig. 42 is a diagram illustrating a state where a CSV output code is displayed as a two-dimensional matrix barcode.
Figs. 43A to 43D are diagrams each illustrating an example of an embodiment of a corresponding one of four measurement result displays including a measurement result display, an olfactogram display, a patient answer display, and a list display.
Fig. 44 is a diagram illustrating an example of an embodiment of a result display based on an olfactogram.
Fig. 45 is a diagram illustrating an example of an embodiment of a result display based on a subject answer.
Fig. 46 is a diagram illustrating an example of an embodiment of a discrimination result display.
Fig. 47 is a diagram illustrating an example of an embodiment of a list display.
Fig. 48 is a diagram illustrating a state where a result of the list display is printed.
Figs. 49A and 49B are diagrams each illustrating an example of an embodiment of a screen after the end of measurement.
Fig. 50 is a diagram illustrating an example of the embodiment of the screen after the end of measurement.
Fig. 51 is a diagram illustrating screen transition after the end of measurement.
Figs. 52A to 52C are diagrams each illustrating an example of an embodiment of a history screen.
Figs. 53A and 53B are diagrams each illustrating an example of the embodiment of the history screen.
Figs. 54A and 54B are diagrams each illustrating an example of the embodiment of the history screen.
Fig. 55 is a diagram illustrating an example of an embodiment of a comparison display screen.
Fig. 56 is a diagram illustrating an example of the embodiment of the comparison display screen.
Fig. 57 is a diagram illustrating an example of the embodiment of the comparison display screen.
Figs. 58A to 58C are diagrams each illustrating an example of the embodiment of the comparison display screen.
Fig. 59 is a diagram illustrating an example of an embodiment of an odorant selection screen in a detection threshold test screen.
Fig. 60 is a diagram illustrating an example of an embodiment of an odor presentation screen in the detection threshold test screen.
Fig. 61 is a diagram illustrating an example of an embodiment of a detection answer input screen in the detection threshold test screen.
Fig. 62 is a diagram illustrating an example of an embodiment of a result reflection screen in the detection threshold test screen.
Fig. 63 is a diagram illustrating an example of an embodiment of an odorant selection screen in a recognition threshold test screen.
Fig. 64 is a diagram illustrating an example of an embodiment of an odor presentation screen in the recognition threshold test screen.
Fig. 65 is a diagram illustrating an example of an embodiment of an olfactory term table display screen in the recognition threshold test screen.
Fig. 66 is a diagram illustrating an example of the embodiment of the olfactory term table display screen in the recognition threshold test screen.
Fig. 67 is a diagram illustrating an example of an embodiment of an answer confirmation screen in the recognition threshold test screen.
Fig. 68 is a diagram illustrating an example of an embodiment of a result reflection screen in the recognition threshold test screen.
Fig. 69 is a diagram illustrating an example of an embodiment when a result screen of a detection average and a recognition average is displayed as an olfactogram.
Fig. 70 is a diagram illustrating an example of an embodiment when the result screen of the detection average and the recognition average is displayed as a patient answer.
Fig. 71 is a diagram illustrating an example of an embodiment of a three-option detection measurement screen.
Fig. 72 is a diagram illustrating an example of the embodiment of the three-option detection measurement screen.
Fig. 73 is a diagram illustrating an example of the embodiment of the three-option detection measurement screen.
Fig. 74 is a diagram illustrating an example of the embodiment of the three-option detection measurement screen.
Fig. 75 is a diagram illustrating an example of the embodiment of the three-option detection measurement screen.
Fig. 76 is a diagram illustrating an example of the embodiment of the three-option detection measurement screen.
Fig. 77 is a diagram illustrating an example of the embodiment of the three-option detection measurement screen.
Fig. 78 is a diagram illustrating an example of the embodiment of the three-option detection measurement screen.
Fig. 79 is a diagram illustrating an example of the embodiment of the three-option detection measurement screen.
Fig. 80 is a diagram illustrating an example of the embodiment of the three-option detection measurement screen.
Fig. 81 is a diagram illustrating an example of the embodiment of the three-option detection measurement screen.
Fig. 82 is a diagram illustrating an example of the embodiment of the three-option detection measurement screen.
Fig. 83 is a diagram illustrating an example of the embodiment of the three-option detection measurement screen.
Fig. 84 is a diagram illustrating an example of the embodiment of the three-option detection measurement screen.
Fig. 85 is a diagram for describing the olfactory term table.
Figs. 86A to 86C are diagrams for describing a discrimination test measurement flow.
Fig. 87 is a diagram illustrating an example of an embodiment of a discrimination test measurement screen.
Fig. 88 is a diagram illustrating an example of the embodiment of the discrimination test measurement screen.
Fig. 89 is a diagram illustrating an example of the embodiment of the discrimination test measurement screen.
Fig. 90 is a diagram illustrating an example of the embodiment of the discrimination test measurement screen.
Fig. 91 is a diagram illustrating an example of the embodiment of the discrimination test measurement screen.
Fig. 92 is a diagram illustrating an example of the embodiment of the discrimination test measurement screen.
Fig. 93 is a diagram illustrating an example of the embodiment of the discrimination test measurement screen.
Fig. 94 is a diagram illustrating an example of the embodiment of the discrimination test measurement screen.
Fig. 95 is a diagram illustrating an example of the embodiment of the discrimination test measurement screen.
Fig. 96 is a diagram illustrating an example of the embodiment of the discrimination test measurement screen.
Fig. 97 is a perspective view of an embodiment of the cartridge 10.
Fig. 98 is a cross-sectional view, conceptually illustrating a state where odor component-containing air is released from the cartridge 10.
Fig. 99 is a diagram illustrating an embodiment of an odor generation system 100 according to a fifth embodiment.

### MODE FOR CARRYING OUT THE INVENTION

Hereinafter, preferred modes for carrying out the present technology will be described with reference to the drawings. Embodiments to be described hereinafter illustrate examples of representative embodiments of the present technology, and any embodiments can be combined. Furthermore, the scope of the present technology is not narrowly construed based on these. Note that the description will be given in the following order.
1. First Embodiment (Information Processing Device 3)
   (1) Overall configuration
   (2) Processing unit 31
   (3) Storage unit 32
   (4) User interface unit 33
   (5) Output unit 34
   (6) Operation example of information processing device 3
2. Second Embodiment (Information Processing System 200)
   (1) Overall configuration
   (2) Information processing device 3
   (3) Display device 2
   (4) Operation example of information processing system 200
      (4-1) Operation example 1
      (4-2) Operation example 2
      (4-3) Operation example 3
      (4-4) Operation example 4
      (4-5) Operation example 5
      (4-6) Operation example 6
3. Third Embodiment (Odor Generation System 100)
   (1) Overall configuration
   (2) Information processing device 3
   (3) Odor generation device 1
      (3-1) Cartridge holding portion 11 and movable portion 12
      (3-2) Cartridge 10 and cartridge unit 20
      (3-3) Releasing portion 140 and front storage portion 14
      (3-4) Drive mechanism portion 151, positioning drive portion 152, and back storage portion 15
      (3-5) User interface unit, storage unit, and output unit
   (4) Operation example of odor generation system 100
4. Fourth Embodiment (Odor Generation System 100)
   (1) Overall configuration
   (2) Information processing device 3
   (3) Odor generation device 1
      (3-1) Front storage portion 14 and back storage portion 15
      (3-2) Guide portion 141
5. Fifth Embodiment (Odor Generation System 100)
   (1) Overall configuration
   (2) Information processing device 3
      (2-1) Measurement preparation screen
      (2-2) Free measurement screen
      (2-3) Result display screen
      (2-4) Screen after end of measurement
      (2-5) History screen
      (2-6) Comparison display screen
   (3) Example of detection threshold test result screen flow
   (4) Example of recognition threshold test result screen flow
   (5) Example of result screen of detection average and recognition average
   (6) Example of three-option detection measurement flow
      (6-1) Overview of three-option detection measurement
      (6-2) Three-option detection measurement screen
      (6-3) Behavior when odor presentation button is tapped
   (7) About olfactory term table
   (8) Example of discrimination test measurement flow
      (8-1) Overview of discrimination test measurement
      (8-2) Discrimination test measurement screen
   (9) Odor generation device 1
      (9-1) Cartridge 10
      (9-2) Cartridge holding portion 21
      (9-3) Front storage portion 22
      (9-4) Back storage portion 23

### 1. First Embodiment (Information Processing Device 3)

### (1) Overall configuration

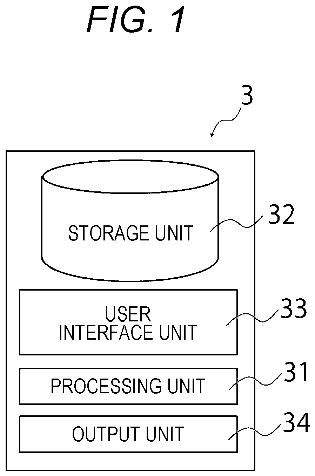

An overall configuration of an information processing device 3 according to a first embodiment of the present technology will be described with reference to Fig. 1. The information processing device 3 according to the present embodiment includes a processing unit 31, a storage unit 32, and a user interface unit 33. Furthermore, an output unit 34 or the like may be included as necessary.

Such components may be connected over a network. Furthermore, the components may be each provided in multiple instances and may be provided outside such as on a cloud and connected over a network. Hereinafter, each component will be described in detail.

### (2) Processing unit 31

The processing unit 31 is a component that identifies information regarding an odor component to be released on the basis of information associated with the user and controls the release of the odor component.

The "information associated with the user" given herein may include all matters regarding the user including a tester, a subject, and the like. Specific examples of the information associated with the user include biological information regarding the user, physical information regarding the user, information regarding an answer from the user, and the like. Furthermore, a combination of two or more pieces of such information may constitute the information associated with the user.

Examples of the biological information regarding the user may include a body temperature, a pulse rate, a heart rate, a respiration rate, a respiration frequency, a blood pressure, pupil (or eye) dilatation, a blink frequency, a brain wave, a perspiration rate, a yawning frequency, a urination status, a defecation status, and the like.

Examples of the physical information regarding the user may include, gender, age, a medical history, height, weight, body composition, eyesight, genetic information, a medication history, a supplement usage history, presence or absence of pregnancy, activity information regarding the user (for example, an occupation, a cohabitation family structure, sleep duration, an exercise habit, an eating habit, a smoking status, a drinking status, and the like), and the like.

Although details will be described later, examples of the information regarding an answer from the user may include a correct/incorrect result, an answer result, a test date and time, a time taken for an answer, the number of correct answers, a correct answer rate, the number of incorrect answers, an incorrect answer rate, and the like.

The "information regarding an odor component to be released" given herein may include all matters regarding an odor component to be released to the user. Specific examples of the information regarding an odor component to be released include a type of the odor component, a mixing ratio of the odor component, a concentration of the odor component, a release time of the odor component, a time until start of the release of the odor component, and the like. Furthermore, a combination of two or more pieces of such information may constitute the information regarding the release.

The number of types and concentrations of odor components are not particularly limited; however, in a case where the information processing device 3 according to the present technology is used in accordance with the Japanese standard olfactory acuity test (T&T olfactometer), odorants having concentrations of five types × eight levels (5 indicates the highest concentration, and the concentration is on a scale of 5, 4, 3, 2, 1, 0, -1, and -2 that differ by a factor of ten. Note that one of the odorants has no level 5 and thus has seven levels) can be used. Furthermore, for tests outside Japan, it is possible to make settings as appropriate so as to allow odor components of the number of types and concentrations determined in accordance with test criteria or test method law of the country to be released.

A method for mixing a plurality of odor components at a specific mixing ratio is not particularly limited, and for example, the method may be managed on the basis of the number of times or passing time of communication between a single inlet flow path and a plurality of outlet flow paths and set a ratio of the number of times of communication equal to the mixing ratio.

The release time of the odor component is not particularly limited, and can be set, for example, in a range of 0.01 to 30 seconds, 0.1 to 15 seconds, or 1 to 10 seconds as appropriate. Furthermore, the time until the release of the odor component is specifically a time until air is supplied to a cartridge 10 to be described later, for example. The time until the release of the odor component is not particularly limited, and can be set to, for example, 10 seconds or less, 5 seconds or less, or the like as appropriate.

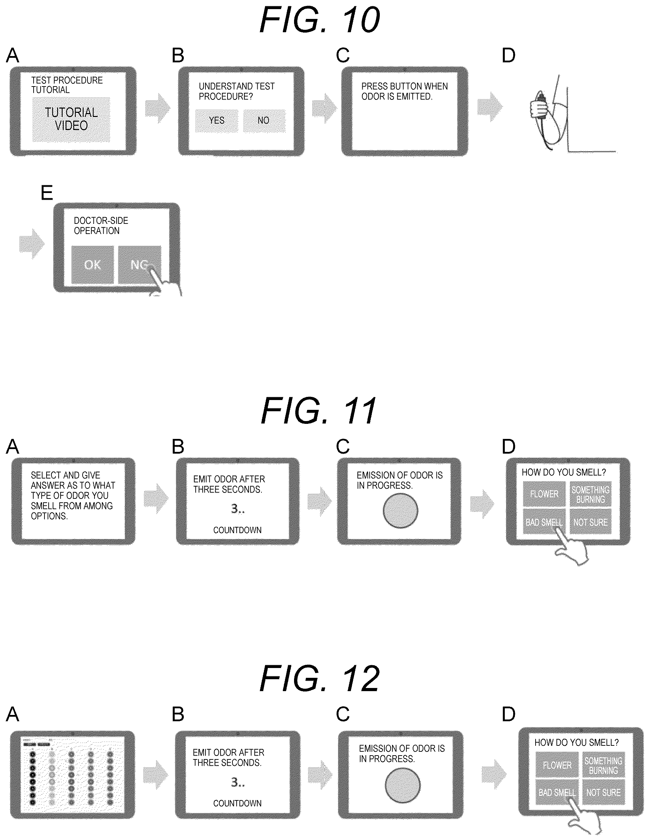

The processing unit 31 can further identify the concentration of the odor component to be released or the release time of the odor component on the basis of the information regarding the odor component and the information associated with the user thus identified. Specifically, as will be described for a recognition threshold flow illustrated in Figs. 11 and 12 to be described later, a test can be conducted with the same concentration as a concentration of an odor component identified by means of a detection threshold test conducted on a specific user. Furthermore, at this time, the release time of the odor component may also be adjusted on the basis of the answer result of the detection threshold or the information associated with the user.

In the present embodiment, more specifically, the information regarding the answer from the user can be a result of any one or more tests selected from the group consisting of an identification test, a threshold test, and a discrimination test. The identification test is a test to identify what type of odor it is. The threshold test is a test to determine the lowest concentration of an odor that can be detected or recognized. The discrimination test is a test to discriminate between different odors.

Furthermore, the processing unit 31 can control the release of the odor component on the basis of environmental information that affects the release of the odor component. Here, the "environmental information that affects the release of the odor component" may include all matters that affect the release of the odor component, and specific examples thereof include temperature, humidity, atmospheric pressure, and weather. Such environmental information is considered to affect the olfactory sense, so that it is possible to obtain an accurate result by releasing the odor component in accordance with the environmental information. Specifically, for example, the processing unit 31 adjusts, as appropriate, the release amount of the odor component, the concentration of the odor component, and the like on the basis of a result of the environmental information obtained from a conventionally known sensor or the like.

Moreover, the processing unit 31 can issue an instruction to output, to the output unit 34 in the device or a network such as a cloud, a result of any one or more tests selected from the group consisting of the identification test, the threshold test, and the discrimination test. Furthermore, an output format in this case is not particularly limited. Specifically, the information output by the processing unit 31 can be, for example, any one or more results selected from the group consisting of the detection threshold, the recognition threshold, and a difference between the detection threshold and the recognition threshold. The detection threshold corresponds to the lowest concentration at which the presence of an odor can be detected, and the recognition threshold corresponds to the lowest concentration at which the type of the odor can be recognized (the type of the odor can be expressed). This is useful, as will be described in detail later, for determining a medical history and a physical function of the user such as an olfactory discrimination ability.

### (3) Storage unit 32

The storage unit 32 is a component that stores the information associated with the user and/or the information regarding the odor component identified.

Furthermore, the storage unit 32 can store not only the above-described matters but also all matters regarding the test. For example, information regarding a cartridge holding an odor component (for example, a usage count, a usage period, a type of an odor component, a concentration of an odor component, and the like), information regarding a device (for example, a usage count, a usage period, a usage history, and the like), and the like can be stored. Note that, an external storage device or the like may be used as the storage unit 32 to store all matters regarding the test.

Note that a location where the storage unit 32 is installed and the number of the storage units 32 are not particularly limited, and the storage unit 32 may be installed in a casing accommodating the processing unit 31 or may be installed in an odor generation device 1 to be described later. Furthermore, the storage unit 32 may be installed outside such as on a cloud and connected to the processing unit 31 over a network.

### (4) User interface unit 33

The user interface unit 33 is a component to be operated by the user such as a tester or a subject. The user interface unit 33 displays the information regarding the odor component to be released, the information associated with the user, and the like to the user.

The user can access each component through the user interface unit 33 and control each component of the information processing device 3 according to the present technology. Note that a location where the user interface unit 33 is installed and the number of the user interface units 33 are not particularly limited, and the user interface unit 33 may be installed in the casing accommodating the processing unit 31, may be installed in the odor generation device 1 to be described later, or may be installed in both the casing and the odor generation device 1. For example, the user interface unit 33 installed in the information processing device 3 can be configured to allow operation for a test technician, a nurse, a doctor, and the like to be performed, and the user interface unit 33 installed in the odor generation device 1 can be configured to allow operation for the subject to be performed.

Furthermore, in addition to the above-described matters, all matters regarding the test are displayed to the user, and an instruction from the user is received. For example, information regarding the set cartridge 10 (for example, a usage count, a usage period, the type of an odor component, a concentration of an odor component, and the like), test progress, and information regarding the device (for example, a usage count, a usage expiration date, a usage period, a usage history, and the like) can be displayed. It is therefore possible to manage the usage count of the cartridge 10 or the device, and the like. Furthermore, the user interface unit 33 can acquire various types of information from the cartridge 10 or an identification portion 331 provided on the device via a reader 332, and manage a number for identifying each cartridge 10, a serial number, a type of an odor component, a concentration of an odor component, a production date, and the like on the basis of the various types of information. Examples of the identification portion 331 include a marker, an IC chip, an IC tag, a barcode, and a two-dimensional matrix barcode.

Furthermore, the user interface unit 33 may display an alert in a case where the state of the cartridge 10 to be described later exceeds a specific threshold. For example, the alert can be displayed in a case where the usage count of the cartridge 10 exceeds a predetermined count, in a case where the usage period of the cartridge 10 exceeds a predetermined period, in a case where the type of the odor component held in the cartridge 10 is different from a component intended for a test, in a case where the concentration of the odor component held in the cartridge 10 is different from a concentration intended for the test, or the like.

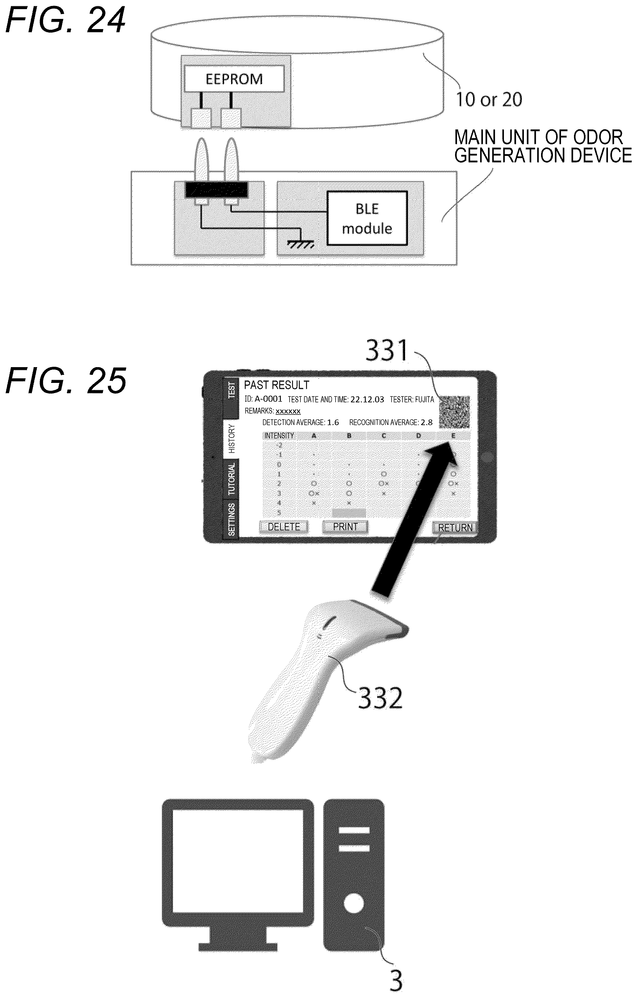

Furthermore, in the present embodiment, as illustrated in Fig. 24, an electrically erasable programmable read-only memory (EEPROM), which is a type of non-volatile memory, is incorporated in the cartridge 10 or a cartridge unit 20 itself to allow information such as the ID, the usage count, the usage expiration date, the usage period, the usage history, and the like of the cartridge 10 or the cartridge unit 20 to be stored in the cartridge 10 or the cartridge unit 20. It is therefore possible to prevent the cartridge 10 or the cartridge unit 20 from being used beyond the usage count, the usage expiration date, or the like. Furthermore, even when installed in another odor generation device 1, the information regarding the cartridge 10 or the cartridge unit 20 is not reset. Moreover, at the time of shipment or the like, it is possible to record the information such as the usage count, the usage expiration date, and the like for each cartridge 10 or cartridge unit 20.

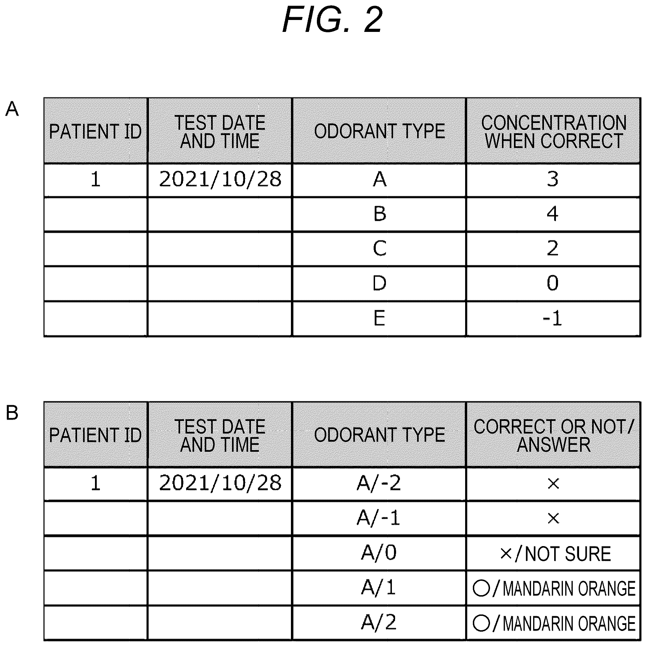

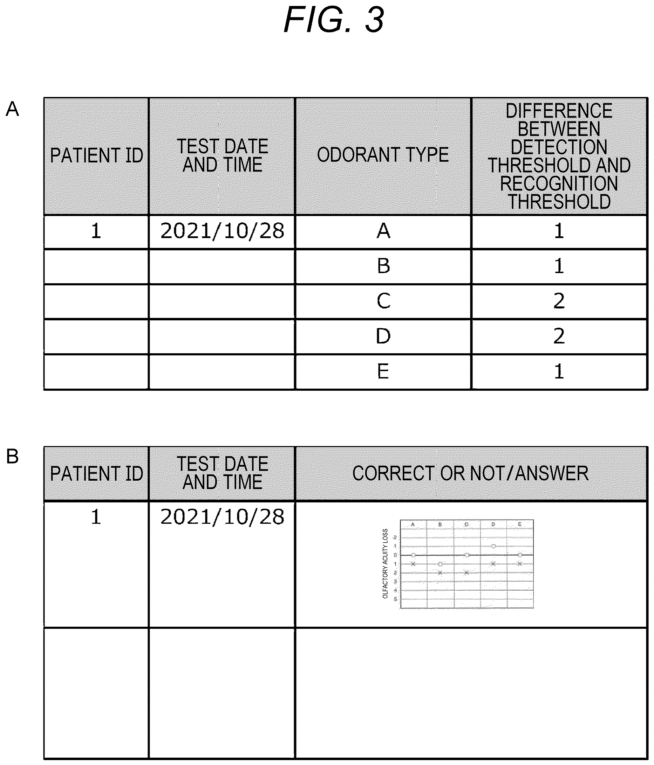

Furthermore, the user interface unit 33 can display, in various forms, information to the user such as a tester or a subject. Specifically, for example, a form in which a concentration at which an answer is correct is described for each odorant type as illustrated in Fig. 2A, a form in which a correct/incorrect result or an answer result is described for each odorant type or concentration as illustrated in Fig. 2B, or the like can be employed. Furthermore, for example, a form in which a difference between the detection threshold and the recognition threshold is described for each odorant as illustrated in Fig. 3A, a diagram or a graph conforming to a T&T olfactogram as illustrated in Fig. 3B, or the like may be employed.

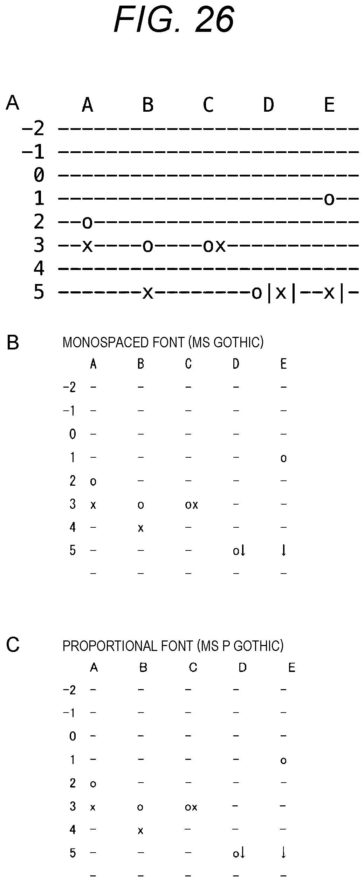

Moreover, the T&T olfactogram may be formed in a CSV format, and as illustrated in Fig. 25, the T&T olfactogram may be used as the identification portion 331(for example, a barcode, a two-dimensional matrix barcode, or the like) in the user interface unit 33, and data may be transferred to another information processing device 3 or the like by the reader 332. In the present embodiment, as the data transfer, for example, a transfer over a network such as Wi-Fi connection is possible, but a transfer without a network or Bluetooth (registered trademark) connection is also possible. Moreover, it is only required that the transfer be performed via the general-purpose reader 332 and electronic medical record software or the like be adapted to text input, which eliminates the need of special Input/Output. Figs. 26A to 26C illustrate examples of an ASCII art graph read by the reader 332.

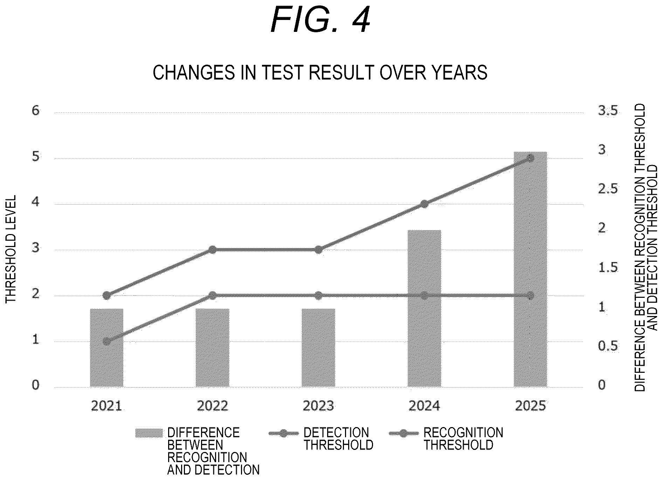

In addition, the user interface unit 33 can display the information associated with the user in time series to the user as illustrated in Fig. 4. Specifically, for example, any one or more pieces of information selected from the group consisting of the detection threshold, the recognition threshold, and the difference between the detection threshold and the recognition threshold can be displayed in time series using a graph or a table for each odorant or each test. The detection threshold and the recognition threshold may be each converted into a numeric value as illustrated in Fig. 4. Furthermore, such a numeric value may be an average in a case where the test is conducted a plurality of times. Note that, to be specific, it conforms to the determination criteria of the T&T olfactometer. The numeric conversion makes it possible to calculate a difference between the detection threshold and the recognition threshold. In the graph or the table described above, for example, as illustrated in Fig. 4, the horizontal axis represents a test date or a test year, and the vertical axis represents the user detection threshold, the user recognition threshold, and the difference between the user detection threshold and the user recognition threshold.

Here, as described above, in a specific neurodegenerative disease, it is also known that olfactory dysfunction occurs prior to decline in cognitive function, and for example, it is known that in Alzheimer's dementia, deposition of amyloid β protein or phosphorylated tau protein in an olfaction-related area is observed prior to hippocampus atrophy. Therefore, the information processing device 3 according to the present technology is considered to be effective as one of means for discovering a neurodegenerative disease or the like.

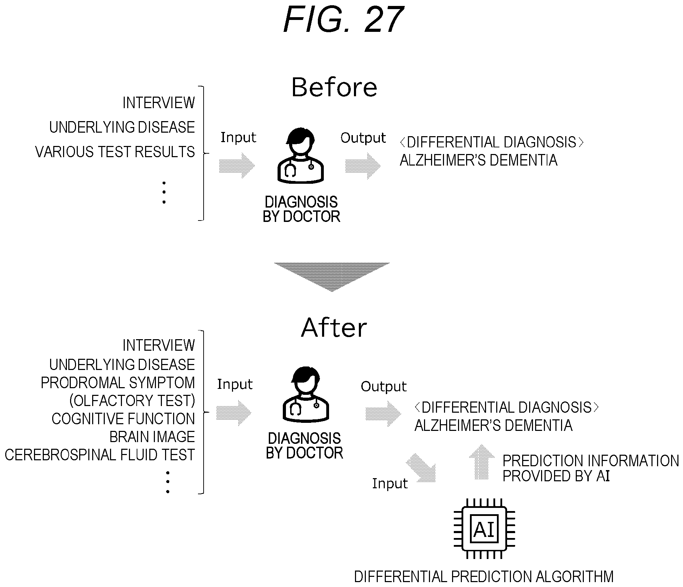

Fig. 27 is a diagram illustrating a neurodegenerative disease prediction model based on olfactory data analysis. Conventionally, a doctor make a differential diagnosis of Alzheimer's dementia, for example, on the basis of an interview, an underlying disease, various test results, and the like; however, the prediction model is used, that is, a prodromal symptom test (olfactometry) is added to various tests, information such as an interview, an underlying disease, and a prodromal symptom test, a cognitive function, a brain image, and a cerebrospinal fluid test is input to AI for memory learning to create a differential prediction algorithm, so that the diagnosis made by the doctor can be quickly and accurately supported.

That is, it is possible to confirm, by displaying the information associated with the user in time series, the history of the information associated with each user in a list form from a higher perspective. Furthermore, for example, a threshold is defined in advance for the difference between the user detection threshold and the user recognition threshold, and in a case where the processing unit 31 determines that the threshold exceeds a predetermined threshold, it can be determined to be an initial stage of a specific neurodegenerative disease, for example.

As the user interface unit 33, for example, a display, one or a plurality of buttons, a mouse, a keyboard, a touch panel, a personal digital assistant, or the like can be used. Furthermore, the user interface unit 33 is not an essential component for the information processing device 3, and an external display device may be used as illustrated in an information processing system 200 to be described later.

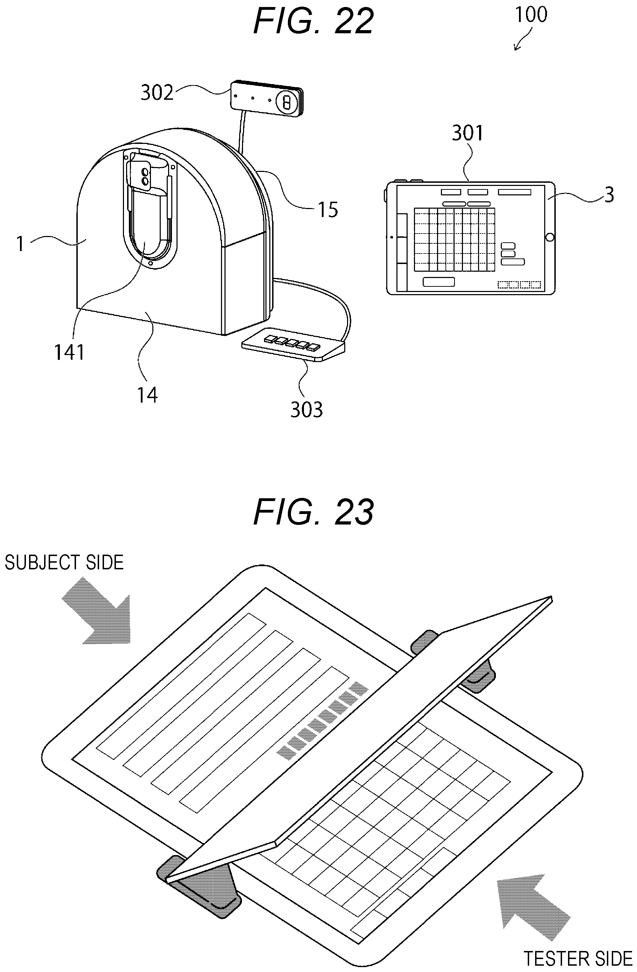

Fig. 23 is a diagram illustrating an embodiment of the user interface unit 33. As illustrated in Fig. 23, in a case where a display, a touch panel, a personal digital assistant, or the like is used in the user interface unit 33, in the present technology, even if two devices are not provided, a screen of one device can be split in two to be used separately between the subject and the tester. Furthermore, in Fig. 23, providing a partition at the screen split point and further installing the partition obliquely allow the subject to view only one of the split screens such as an input screen and on the other hand, allow the tester to confirm two screens (that is, both of the split screens), that is, a screen on which detailed information such as previous data and whether or not the answer from the subject is correct is displayed and the screen viewed by the subject. In a case of two screens, for example, an answer input screen such as an answer option can be displayed on the subject side, and a screen for a correct result of the answer or device control can be displayed on the tester side, for example.

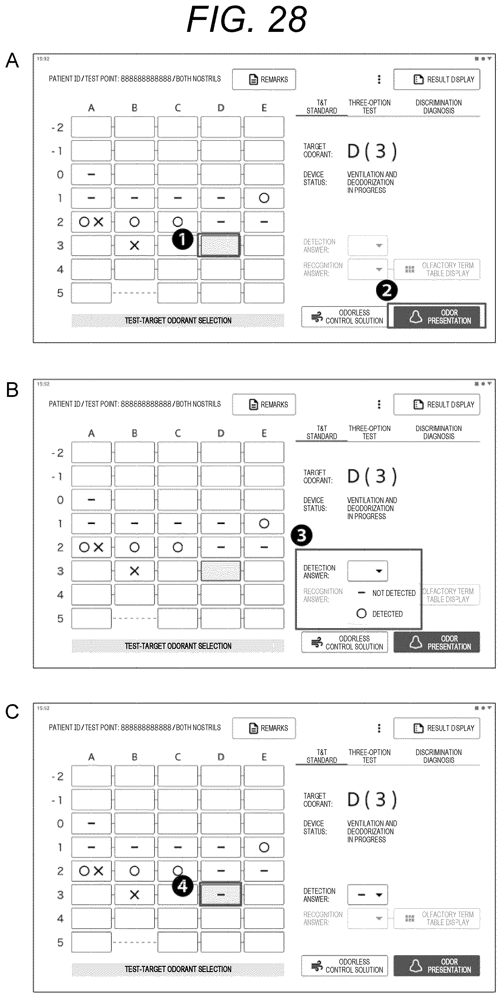

Fig. 28 is a diagram illustrating an example of an embodiment of a UI of the user interface unit 33. The UI illustrated in Fig. 28 has a layout like an olfactogram, and allows an odor component to be visually identified and touched with a pen, a finger, or the like to present the odor component. Through this UI, for example, the recognition test can be conducted after the detection test. Specifically, for example, an olfactory term table display can be activated after the detection test. In the detection test, for example, a three-option test can be conducted, and whether the olfactory term table is presented or not can be selected for each odorant presentation. Furthermore, through the UI, it is also possible to enable a discrimination test mode and to analyze changes over time using the result history. Moreover, when an odorant is presented, it is possible to count down by voice like "3, 2, 1, sniff it", to notify the subject of the timing at which the odor component is emitted. Specifically, the odorant is selected as indicated by "1" in Fig. 28, the odor component is presented by tapping an odor presentation button as indicated by "2" in Fig. 28, the measurer (tester) inputs an answer to the detection test as indicated by "3" in Fig. 28, and a test result is automatically reflected in the olfactogram as indicated by "4" in Fig. 28.

### (5) Output unit 34

The output unit 34 is a component that outputs, upon receipt of an instruction from the processing unit 31, the test result, for example.

The output unit 34 can output all matters regarding the test in addition to the test result. Note that a location where the output unit 34 is installed and the number of the output units 34 are not particularly limited, and the output unit 34 may be installed in the casing accommodating the processing unit 31 or may be installed in the odor generation device 1 to be described later. Further, upon receipt of an instruction from the processing unit 31, the output unit 34 may output a different content in a manner that depends on its installation location. For example, the output unit 34 installed in the information processing device 3 can be configured to output a detailed test result for a test technician, a nurse, a doctor, and the like, and the output unit 34 installed in the odor generation device 1 can be configured to output a simple test result for the subject.

As the output unit 34, a printer, a speaker, a personal digital assistant, or the like can be used. Furthermore, the output unit 34 is not an essential component for the information processing device 3, and an external output device may be used as the output unit 34.

### (6) Operation example of information processing device 3

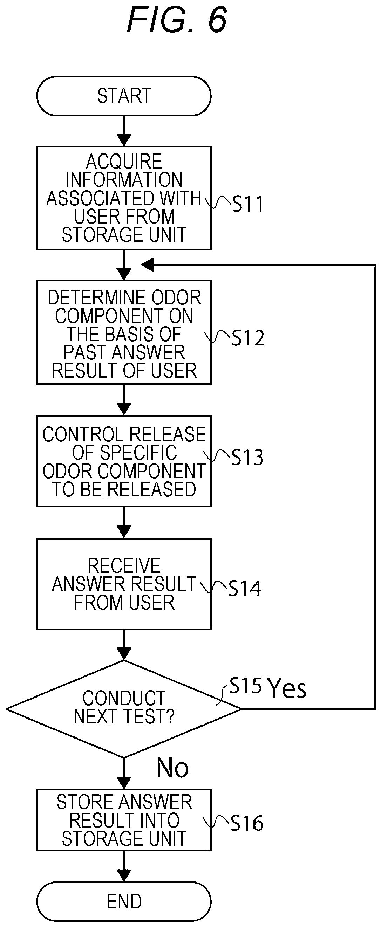

How the information processing device 3 operates will be described below with reference to Fig. 6.

First, the tester such as a doctor, a nurse, or a test technician acquires information associated with the subject (user) from the storage unit 32 (S11). At this time, the tester may collectively acquire the information as illustrated in step S11, or may acquire the information from the storage unit 32 at the first test or when switching to the second or subsequent test. In the present embodiment, for example, a past answer result, which is information regarding an answer from the user, can be acquired from the storage unit 32 as the information associated with the user. The processing unit 31 determines, on the basis of the answer result, for example, a specific odor component (odorant) for which the user correctly answered in the past (S12).

Next, the processing unit 31 controls the release of the specific odor component to be released on the basis of the information acquired at the first test or when switching to the second and subsequent test (S13). Specifically, for example, an initial concentration of the specific odor component to be released is determined. The release control is performed, for example, by changing the concentration of the specific odor component to be released or regulating the amount of air to be released from a releasing portion 140 of the odor generation device 1 to be described later. In step S13, the former method is used to select a cartridge 10 containing the odor component with a concentration lower by one or a plurality of levels than a concentration with which the answer was correct last time, and start the release of the odor component from the cartridge 10.

Next, the processing unit 31 receives an answer result from the user via the user interface unit 33 (S14). Note that the answer result may be received in the form of user's voice, gesture, or the like without using the user interface unit 33. Next, in a case where the next test is conducted (Yes in S15), the processing returns to step S12, for example. In a case where the next test is not conducted (No in S15), the flow proceeds to step S16, the answer result from the user is stored in the storage unit 32, and the test is ended.

As described above, the use of the information processing device 3 according to the present technology makes it possible to determine an odor component for the first time or the second or subsequent time on the basis of the information associated with the user, and to conduct a test suitable for each user. Furthermore, for example, it is also possible to control the release of the odor component by training an AI model for each user using a calculated initial concentration and an actual threshold result and estimating a better initial concentration.

### 2. Second Embodiment (Information Processing System 200)

### (1) Overall configuration

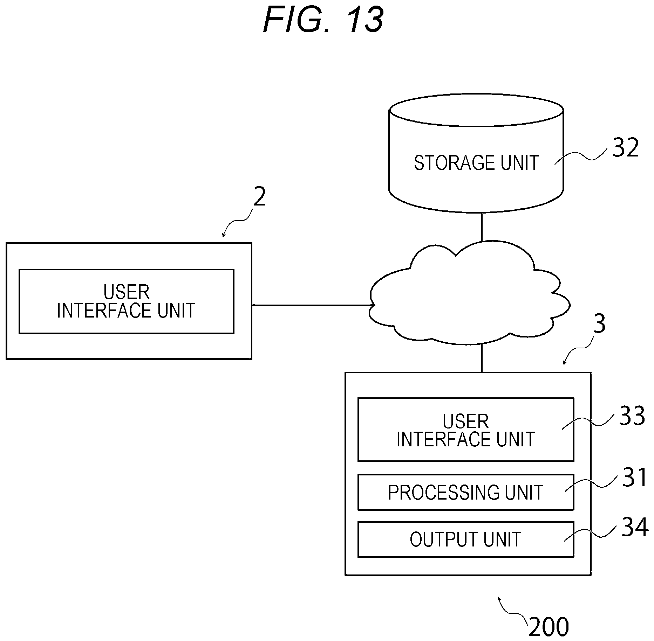

An overall configuration of the information processing system 200 according to the second embodiment of the present technology will be described with reference to Fig. 13. The information processing system 200 according to the present embodiment includes an information processing device 3 and a display device 2.

Such devices may be connected over a wired or wireless network. Furthermore, the devices may be each provided in multiple instances and may be provided outside such as a cloud and connected over a network.

Each device will be described in detail below.

### (2) Information processing device 3

The information processing device 3 includes a processing unit 31 that identifies information regarding an odor component to be released and controls the release of the odor component on the basis of information associated with the user. The information processing device 3 is similar to that described above, so that the description thereof will be omitted here.

### (3) Display device 2

The display device 2 displays information regarding the odor component to be released. The display device 2 corresponds to a configuration where the above-described user interface unit 33 is provided as an external display device. Furthermore, the above-described processing unit 31 (not illustrated) may be provided in the display device 2. The user interface unit 33 is similar to that described above, so that the description thereof will be omitted here.

### (4) Operation example of information processing system 200

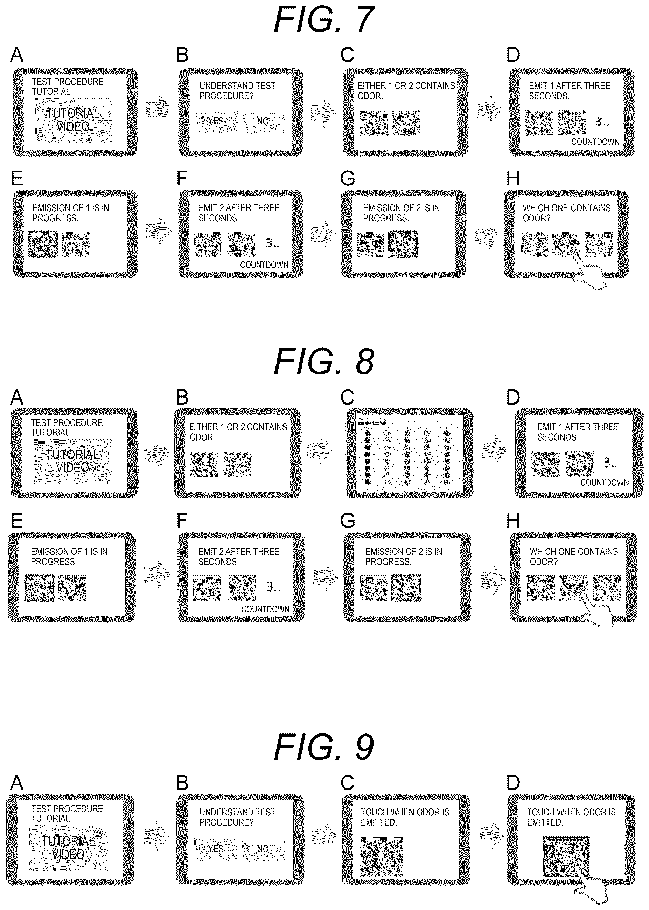

How the information processing system 200 operates will be described below with reference to Figs. 7 to 12.

### (4-1) Operation example 1

Fig. 7 illustrates a flow of a detection threshold test. Specifically, a test is conducted to determine which one of 1 or 2 has an odor. The flow illustrated in Fig. 7 is on the assumption that the subject himself/herself operates the display device 2.

First, the subject operates the display device 2 to select and input an initial odor component concentration.

Next, as illustrated in Fig. 7A, a description of a test procedure is given to the subject using a voice, a text, a diagram, or the like while using the user interface unit 33 as necessary. For example, a description like "Either 1 or 2 contains an odor. From now on, sniff 1 and 2 one by one" is given. At this time, a video or the like may be displayed on the user interface unit 33 as necessary.

Next, as illustrated in Fig. 7B, a confirmation is made as to whether or not the subject has correctly understood the test procedure while using the user interface unit 33 as necessary. For example, when a confirmation button is touched, the flow proceeds to the next step.

Next, the processing unit 31 automatically determines, at random, one type of odorant with the initial odor component concentration, and as illustrated in Fig. 7C, and automatically determine whether the odorant is released from which one of 1 or 2. Then, guidance like "Touch the start to begin the test" is displayed on the user interface unit 33, and when the subject touches the start, the flow proceeds to the next step.

Next, as illustrated in Fig. 7D, "Release 1 after three seconds" or the like is displayed to the subject via the display device 2, the time until the release of the odor component is displayed via the display device 2, and a countdown is performed using a voice, a lamp, a text, a diagram, or the like.

Then, as illustrated in Fig. 7E, 1 is released after three seconds, and the release of 1 is emphasized using a voice, a sign, or the like.

Next, as illustrated in Fig. 7F, "Release 2 after three seconds" or the like is displayed to the subject, the time until the release of the odor component is displayed via the display device 2, and a countdown is performed using a voice, a lamp, a text, a diagram, or the like.

Then, as illustrated in Fig. 7G, 2 is released after three seconds, and the release of 2 is emphasized using a voice, a lamp, a text, a diagram, or the like.

Then, as illustrated in Fig. 7H, "Touch either 1 or 2 from which you smell an odor to answer. If you are not sure, touch "Not sure"" is displayed via the display device 2, and the subject touches the corresponding option to answer.

When the subject finishes the input of the answer, the test may be ended, or alternatively, for example, a similar test may be conducted without interruption using another odor component or concentration. At this time, in a case where the result of the test using the same concentration is incorrect twice in a row, the processing unit 31 may reduce the concentration by one level and conduct the test. Furthermore, in a case where the result of the test using the same concentration is correct twice in a row, the test may be ended.

The use of the test flow as illustrated in Fig. 7 makes it possible to conduct a test even without the tester and to eliminate false answers and realize a test accurate for each user because the subject answers as to when the subject smells the odor among a plurality of times of presentation.

### (4-2) Operation example 2

As with Fig. 7, Fig. 8 illustrates a flow of the detection threshold test. Specifically, as in Fig. 7, the test is conducted to determine which one of 1 or 2 has an odor. The flow illustrated in Fig. 8 is, unlike Fig. 7, on the assumption that the tester such as a doctor, a nurse, or a test technician operates the display device 2.

First, as illustrated in Fig. 8A, a description of a test procedure is given to the subject using a voice, a text, or the like while using the user interface unit 33 as necessary. For example, a description like "Either 1 or 2 contains an odor. From now on, sniff 1 and 2 one by one" is given. At this time, a video or the like may be displayed on the user interface unit 33 as necessary.

Next, the tester confirms whether or not the subject has correctly understood the test procedure. Once confirmed, the flow proceeds to the next step.

Next, the tester operates the display device 2 to select the initial odor component concentration and determine one type of odor component (odorant) to be released with the concentration. Then, the tester also determines which one of 1 or 2 is to be released. This operation is not shown to the subject. Then, guidance like "Touch the start to begin the test" is displayed on the display device 2, and when the subject touches the start, the flow proceeds to the next step. Note that the guidance may be given verbally by the tester.

Next, as illustrated in Fig. 8D, "Release 1 after three seconds" or the like is displayed to the subject via the display device 2, the time until the release of the odor component is displayed via the display device 2, and a countdown is performed using a voice, a lamp, a text, a diagram, or the like.

Then, as illustrated in Fig. 8E, 1 is released after three seconds, and the release of 1 is emphasized using a voice, a lamp, a text, a diagram, or the like.

Next, as illustrated in Fig. 8F, "Release 2 after three seconds" or the like is displayed to the subject, the time until the release of the odor component is displayed via the display device 2, and a countdown is performed using a voice, a lamp, a text, a diagram, or the like.

Then, as illustrated in Fig. 8G, 2 is released after three seconds, and the release of 2 is emphasized using a voice, a lamp, a text, a diagram, or the like.

Then, as illustrated in Fig. 8H, "Touch either 1 or 2 from which you smell an odor to answer. If you are not sure, touch "Not sure"" is displayed via the display device 2, and the subject touches the corresponding option to answer. The answer may be given verbally by the subject.

When the subject finishes the input of the answer, the test may be ended, or alternatively, for example, a similar test may be conducted without interruption using another odor component or concentration. At this time, in a case where the result of the test using the same concentration is incorrect twice in a row, the tester may reduce the concentration by one level and conduct the test. Furthermore, in a case where the result of the test using the same concentration is correct twice in a row, the test may be ended.

The use of the test flow as illustrated in Fig. 8 makes it possible to eliminate false answers and realize a test accurate for each user because the subject answers as to when the subject smells the odor among a plurality of times of presentation.

### (4-3) Operation example 3

As with Figs. 7 and 8, Fig. 9 illustrates a flow of the detection threshold test. Specifically, the test is conducted in a manner similar the hearing test in Japan in which a notification is made only when smelling an odor. The flow illustrated in Fig. 9 is on the assumption that the subject himself/herself operates the display device 2.

First, the subject operates the display device 2 to select and input an initial odor component concentration.

Next, as illustrated in Fig. 9A, a description of a test procedure is given to the subject using a voice, a text, a diagram, or the like while using the user interface unit 33 as necessary. For example, a description like "During the next 15 seconds, an odor is released only for three seconds at a certain timing. If you smell the odor, touch the button" is given. At this time, a video or the like may be displayed on the user interface unit 33 as necessary.

Next, as illustrated in Fig. 9B, a confirmation is made as to whether or not the subject has correctly understood the test procedure while using the user interface unit 33 as necessary. For example, when a confirmation button is touched, the flow proceeds to the next step.

Next, the processing unit 31 automatically determines, at random, one type of odorant with the initial odor component concentration. Furthermore, the timing of the release of the odor within the 15 seconds is also determined. Then, guidance like "Touch the start to begin the test" is displayed on the user interface unit 33, and when the subject touches the start, the flow proceeds to the next step.

Next "Release 1 after three seconds" or the like is displayed to the subject via the display device 2, the time until the release the odor component is displayed via the display device 2, and a countdown is performed using a voice, a lamp, a text, a diagram, or the like.

Next, a countdown of 15 seconds is displayed using a voice, a lamp, a text, a diagram, or the like via the display device 2, and then the odor is presented to the subject at the set timing. At the other timing, for example, air is released from an empty cartridge 10 that does not hold the odor component. As illustrated in Fig. 9C, the subject touches the user interface unit 33 only while smelling the odor within the 15 seconds. When the user interface unit 33 is touched, as illustrated in Fig. 9D, a state where a screen or the like is touched may be emphasized using a voice, a lamp, a text, a diagram, or the like. Note that, in a case where the subject is an elderly person or the like, the subject may give the answer verbally rather than by means of the touch of the user interface unit 33.

Next, the processing unit 31 determines whether the answer is correct or incorrect. For the determination, for example, a threshold may be set, and a state where the user interface unit 33 has been touched within a predetermined number of seconds (for example, within 2 seconds, preferably within 1 second, or the like) from the presentation of the odor may be determined to be correct.

When the subject finishes the input of the answer, the test may be ended, or alternatively, for example, a similar test may be conducted without interruption using another odor component or concentration. At this time, in a case where the result of the test using the same concentration is incorrect twice in a row, the processing unit 31 may reduce the concentration by one level and conduct the test. Furthermore, in a case where the result of the test using the same concentration is correct twice in a row, the test may be ended.

The use of the test flow as illustrated in Fig. 9 makes it possible to conduct a test even without the tester and to eliminate false answers and realize a test accurate for each user because the test flow is based on a method where an odor is presented only at a certain time as in the hearing test in Japan, and a notification is made only when smelling the odor.

### (4-4) Operation example 4

As with Figs. 7 to 9, Fig. 10 illustrates a flow of the detection threshold test. Specifically, the test is conducted in a manner similar the hearing test in Japan in which a notification is made only when smelling an odor. The flow illustrated in Fig. 10 is, unlike Fig. 9, on the assumption that the tester such as a doctor, a nurse, or a test technician operates the display device 2.

First, as illustrated in Fig. 10A, a description of a test procedure is given to the subject using a voice, a text, a diagram, or the like while using the user interface unit 33 as necessary. For example, a description like "During the next 15 seconds, an odor is released only for three seconds at a certain timing. If you smell the odor, touch the button" is given. At this time, a video or the like may be displayed on the user interface unit 33 as necessary.

Next, as illustrated in Fig. 10B, the tester confirms whether or not the subject has correctly understood the test procedure. Once confirmed, the flow proceeds to the next step.

Next, the tester operates the display device 2 to select the initial odor component concentration and determine one type of odor component (odorant) to be released with the concentration. Then, the tester further determines the timing of the release of the odor within the 15 seconds. This operation is not shown to the subject. Then, guidance like "Touch the start to begin the test" is displayed on the display device 2, and when the subject touches the start, the flow proceeds to the next step. Note that the guidance may be given verbally by the tester.

Next "Release 1 after three seconds" or the like is displayed to the subject via the display device 2, the time until the release the odor component is displayed via the display device 2, and a countdown is performed using a voice, a lamp, a text, a diagram, or the like.

Next, a countdown of 15 seconds is displayed using a voice, a lamp, a text, a diagram, or the like via the display device 2, and then the odor is presented to the subject at the set timing. At the other timing, for example, air is released from an empty cartridge 10 that does not hold the odor component. As illustrated in Fig. 9C, the subject touches a button only while smelling the odor within the 15 seconds. Note that, in a case where the subject is an elderly person or the like, the subject may give the answer verbally rather than by means of the button.

Next, the tester determines whether the answer is correct or incorrect, and inputs the determination result via the display device 2 as illustrated in Fig. 10E. For the determination, for example, a threshold may be set, and a state where the button is pressed (for an elderly person or the like, when a verbal answer is obtained) within a predetermined number of seconds (for example, within 2 seconds) from the presentation of the odor may be determined to be correct. Alternatively, the tester may confirm the response of the subject and determine whether the answer is correct or incorrect on the basis of the response.

When the subject finishes the input of the answer, the test may be ended, or alternatively, for example, a similar test may be conducted without interruption using another odor component or concentration. At this time, in a case where the result of the test using the same concentration is incorrect twice in a row, the tester may reduce the concentration by one level and conduct the test. Furthermore, in a case where the result of the test using the same concentration is correct twice in a row, the test may be ended.

The use of the test flow as illustrated in Fig. 10 makes it possible to eliminate false answers and realize a test accurate for each user because the test flow is based on a method where an odor is presented only at a certain time as in the hearing test in Japan, and a notification is made only when smelling the odor.

### (4-5) Operation example 5

Fig. 11 illustrates a flow of a recognition threshold test. Specifically, the test is preferably conducted after the detection threshold. The flow illustrated in Fig. 11 is on the assumption that the subject himself/herself operates the display device 2.

First, the subject operates the display device 2 to select and input an initial odor component concentration. Note that the concentration is preferably the same as the concentration with which the answer is correct for the detection threshold. Note that the processing unit 31 may be configured to automatically start with the concentration with which the answer is correct for the detection threshold.

Next, as illustrated in Fig. 11A, a description of a test procedure is given to the subject using a voice, a text, a diagram, or the like while using the user interface unit 33 as necessary. For example, a description like "Select and give an answer as to what type of odor you smell from among options" is given. At this time, a video or the like may be displayed on the user interface unit 33 as necessary.

Next, a confirmation is made as to whether or not the subject has correctly understood the test procedure while using the user interface unit 33 as necessary. For example, when a confirmation button is touched, the flow proceeds to the next step.

Next, the processing unit 31 automatically determines, at random, one type of odorant with the detection threshold concentration. Then, guidance like "Touch the start to begin the test" is displayed on the user interface unit 33, and when the subject touches the start, the flow proceeds to the next step.

Next, as illustrated in Fig. 11B, "Release 1 after three seconds" or the like is displayed to the subject via the display device 2, the time until the release of the odor component is displayed via the display device 2, and a countdown is performed using a voice, a lamp, a text, a diagram, or the like.

After the end of the countdown, the odor is presented to the subject for a predetermined time. The time can be, for example, the same as the time for the detection threshold (for example, 0.01 to 30 seconds, preferably 0.1 to 15 seconds, more preferably 1 to 10 seconds), or may be adjusted on the basis of the information associated with the user. At this time, as illustrated in Fig. 11C, the presentation of the odor may be emphasized using a sound, a lamp, a text, a diagram, or the like.

Next, as illustrated in Fig. 11D, a plurality of options is displayed on the user interface unit 33. One of the options preferably includes "Not sure (uncertain)". The subject selects an answer from the plurality of options and inputs the answer via the display device 2.

Next, the processing unit 31 automatically determines whether the answer is correct or incorrect.

When the subject finishes the input of the answer, the test may be ended, or alternatively, for example, a similar test may be conducted without interruption using another odor component. At this time, in a case where the result of the test using the same concentration is incorrect twice in a row, the processing unit 31 may reduce the concentration by one level and conduct the test. Furthermore, in a case where the result of the test using the same concentration is correct twice in a row, the test may be ended.

The use of the test flow as illustrated in Fig. 11 makes it possible to conduct a test even without the tester and to make a comparison between testers or between hospitals by presenting unified expressions of blended odors to the subject because odor evaluations are presented as options to the subject to cause the subject to give an answer.

### (4-6) Operation example 6

As with Fig. 11, Fig. 12 illustrates a flow of the detection threshold test. Specifically, the test is preferably conducted after the detection threshold. The flow illustrated in Fig. 12 is, unlike Fig. 11, on the assumption that the tester such as a doctor, a nurse, or a test technician operates the display device 2.

First, as illustrated in Fig. 12A, a verbal description is given to the subject while using the user interface unit 33 as necessary. For example, a description like "Select and give an answer as to what type of odor you smell from among options" is given. At this time, a video or the like may be displayed on the user interface unit 33 as necessary.

Next, the tester confirms whether or not the subject has correctly understood the test procedure. Once confirmed, the flow proceeds to the next step.

Next, as illustrated in Fig. 12A, the tester operates the display device 2 to select the initial odor component concentration and determine one type of odor component (odorant) to be released with the concentration. This operation is not shown to the subject. Note that the concentration is preferably the same as the concentration with which the answer is correct for the detection threshold.

Next, the tester determines one type of odorant with the detection threshold concentration. Then, guidance like "Touch the start to begin the test" is displayed on the display device 2, and when the subject touches the start, the flow proceeds to the next step. Note that the guidance may be given verbally by the tester.

Next, as illustrated in Fig. 12B, "Release 1 after three seconds" or the like is displayed to the subject via the display device 2, the time until the release of the odor component is displayed via the display device 2, and a countdown is performed using a voice, a lamp, a text, a diagram, or the like.

After the end of the countdown, the odor is presented to the subject for a predetermined time. The time can be, for example, the same as the time for the detection threshold (for example, 0.01 to 30 seconds, preferably 0.1 to 15 seconds, more preferably 1 to 10 seconds), or may be adjusted on the basis of the information associated with the user. At this time, as illustrated in Fig. 12C, the presentation of the odor may be emphasized using a sound, a lamp, a text, a diagram, or the like.

Next, as illustrated in Fig. 12E, the tester verbally notifies the user of options, or a plurality of options is displayed on the user interface unit 33. Note that one of the options preferably includes "Not sure (uncertain)". The subject selects an answer from the plurality of options, and verbally communicates the answer to the tester or inputs the answer via the display device 2.

Next, the tester or the processing unit 31 automatically determines whether the answer is correct or incorrect. Note that the tester may confirm the response of the subject and determine whether the answer is correct or incorrect on the basis of the response.

When the subject finishes the input of the answer, the test may be ended, or alternatively, for example, a similar test may be conducted without interruption using another odor component. At this time, in a case where the result of the test using the same concentration is incorrect twice in a row, the tester may reduce the concentration by one level and conduct the test. Furthermore, in a case where the result of the test using the same concentration is correct twice in a row, the test may be ended.

The use of the test flow as illustrated in Fig. 12 makes it possible to make a comparison between testers or between hospitals by presenting unified expressions of blended odors to the subject because odor evaluations are presented as options to the subject to cause the subject to give an answer.

### 3. Third Embodiment (Odor Generation System 100)

### (1) Overall configuration

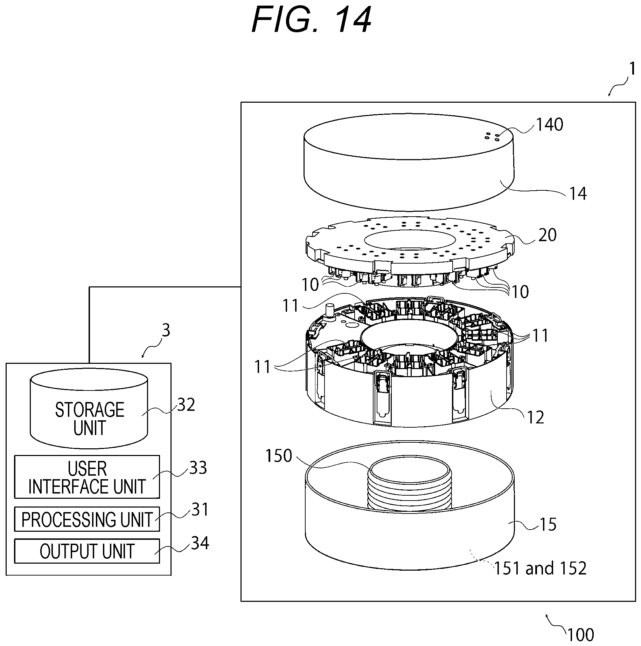

An overall configuration of an odor generation system 100 according to the third embodiment of the present technology will be described with reference to Fig. 14. The odor generation system 100 according to the present embodiment includes an odor generation device 1 and an information processing device 3.

Such devices may be connected over a wired or wireless network. Furthermore, the devices may be each provided in multiple instances and may be provided outside such as a cloud and connected over a network. Each device will be described in detail below.

### (2) Information processing device 3

The information processing device 3 includes a processing unit 31 that identifies information regarding an odor component to be released and controls the release of the odor component on the basis of information associated with the user. The information processing device 3 is similar to that described above, but in the present embodiment, the information processing device 3 may control each component of the odor generation device 1 to be described later. In this case, the form of the information processing device 3 is, for example, one of various devices having the user interface unit 33 or the like, and the user can give an instruction to each component of the odor generation device 1 via the user interface unit 33. Specific examples of the information processing device 3 include a tablet terminal, a wearable terminal, a personal computer terminal, and a smartphone.

### (3) Odor generation device 1

The odor generation device 1 is a device that releases an odor component to the outside. Note that, in the present technology, the "odor" may include any odor that stimulates some or all of the receptors present in the nasal cavity, such as a good odor and a bad odor. As will be described later, the nasal cavity has not only the olfactory receptors but also the trigeminal nerve receptors and the like, and the odor in the present technology is a broad concept including all odors that stimulate some or all of such receptors.

The odor generation device 1 is used, for example, as a device that releases an odor to a limited space, and is specifically used for olfactometry, olfactory training (olfactory stimulation therapy), and the like. Note that the olfactory training given herein is interpreted in a broad sense, and may include practice for examinations such as an odor analyst examination, a sommelier examination, and an aromatherapy examination. Moreover, it may be used for flavor simulation in the development of food and beverage products. Furthermore, it may be mounted on an automobile, a head mounted display, a relaxation product such as a neck pillow or an eye pillow, or the like. In a case where it is mounted on an automobile, it can generate an odor on the basis of, for example, an instruction from a driver or a passenger and may detect information regarding the location of the automobile, a motion or a biological signal of the driver or the passenger, or the like and generate an odor on the basis of the detection result. In a case where it is mounted on a head mounted display, it can generate an odor in conjunction with an image presented on the display and may detect a motion or a biological signal of the user, or the like and generate an odor on the basis of the detection result. In the case where it is mounted on a relaxation product such as a neck pillow or an eye pillow, the odor generation device 1 can generate an odor on the basis of an instruction from the user and may detect a motion or a biological signal of the user and generate an odor on the basis of the detection result.

Furthermore, the odor generation device 1 may be used as a device that releases an odor to a wide space, and specifically, the odor generation device 1 can be mounted on a customer attraction product such as a vending machine, a digital signage, or a robot. In a case where it is mounted on a customer attraction product, it can detect actions, facial expressions, or the like of a large number of unspecified individuals and generate an odor on the basis of the detection result.

The odor generation device 1 may be a portable device that can be carried by the user, or may be a stationary device.

The odor generation device 1 according to the present embodiment includes a cartridge 10, a cartridge holding portion 11, a movable portion 12, a support portion 13, a cartridge unit 20, a front storage portion 14 including a releasing portion 140, and a back storage portion 15 including a drive mechanism portion 151 and a positioning drive portion 152. Furthermore, although not illustrated, a user interface unit, a storage unit, an output unit, and the like may be included as necessary. Hereinafter, each component will be described in detail.

### (3-1) Cartridge holding portion 11 and movable portion 12

The cartridge holding portion 11 holds the cartridge 10 that holds an odor component. Note that the cartridge 10 is not an essential component in the present embodiment.

The cartridge holding portion 11 can hold one or more cartridges 10. The number of the cartridges 10 held by the cartridge holding portion 11 is not particularly limited, and can be set as appropriate according to a test method, a purpose, or the like. For example, in a case where it is used for the Japanese standard olfactory acuity test, 40 cartridges 10 are used (5 indicates the highest concentration, the concentration is on a scale of 5, 4, 3, 2, 1, 0, -1, and -2 that differ by a factor of ten. Note that one of the odorants has no level 5 and thus has seven levels), and it is possible to hold the cartridges 10 so as to allow a number of odors in accordance with the law of a country other than Japan to be generated.

A space into which the cartridge 10 can be attached is formed in the cartridge holding portion 11. The shape of the space is not particularly limited, can be adapted to the shape of the cartridge 10, and can be formed in, for example, a roughly rectangular parallelepiped shape, a roughly columnar shape, a roughly cubic shape, or the like. Furthermore, a communication hole 142 through which a pusher of the drive mechanism portion 151 to be described later extends may be formed in a lower surface of the cartridge holding portion 11.

In the present embodiment, the cartridge holding portion 11 is preferably provided with a crushing means that breaks a glass tube 102 in the cartridge 10. The crushing means includes both a chemical means and a physical means, and may be either a contact means or a non-contact means. Specifically, for example, in a case where the cartridge 10 is not provided with a pressing portion 107, it is preferable that a pressing mechanism capable of applying an external force to the glass tube 102 in the cartridge 10 be provided in the cartridge holding portion 11. Furthermore, for example, in a case where the cartridge 10 includes the pressing portion 107, a mechanism for pressing the pressing portion 107 may be provided in the cartridge holding portion 11. Moreover, in a case where the cartridge 10 is mounted while being slid relative to the cartridge holding portion 11, it is also possible to make a design so as to allow the pressing portion 107 of the cartridge 10 to be pushed by a convex portion by designing the convex portion on a part of a wall surface of the cartridge holding portion 11 or the like.

The material forming the cartridge holding portion 11 is not particularly limited, and the cartridge holding portion 11 can be formed of, for example, an organic polymer material. As the organic polymer material, for example, one or more of materials including polyvinyl chloride, polyethylene, a phenol resin, an olefin resin, nylon, polyester, a synthetic rubber, a silicone resin, a natural rubber, a protein, a nucleic acid, a lipid, or a polysaccharide may be used. Furthermore, in addition, one or more of materials including, for example, a polymer resin such as an acrylic resin, a urethane resin, an ABS resin, a polyether ether ketone (PEEK) resin, a polyacetal (POM) resin, a fluororesin, a cycloolefin polymer resin, a polyimide resin, a metal such as stainless steel or aluminum, an inorganic crystal such as quartz, and glass may be used. Moreover, the cartridge holding portion 11 may be formed in a porous structure, and as the porous structure, for example, a mesh structure, cork, mesoporous silica, calcium carbonate, or the like can be used. Furthermore, a fiber structure, a layered structure (for example, clay mineral or the like), ceramic, or the like can also be used other than the porous structure.

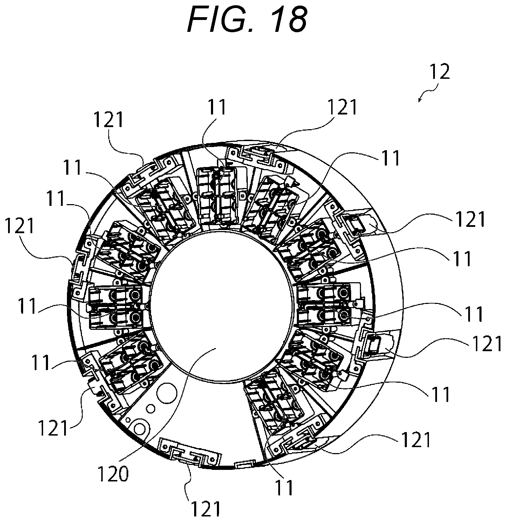

In the present embodiment, as illustrated in Fig. 18, the movable portion 12 including a plurality of the cartridge holding portions 11 may be provided. The material forming the movable portion 12 is not particularly limited, and examples of the material include the same materials as the examples of the material forming the cartridge holding portion 11, and thus the description thereof will be omitted here.

The shape of the movable portion 12 is not particularly limited as long as the movable portion 12 is formed to be able to hold the plurality of cartridge holding portions 11, and can be formed to adapt to the shape of the cartridge holding portion 11, but the movable portion 12 preferably includes the plurality of cartridge holding portions 11 arranged side by side as illustrated in Fig. 18. It is therefore possible to save space. Examples of the form in which the movable portion 12 includes the plurality of holding portions 11 arranged side by side include a form in which the plurality of cartridge holding portions 11 are arranged side by side in one direction, a form in which the plurality of cartridge holding portions 11 are arranged side by side in one direction and a direction orthogonal to the one direction, and a form in which the plurality of cartridge holding portions 11 are arranged side by side in a ring pattern as illustrated in Fig. 18.

### (3-2) Cartridge 10 and cartridge unit 20

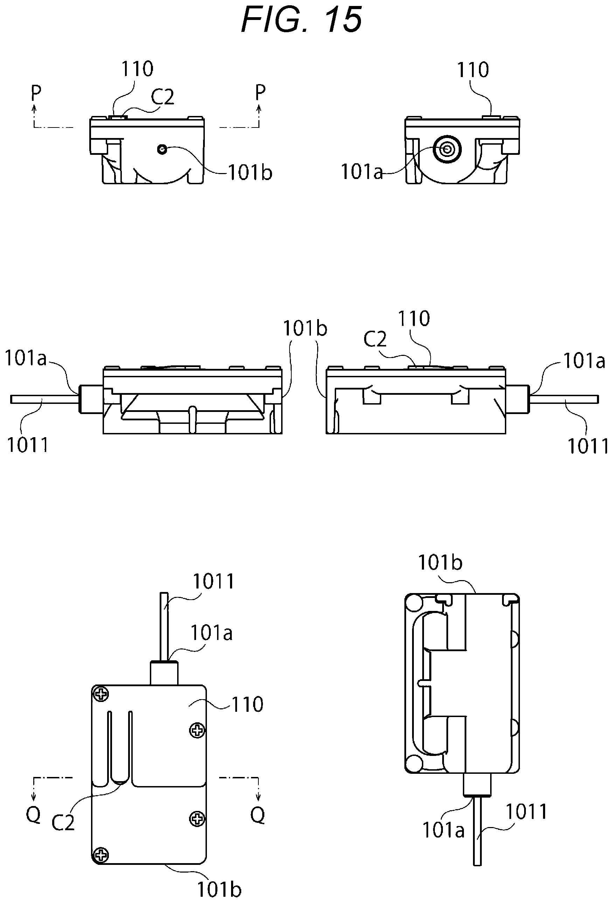

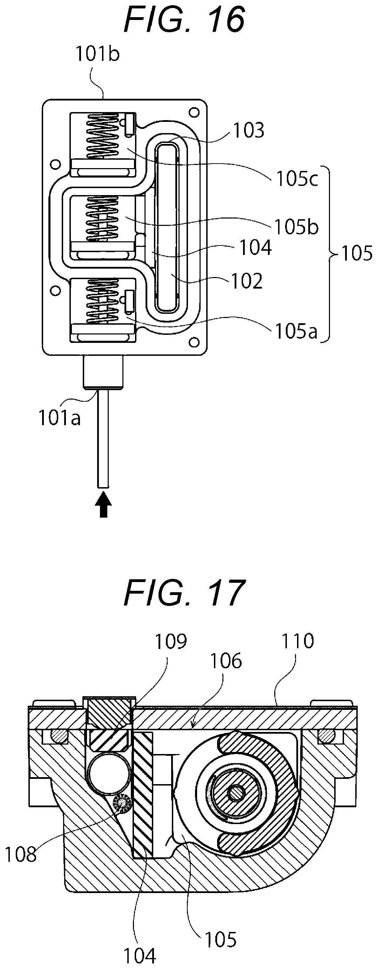

Fig. 15 is a six-sided view of an embodiment of the cartridge 10. Fig. 16 is a cross-sectional view taken along line P-P of the cartridge 10. Fig. 17 is a cross-sectional view taken along line Q-Q of the cartridge 10.

The cartridge 10 used in the present technology includes at least an opening 101 (101a and 101b), the glass tube 102, a glass pipe holding portion 103, an odor holder 104, and a ventilation portion 105 (105a, 105b, and 105c). Furthermore, the pressing portion 107 or the like may be provided as necessary. When the pressing portion 107 is pushed, pressure is applied to the glass tube 102 to crush the glass tube 102. An odor component is released from the glass tube 102 thus crushed, and the released odor component is absorbed and held by the odor holder 104.

The odor component held by the cartridge 10 may include any component that stimulates some or all of the receptors present in the nasal cavity, such as odor molecules. The nasal cavity has not only the olfactory receptors but also the trigeminal nerve receptors that are responsible for sensations of cold, heat, and pain, and the like, and the odor component in the present technology is a broad concept including all odors that stimulate some or all of such receptors. Specifically, for example, in a case where menthol is used as the odor component, the menthol can provide a stimulus through the olfactory receptors as well as a cold stimulus through the trigeminal nerve receptors (TRPA1 channel).

Note that the pressing portion 107 is not essential component for the cartridge 10, and for example, the glass tube 102 can be pressed by a pressing portion provided in the odor generation device 1 or using an external pressing device or the like.

When the odor is released from the cartridge 10, air flows in from the outside when one opening 101a (inflow opening 101a) is opened with the odor component held by the odor holder 104. The incoming air flows into the ventilation portion 105 to mix with the odor component held by the odor holder 104. In this state, opening the other opening 101b (release opening 101b) causes the air containing the odor component to be released to the outside.

In the present embodiment, the number of cartridges 10 is not particularly limited, and one or more cartridges 10 can be used according to the purpose of the test or the like. For example, in a case where the odor generation device 1 according to the present technology is used in accordance with the Japanese standard olfactory acuity test (T&T olfactometer), 40 cartridges 10 can be used (5 indicates the highest concentration, the concentration is on a scale of 5, 4, 3, 2, 1, 0, -1, and -2 that differ by a factor of ten. Note that one of the odorants has no level 5 and thus has seven levels). Furthermore, for tests outside Japan, it is possible to make a selection as appropriate so as to allow odor components of the number of types and concentrations determined in accordance with test criteria or test method law of the country to be released.

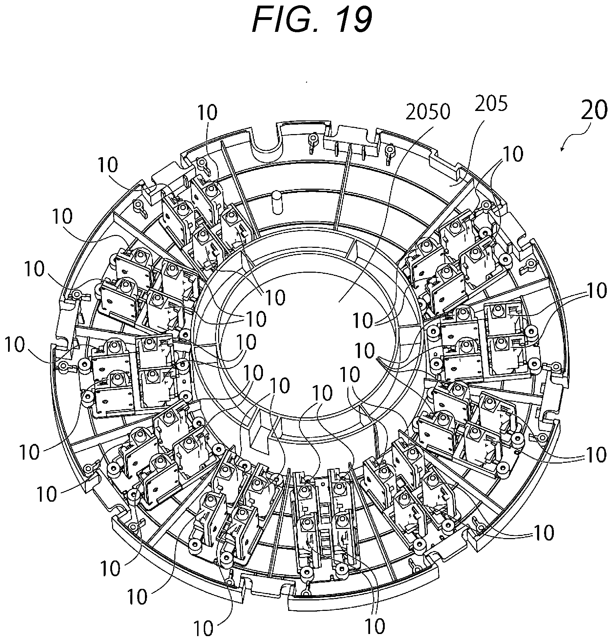

The cartridge 10 is used with the cartridge 10 attached to the cartridge holding portion 11 described above. In a case where the number of cartridges 10 is small, the cartridges 10 can be attached to predetermined cartridge holding portions 11 one by one; however, in a case where the number of cartridges 10 to be attached is large, a plurality of cartridges 10 can be attached to the odor generation device 1 at a time by using the cartridge unit 20 in which the plurality of cartridges 10 is supported in advance as illustrated in Fig. 19.

The number of cartridges 10 included in the cartridge unit 20 is, for example, 40, but is not limited to 40 in the present technology. However, in the present embodiment, since the cartridge unit 20 engages with the movable portion 12 described above with the plurality of cartridges 10 held by the cartridge unit 20, the number of the cartridge holding portions 11 held by the movable portion 12 and the number of the cartridges 10 held by the cartridge unit 20 are preferably the same.

The form of the cartridge unit 20 is not particularly limited as long as the cartridge unit 20 is formed so as to hold a plurality of cartridges 10, and can be formed to adapt to the shape of the cartridge 10 or the like. For example, the plurality of cartridges 10 can be supported with the plurality of cartridges 10 arranged side by side in one direction, the plurality of cartridges 10 can be supported with the plurality of cartridges 10 arranged side by side in one direction and a direction orthogonal to the one direction, or the plurality of cartridges 10 can be supported in a ring pattern. Note that the cartridge unit 20 may support not only the plurality of cartridges 10 in one plane but also the plurality of cartridges 10 in a plurality of planes.

In the present embodiment, the cartridge unit 20 preferably includes a retaining portion 205 that retains the plurality of cartridges 10. The form of the retaining portion 205 is not particularly limited, and the retaining portion 205 can be formed so as to adapt to the shape of the cartridge 10, the arrangement form of the cartridge 10, and the like; however, in the embodiment illustrated in Fig. 19, the retaining portion 205 has a hole 2050 to engage with another portion and supports the plurality of cartridges 10 in a multiple ring pattern as viewed from above, and in case where the number of cartridges 10 is large, the plurality of cartridges 10 is supported in a multiple ring pattern as viewed from above, so that it is possible support more cartridges 10 and save space.

For example, the material forming the retaining portion 205 is not particularly limited, and, for example, one or more of materials including an organic polymer material, a polymer resin such as an acrylic resin, a urethane resin, an ABS resin, a polyether ether ketone (PEEK) resin, a polyacetal (POM) resin, a fluororesin, a cycloolefin polymer resin, or a polyimide resin, and a metal such as stainless steel or aluminum may be used.

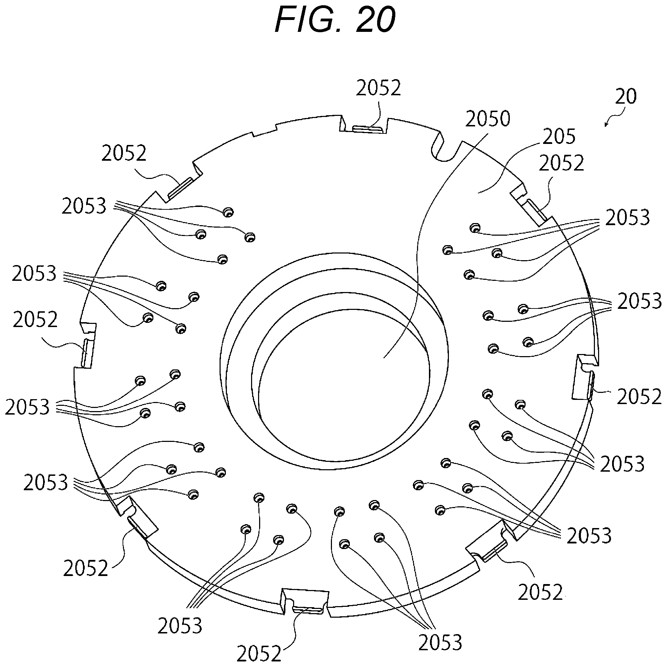

The embodiment illustrated in Fig. 20 is a diagram schematically illustrating a surface (back surface in Fig. 19) of the retaining portion 205 without the plurality of cartridges 10. The retaining portion 205 preferably has an emission hole 2053 through which the odor component is emitted. The number of emission holes 2053 is not particularly limited, but it is more preferable that a plurality of emission holes 2053 be provided, and it is particularly preferable that the number of emission holes 2053 be the same as the number of cartridges 10 supported by the cartridge unit 20. Furthermore, the emission hole 2053 is preferably located at a position corresponding to the releasing portion 140 to be described later. In this case, an air current from the releasing portion 140 can be emitted through the emission hole 2053. Furthermore, the retaining portion 205 preferably includes a fitted portion 2052 to fit with the movable portion 12 and a fitting portion 150 to be described above. The number and form of the fitted portions 2052 can be freely designed according to the number and form of the movable portions 12 and the fitting portions 150.

Note that, in the cartridge unit 20, a plurality of cartridges 10 may be attached in advance to some or all of the support portions 13, and in this case, the cartridge unit 20 may be distributed with the plurality of cartridges 10 provided in advance in some or all of the support portions 13. Furthermore, in a case where there is no odor in some or all of the cartridges 10, a case where a certain period has elapsed since the purchase of the cartridge unit 20, or the like, the user can cope with such a case by replacing only the cartridge unit 20. Therefore, the cartridge unit 20 according to the present technology can be distributed alone. Furthermore, it is also possible to replace only some or all of the cartridges 10 in the cartridge unit 20. Therefore, the cartridge 10 can also be distributed alone.

Furthermore, the cartridge 10 and the cartridge unit 20 may include an identification portion 331. The identification portion 331 is similar to that described above, so that the description thereof will be omitted here. A reader 332 performs reading via the identification portion 331, and the read information can be output to the processing unit 31.

### (3-3) Releasing portion 140 and front storage portion 14

The releasing portion 140 is a component that releases air containing the odor component to the outside.

For example, as illustrated in Fig. 14, the releasing portion 140 may be provided in the front storage portion 14, and in this case, the releasing portion 140 may communicate with the emission hole 2053 of the cartridge unit 20.

In the present embodiment, the releasing portion 140 may include a guide portion (not illustrated) that guides an air current containing the odor component toward the nose of the user. The material forming the guide portion is not particularly limited, and examples of the material include paper (including recycled paper), wood, a bamboo sheath, plastic, and coal. The guide portion may be formed in a partially or entirely detachable form, and in this case, for example, the guide portion may be disposable for each user.

### (3-4) Drive mechanism portion 151, positioning drive portion 152, and back storage portion 15

The back storage portion 15 may be provided with the fitting portion 150 that fits with the movable portion 12 and the fitted portion 2052 described above. The fitting portion 150 is preferably configured to be able to withstand, when the plurality of cartridges 10 is crushed, a load applied in a direction in which the cartridge 10 is detached or attached.

Furthermore, as illustrated in Fig. 14, the back storage portion 15 includes the drive mechanism portion 151. Furthermore, a board for supplying power to the drive mechanism portion 151, the positioning drive portion 152, and the like on the basis of an instruction or the like from the user interface unit 33 may be provided. The drive mechanism portion 151 includes a drive mechanism accommodation portion, and is connected to an operation shaft that is the movable portion 12 and a first shaft in the cartridge 10 to drive the operation shaft and the first shaft. The drive mechanism accommodation portion can be formed in, for example, a cylindrical shape, or alternatively, can be formed in a columnar shape, a rectangular parallelepiped shape, a cubic shape, or the like so as to adapt to the shape of the cartridge 10.

The drive mechanism portion 151 includes, in the drive mechanism accommodation portion, a pusher connected to the operation shaft and a shape memory alloy SMA of a thin wire that is a drive source for driving the pusher. A rear end of the pusher is fixed to a drive mechanism fixing portion provided at the inner rear end of the drive mechanism accommodation portion. An SMA sliding portion for folding back and sliding the shape memory alloy SMA is provided near the distal end of the pusher. Furthermore, the drive mechanism portion 151 is entirely fixed by a support or the like attached below the drive mechanism accommodation portion, and wiring capable of supplying power is connected to the rear end of the shape memory alloy SMA located in the drive mechanism fixing portion.

The pusher is movable inside the drive mechanism accommodation portion in the extending direction by expansion and contraction of the shape memory alloy SMA. The shape of the pusher is not particularly limited as long as the pusher can push the operation shaft, and may be a columnar shape, a conical shape, a cylindrical shape, a prismatic shape, or the like.

The shape memory alloy SMA is folded back in a U-shape at the SMA sliding portion provided near the distal end of the pusher and passes through the inside of the pusher, and both ends thereof are fixed to the drive mechanism fixing portion located at the rear end of the pusher. Moreover, an actuator as a drive source is not limited to the shape memory alloy SMA, and is only required to be, for example, a linear motion mechanism, such as a motor, a solenoid, a linear slide type, a pneumatic (air pump type), or a small electromagnet, which linearly moves the pusher. Here, the linear motion mechanism includes not only a case where one member moves in the linear direction but also a case where a part of a plurality of members connected to each other moves in the linear direction.

Furthermore, as illustrated in Fig. 14, the back storage portion 15 includes the positioning drive portion 152. The positioning drive portion 152 is a component that positions a specific cartridge 10 among the plurality of cartridges 10 in the vicinity of the releasing portion 140. Since the odor generation device 1 includes the positioning drive portion 152, any cartridge 10 can be moved to the vicinity of the releasing portion 140, and the odor component held by the odor holder 104 of the cartridge 10 can be delivered to a desired point (for example, the tip of the nose of the user, or the like). Furthermore, it is therefore possible to minimize odor adhesion to the components constituting the odor generation device 1 as much as possible. In the present embodiment, the positioning drive portion 152 is provided in the above-described back storage portion 15. The back storage portion 15 includes a board for supplying power to the positioning drive portion 152 on the basis of an instruction or the like from the user interface unit 33.

The positioning drive portion 152 can perform driving in accordance with the form of the cartridge unit 20 or the like, and is capable of, for example, linear driving, XY-axis driving, or rotational driving. The positioning drive portion 152 is not particularly limited, and a conventionally known actuator or the like can be used.

### (3-5) User interface unit, storage unit, and output unit

A user interface unit (not illustrated) corresponds to a case where the user interface unit 33 described above is provided in the odor generation device 1, and is not an essential component in the present embodiment. Furthermore, a storage unit (not illustrated) corresponds to a case where the storage unit 32 described above is provided in the odor generation device 1, and is not an essential component in the present embodiment. Moreover, an output unit (not illustrated) corresponds to a case where the output unit 34 described above is provided in the odor generation device 1, and is not an essential component in the present embodiment. Note that the user interface unit, the storage unit, and the output unit are similar to those described above, so that the description thereof will be omitted here.

### (4) Operation example of odor generation system 100

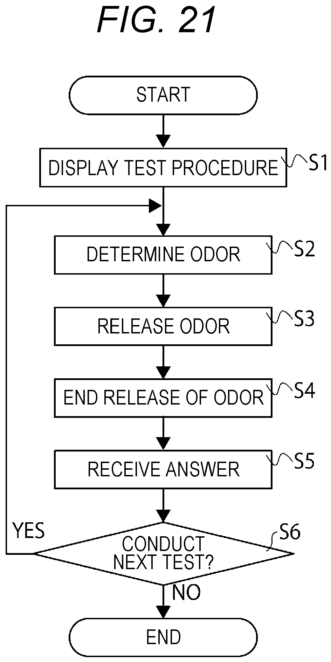

How the odor generation system 100 operates when olfactometry or olfactory training is conducted using the odor generation system 100 will be described below with reference to Fig. 21.

First, the tester such as a doctor, a nurse, or a test technician instructs the information processing device 3 to display the test procedure to the subject (S1). In the present embodiment, various devices each having a user interface unit may be provided not only on the tester side but also on the subject side. Next, the tester determines an odor (odorant) to be released to the subject, and issues an instruction to the odor generation device 1 via the information processing device 3. At this time, upon receipt of the instruction, the positioning drive portion 152 positions a cartridge 10 having a specific odor in the vicinity of the releasing portion 140 to release the odor in accordance with the operation example of the odor generation device 1 described above (S3).

Next, when the release of the odor is completed (S4), the information processing device 3 receives an answer from the subject, and the storage unit 32 stores the answer (S5). Next, the tester determines whether or not to conduct the next test (S6), and in a case of Yes, returns to step S2 again and determines the next odor. In a case where the positioning drive portion 152 performs rotational driving, when the cartridge 10 having the next specific odor is positioned in the vicinity of the releasing portion 140, a deodorizing operation may be performed in accordance with the operation example of the odor generation device 1 described above. In a case of No, the tester simply ends the test.

### 4. Fourth Embodiment (Odor Generation System 100)

### (1) Overall configuration

An overall configuration of an odor generation system 100 according to the fourth embodiment of the present technology will be described with reference to Fig. 22. The odor generation system 100 according to the present embodiment includes an odor generation device 1 and an information processing device 3. Furthermore, the display device 2 described above may be included as necessary.

Such devices may be connected over a wired or wireless network. Furthermore, the components may be each provided in multiple instances and may be provided outside such as on a cloud and connected over a network. Each device will be described in detail below.

### (2) Information processing device 3

The information processing device 3 includes a processing unit 31 that identifies information regarding an odor component to be released and controls the release of the odor component on the basis of information associated with the user. The information processing device 3 is similar to that described above, but in the present embodiment, the information processing device 3 may control each component of the odor generation device 1 to be described later. In this case, the form of the information processing device 3 is, for example, one of various devices having the user interface unit 33 or the like, and the user can give an instruction to each component of the odor generation device 1 via the user interface unit 33. Specific examples of the information processing device 3 include a tablet terminal 301 as illustrated in Fig. 22, a wearable terminal, a personal computer terminal, and a smartphone. The tablet terminal 301 or the like may be provided on both the subject side and the tester side, or may be provided only on the tester side.

Furthermore, in the present embodiment, the information processing device 3 may include a display module 302, a button module 303, and the like connected to the information processing device 3 over a network, as illustrated in Fig. 22, in addition to the tablet terminal 301 described above. The display module 302 is a component mainly responsible for the countdown until an odor is released. It is therefore possible for the subject to sniff the odor at the instant when the odor is emitted, which facilitates a transition to the next operation of the odor generation device 1 such as deodorization to be described later. Furthermore, it also serves as notification for the subject, thereby increasing the accuracy and making the subject ready for the generation of the odor without surprise. Furthermore, it is possible to gain time for the rotational operation of the odor generation device 1 and the operation of the actuator, and it is possible to reduce discomfort while the user is waiting. Furthermore, the button module 303 is a component mainly responsible for the input of the answer from the user.

Note that the display module 302 and the button module 303 are preferably installed at positions suitable for the subject. For example, the display module 302 is preferably installed at a distance at which the user's eyes come into focus. In particular, in a case of an elderly person, a child, or the like, the button module 303 is preferably installed at a position where the button module 303 is easily operated with a hand.

### (3) Odor generation device 1

The odor generation device 1 according to the present embodiment includes the cartridge 10, the cartridge holding portion 11, the movable portion 12, the support portion 13, the cartridge unit 20, the front storage portion 14 having the releasing portion 140 and the communication hole 142, a guide portion 141, and the back storage portion 15 having the drive mechanism portion 151 and the positioning drive portion 152. Furthermore, a different component may be provided as necessary. Note that Fig. 22 illustrates a state where each component already engages with the front storage portion 14 and/or back storage portion 15.

Hereinafter, each component will be described in detail.

Note that, in the present embodiment, the cartridge 10, the cartridge holding portion 11, the movable portion 12, the support portion 13, the cartridge unit 20, the releasing portion 140, the communication hole 142, the drive mechanism portion 151, and the positioning drive portion 152 are similar to those described above, so that the description thereof will be omitted here.

### (3-1) Front storage portion 14 and back storage portion 15

In the present embodiment, a casing formed by the front storage portion 14 and the back storage portion 15 engaging with each other has a roughly semi-elliptical columnar shape. It is therefore possible for the user to sniff the odor without bending his/her neck, and in particular, even a user such as an elderly person who has difficulty in maintaining a posture with his/her neck bent can sniff the odor in a stable posture. Note that, also in the present embodiment, a position adjustment portion (not illustrated) can be provided on their respective bottom surfaces of the front storage portion 14 and the back storage portion 15 as necessary.

### (3-2) Guide portion 141

In the present embodiment, the guide portion 141 includes, for example, a nose cover portion that covers the nose of the user, an inflow prevention portion that prevents the inflow of exhalation from the mouth of the user, and a guiding portion that guides air containing the remaining odor component. The guide portion 141 may be disposable for each user. It is therefore possible to eliminate the risk that an odor remains in the guide portion 141 and conduct a test with accuracy. Furthermore, in the present embodiment, the guide portion 141 may include a folded portion. It is therefore possible to form the guide portion 141 threedimensionally in a simple manner and store each guide portion 141 with the folded portions unfolded and placed on top of each other, so that the guide portion 141 is easily distributed, stored, and maintained in shape.

### 5. Fifth Embodiment (Odor Generation System 100)

### (1) Overall configuration

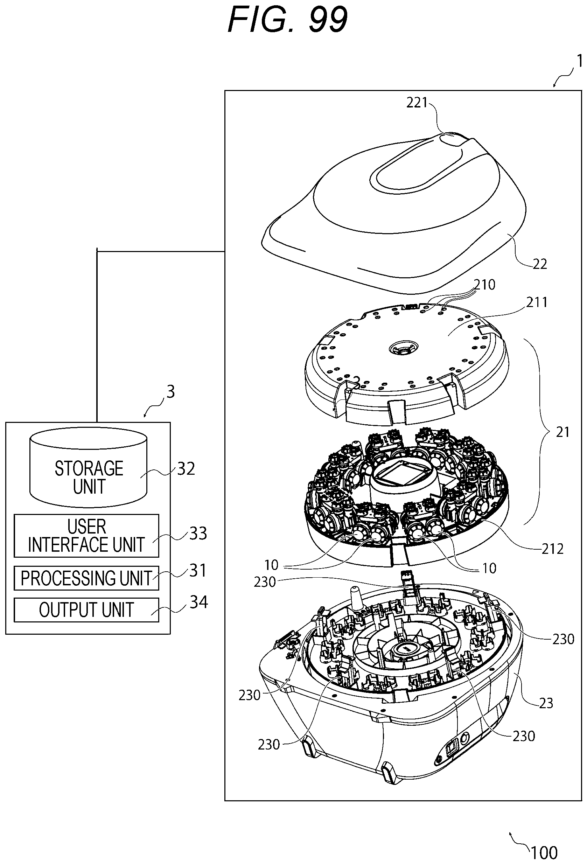

An overall configuration of an odor generation system 100 according to the fifth embodiment of the present technology will be described with reference to Fig. 99. The odor generation system 100 according to the present embodiment includes an odor generation device 1 and an information processing device 3. Furthermore, the display device 2 described above may be included as necessary.

Such devices may be connected over a wired or wireless network as in the fourth embodiment. Furthermore, the components may be each provided in multiple instances and may be provided outside such as on a cloud and connected over a network. Each device will be described in detail below.

### (2) Information processing device 3

The information processing device 3 is similar to that described in the fourth embodiment, but it is particularly preferable that the tablet terminal 301 be used as the user interface unit 33. The tablet terminal 301 may be provided on both the subject side and the measurer (tester) side, or may be provided only on the measurer side.

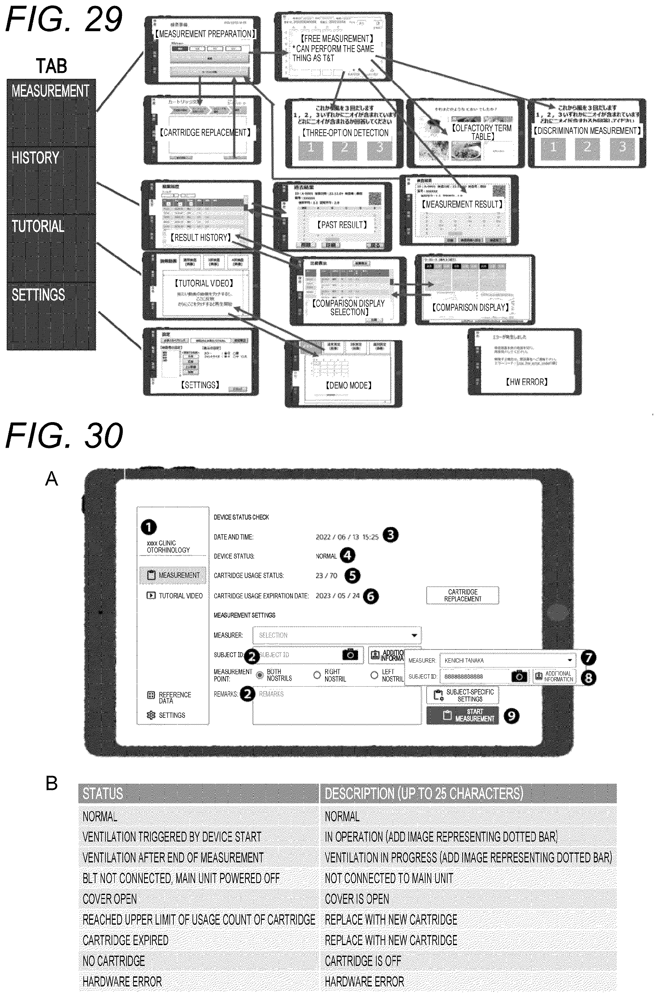

In the fifth embodiment, as illustrated in Fig. 29, an UI displayed on the tablet terminal 301 can hold a screen to which a transition has been made for each tab. Specifically, for example, in a measurement tab, with a free measurement screen active, when switching is made to a history tab, a task is performed, and when a return to the measurement tab is made, a transition to the free measurement is made. That is, a transition to measurement preparation is prevented herein. Without such a setting, a state transition becomes out of sync between the tablet terminal 301 and the odor generation device 1.

The user interface unit 33 (particularly, the tablet terminal 301) displays any one or more pieces of information selected from the group consisting of information associated with the user, information regarding the odor component to be released, information regarding the cartridge, and information regarding measurement using the odor component. Hereinafter, screens of measurement preparation, free measurement, result display, after the end of measurement, history display, and comparison display will be described in detail with reference to the drawings.

### (2-1) Measurement preparation screen

Fig. 30 is a diagram illustrating an example of an embodiment of the measurement preparation screen. In "1" in Fig. 30A, a clinic name can be input. Furthermore, in "2" in Fig. 30A, an icon representing both nostrils is enabled by default. Here, when measurement is set with the same ID and the same date and time (regardless of both nostrils, right nostril, or left nostril), the measurement may be regarded as single measurement. In "3" in Fig. 30A, the time of the tablet terminal 301 is displayed, and long-pressing the time makes the time correctable. In "4" in Fig. 30A, a device status is displayed, and, when the device status is other than "normal", a transition to the measurement is disabled (for example, the measurement button cannot be pressed). Examples of the status other than "normal" are illustrated in Fig. 30B. The status other than "normal" is preferably indicated in red so as to enable the user to easily understand the status. Furthermore, other than the above, a configuration where unless the measurer and the subject ID to be described later have been entered, the transition to the measurement is disabled may be employed.

In "5" in Fig. 30A, a cartridge usage status is displayed, and, for example, when the usage count of the cartridge 10 becomes less than or equal to 5, the portion of "5" is displayed in red and/or blinking. Furthermore, in a case where the cartridge 10 is empty, for example, "There is no remaining count" is displayed in red, and only cartridge replacement may be allowed.

In "6" in Fig. 30A, the usage expiration date of the cartridge 10 is displayed, and, for example, 30 days before the cartridge expiration date, "6" is displayed in red and/or blinking. Furthermore, in a case where the cartridge 10 has expired, "replace the cartridge" is displayed in red with the usage expiration date, and only cartridge replacement may be allowed. In "7" in Fig. 30A, the tester can be selected from the pulldown. For example, up to 10 people can be registered, and 4 people who are frequently responsible of acting as tester are displayed at the top of the list. Note that a tester can be registered as desired from the "settings".

In "8" in Fig. 30A, the subject ID can be input. Furthermore, it is possible to read, by tapping a left camera icon, a barcode, a two-dimensional matrix barcode, or the like using a camera provided in the tablet terminal 301. "9" in Fig. 30A is designed not to respond to tapping in a case where the status of the odor generation device 1 is other than "normal". In this case, a measurement start button may be lighter in color. Furthermore, in a case where the subject ID or the tester has not been input, it is also designed not to respond to tapping. Note that information (for example, the subject ID, the remarks, and the like) illustrated in Fig. 30A may be passed to a measurement screen.

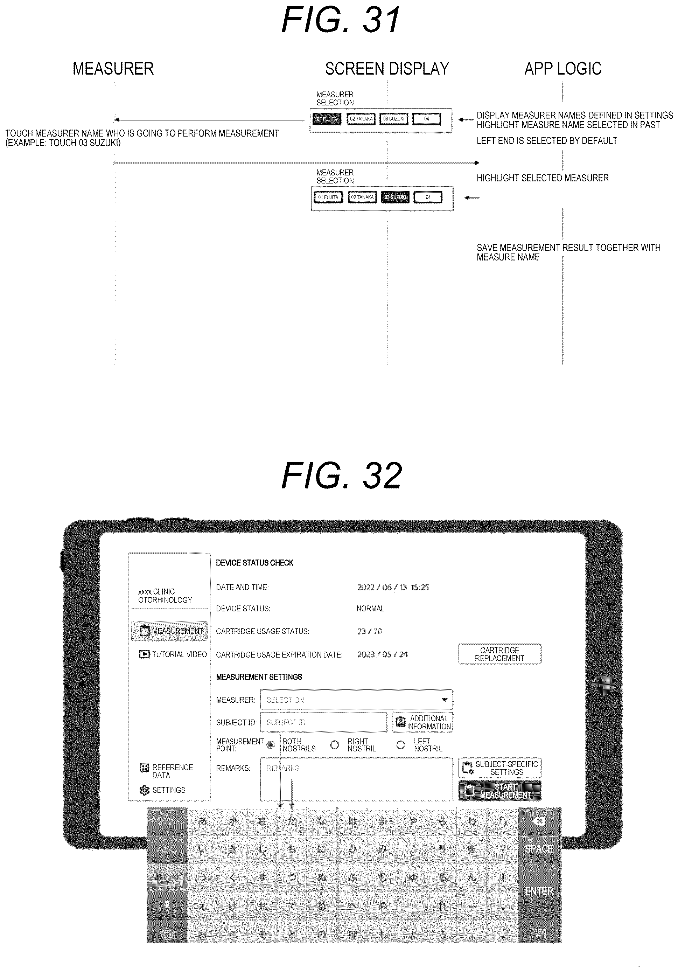

Fig. 31 is a diagram for describing behavior when selecting a measurer. First, on the display screen, the name of the measurer defined in the settings is displayed. On this screen, a measurer selected in the past may be highlighted (for example, the background is blue, the characters are bold white, or the like), and a measurer at the left end may be selected by default. On the other hand, when the measurer or the like taps the name of the measurer who performs measurement from now on (for example, taps 03: Suzuki), the selected measurer is highlighted. Then, the name of the measurer is stored with the name associated with the measurement result.

Fig. 32 is a diagram illustrating an example of the embodiment of the measurement preparation screen. Specifically, the diagram illustrates how the subject ID and the remarks are input on the measurement preparation screen. For the subject ID, when an entry field is tapped, a keyboard for character input appears on, for example, a lower side of the screen, an upper side of the screen, or the like, and the subject ID can be entered using the keyboard. Furthermore, for the remarks, when an entry field is tapped, the keyboard for character input appears on, for example, the lower side of the screen, the upper side of the screen, or the like, and the remarks can be entered using the keyboard. Such entries can be made while the screen illustrated in Fig. 30A is displayed. Furthermore, the keyboard may support not only Japanese but also English or the like. As described above, the subject ID and the remarks are each passed to the measurement screen. Furthermore, in "additional information" on the right side of the subject ID, information such as gender, age, and a medical history can be input. When one-time measurement settings is tapped on this screen, a screen transition is made from "settings" to "one-time measurement settings", and when the measurement of the subject ends, return to default settings.

### (2-2) Free measurement screen

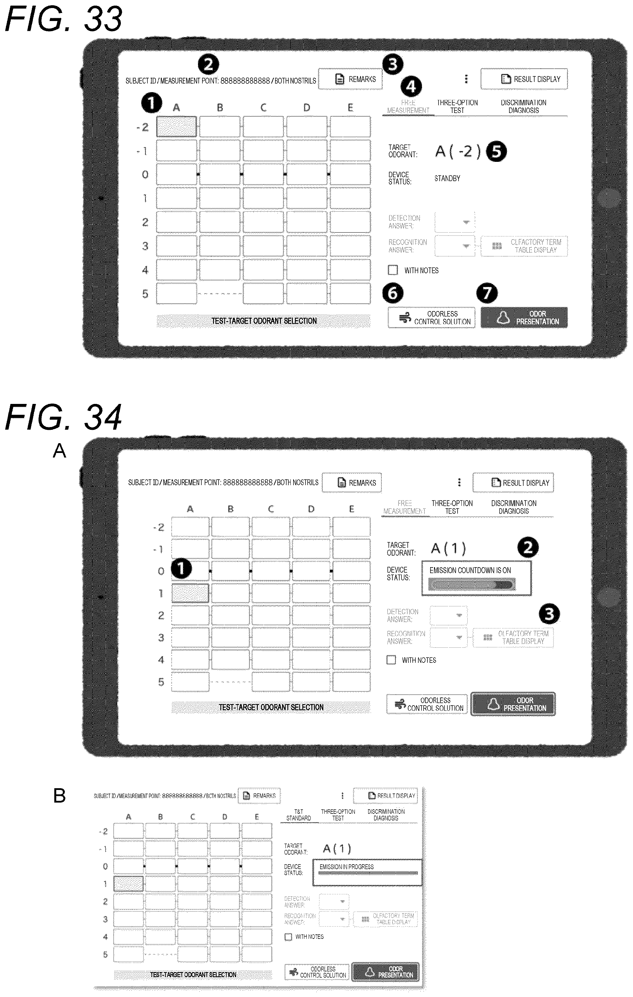

Fig. 33 is a diagram illustrating an example of an embodiment of the free measurement screen. When the start measurement is tapped on the above-described measurement preparation screen, a screen transition is made to a screen illustrated in Fig. 33. In "1" in Fig. 33, an odor type, an odor concentration, and the like can be selected, and when selected, the corresponding cell is highlighted. Furthermore, in response to the above, in "5" in Fig. 33, the selected odorant number (for example, A-2, A1, or the like) is reflected and displayed as a target odorant. To "2" in Fig. 33, the information such as the subject ID and the measurement point entered on the above-described measurement preparation screen is passed. Tapping such icons makes it possible to modify or input contents such as the subject ID and the measurement point even on the screen illustrated in Fig. 33.

In "3" in Fig. 33, when the remarks is tapped, information regarding the remarks entered on the above-described measurement preparation screen is displayed, and the remarks can also be modified or input on this screen. In "4" in Fig. 33, measurement workflow is displayed. The measurement workflow may include, for example, three types: (i) free measurement, (ii) three-option test, and (iii) discrimination diagnosis, and the free measurement may be highlighted by default. In "6" in Fig. 33, presentation of an odorless control solution can be selected. Note that in a case where it is desired to emit the odorless control solution, the odorless control solution is emitted by tapping an odorless control solution button and then tapping an odor presentation button. In "7" in Fig. 33, when the odor presentation button is tapped, a countdown automatically starts, and then the odor component is emitted from the odor generation device 1.

Fig. 34 is a diagram illustrating an example of the embodiment of the free measurement screen. Specifically, a state where when the odor presentation button in "7" in Fig. 33 is tapped, the countdown is automatically performed, and then the odor is presented is illustrated. On this screen, in an odor presentation state (during the countdown or emission of odor component-containing air), all the commands other than the odor presentation button can be set inactive. As illustrated in "1" in Fig. 34A, characters representing the type and concentration of the odor being presented may be highlighted. As illustrated in "2" in Fig. 34A, when the odor presentation button is tapped, an emission countdown and a status are displayed. In this case, such a display may be in conjunction with a voice guide, and, for example, an announcement like "3, 2, 1, sniff it." may be made.

In "3" in Fig. 34A, during the odor presentation, the olfactory term table display is grayed out to be inactive (unselectable). Then, for example, only the detection answer may become active after the end of foursecond odor presentation. Fig. 34B is a display screen during the emission of the odor component-containing air. For example, in a case where the default is set to four seconds, the odor component-containing air continuously emitted for four seconds, a cage corresponding to the four seconds is prepared. When the odor presentation button in Fig. 34A is tapped, as described above, the odor component-containing air is emitted from the odor generation device 1 after the end of the countdown. When the odor is presented, the active state is visualized as illustrated in Fig. 34B. Then, a stop operation can be performed by tapping the odor presentation button again.

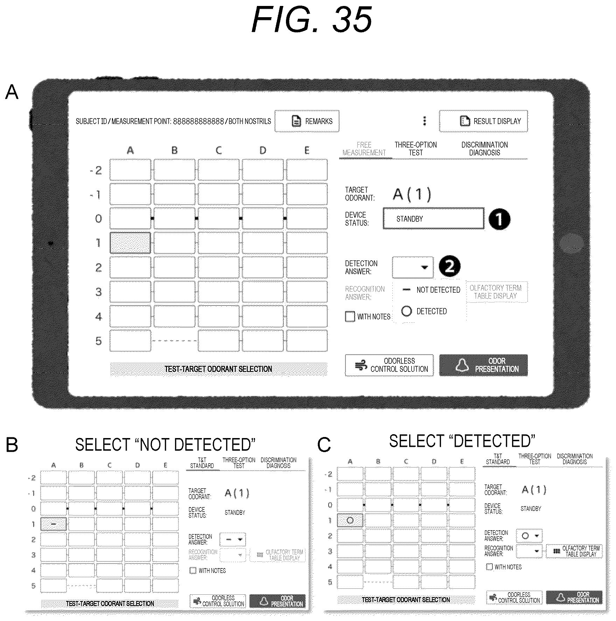

Fig. 35 is a diagram illustrating an example of the embodiment of the free measurement screen. Specifically, a state where a detection answer is selected after the end of odor presentation is illustrated. On this screen, all the commands other than the detection answer can be set inactive. In "1" in Fig. 35A, it can be confirmed that the device status has returned to a standby status after the end of odor presentation. "2" in Fig. 35A is a portion where the answer result of the detection answer is input, and, for example, either "-: not detect" or "∘: detected" can be selected from the pulldown. The selected answer result is reflected in the frame of the corresponding odorant as illustrated in Fig. 35B or 35C.

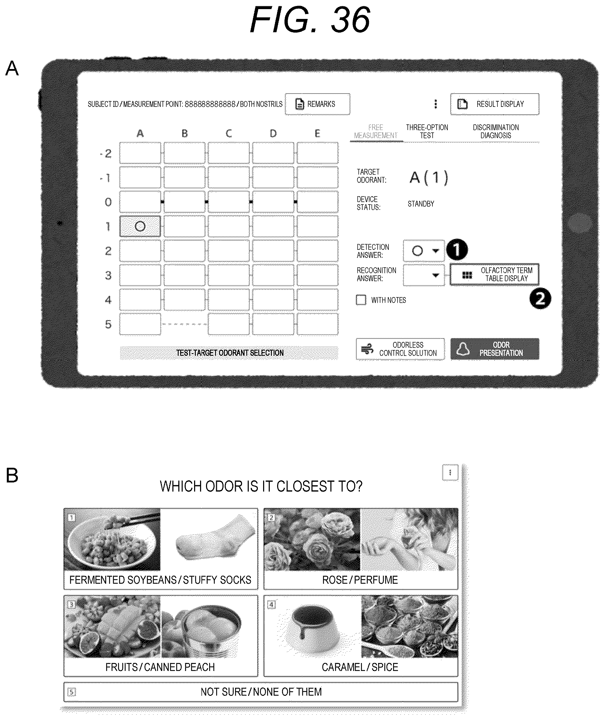

Fig. 36 is a diagram illustrating an example of the embodiment of the free measurement screen. Specifically, a state after the detection answer is illustrated. For example, when the detection answer is "∘: detected", the recognition answer and the olfactory term table display become active. In "1" in Fig. 36A, the recognition answer becomes active when the detection answer becomes "o: detected", and it is possible to select either "-: not recognized" or "o: recognized" from the pulldown, for example. In "2" in Fig. 36A, the olfactory term table display as illustrated in Fig. 36B becomes active when the detection answer becomes "∘: detected", and when the olfactory term table is tapped, the corresponding olfactory term table is displayed on the basis of the odor type, the odor concentration, and the like.

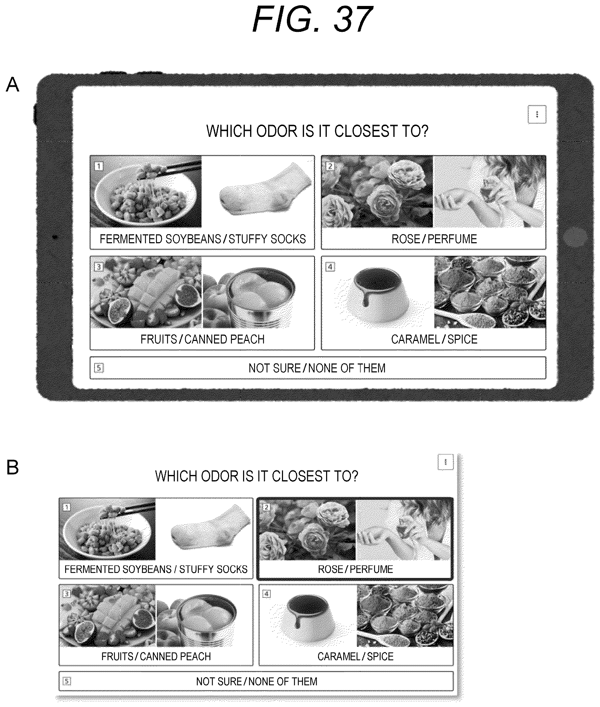

Fig. 37 is a diagram illustrating an example of the embodiment of the free measurement screen. Specifically, a screen transition after selecting A(-2) as an odorant and tapping the olfactory term table is illustrated. A display pattern (combination) varies in a manner that depends on the odor type, the odor concentration, or the like. In Fig. 37A, there are a total of five options including [not sure] in addition to four options of odor expressions [fermented soybeans/stuffy socks, rose/perfume, fruits/canned peach, caramel/spice], and the subject selects from the five options. By default, when selection is made by tapping without highlighting, the selected item may be highlighted as illustrated in Fig. 37B. Furthermore, the position of the correct option can be displayed at random each time except for [not sure].

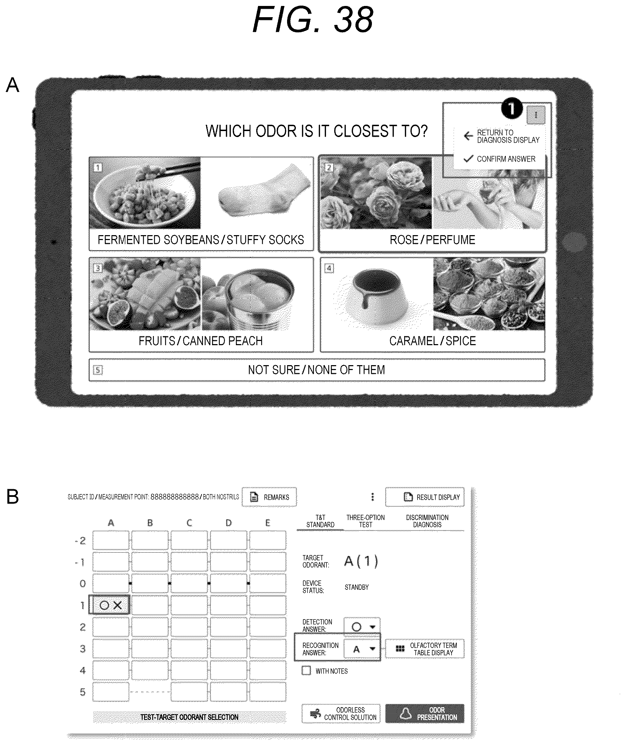

Fig. 38 is a diagram illustrating an example of the embodiment of the free measurement screen. Specifically, a state after any desired option is selected from the olfactory term table is illustrated. In "1" in Fig. 38A, when a "..." icon is tapped, two options of "return to diagnosis screen" and "confirm answer" are made selectable. Fig. 38B is a screen after tapping the "confirm answer" on the olfactory term table (for example, a screen in a case where the answer is correct). The "..." icon is always active regardless of the presence or absence of the answer, and in a case where "return to diagnosis screen" is selected, the screen returns to the screen in Fig. 36A (after the detection answer). On the other hand, in a case where the "confirm answer" is selected, the answer result ("×: recognized" or "-: not recognized") is input on the test screen in response to the selection. In the recognition answer field, a symbol of the selected olfactory term table is reflected.

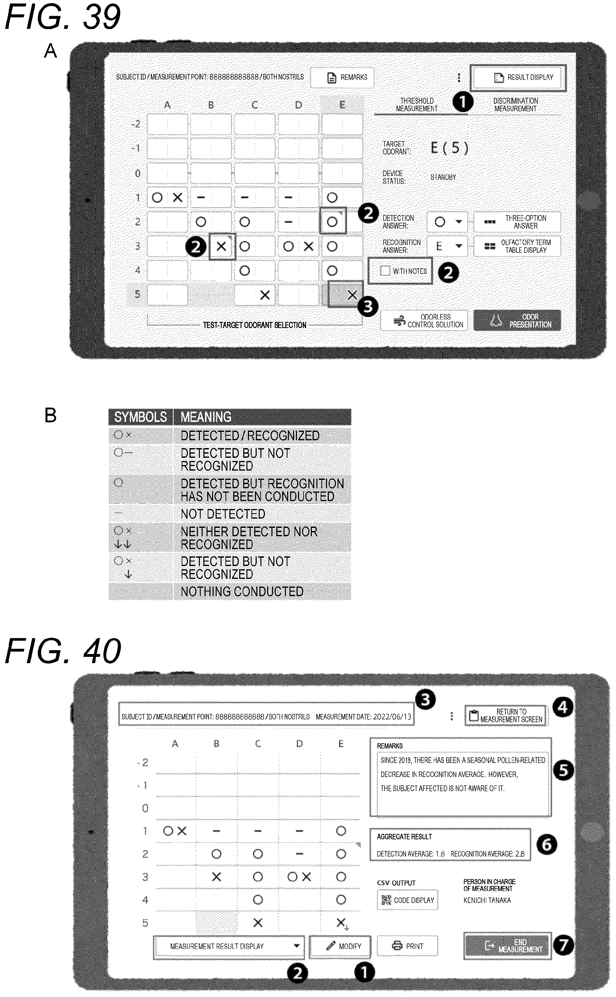

Fig. 39 is a diagram illustrating an example of the embodiment of the free measurement screen. Specifically, a result display pattern during the free measurement is illustrated. In "1" in Fig. 39A, tapping the result display enables selection of a result output pattern. In "2" in Fig. 39A, when a checkbox of with notes is tapped with the odorant selected, a check mark appears, and a predetermined mark (for example, an indicator or the like displayed at a corner of the result input area) is input in the result display portion (for example, E2, B3) described on the left. In "3" in Fig. 39A, "∘↓" or "×↓" is displayed in a case where the most concentrated odorant (that is, A5, B4, C5, D5, or E5) of each odorant (A to E) cannot be detected or cannot be recognized. Note that "↓" is displayed below "∘" or "×", for example. Fig. 39B illustrates each symbol and its meaning.

Fig. 40 is a diagram illustrating an example of the embodiment of the free measurement screen. Specifically, a screen state after tapping the result display in Fig. 39 is illustrated. Note that the free measurement ends on this screen. In "1" in Fig. 40, the measurement result, the subject ID, the date, the tester, the remarks, or the like can be modified. Note that the measurement ID may be freely input. Furthermore, the answer portion and the measurer may be pulled down. In "2" in Fig. 40, by pulling down, it is possible to confirm an acquisition result of five items (for example, a measurement result display, an olfactogram display, a patient answer display, a discrimination test result display, and a list display). In "3" in Fig. 40, the subject ID, the measurement point, and the measurement date are passed from the initial screen of the free measurement and displayed. Furthermore, in "4" in Fig. 40, tapping the "return to measurement screen" makes it possible to return to the continuation of the free measurement.

In a manner similar to "3", "5" in Fig. 40 displays the information passed from the initial screen of the free measurement. In "6" in Fig. 40, a detection average and a recognition average are calculated from the respective levels of the detection threshold and the recognition threshold and displayed. When the denominator of the average is of each average is less than 5 (in a case where there is no measurement of any one of the items), an asterisk may be displayed at the upper right of the average. Specifically, for example, detection average: 1.6* and recognition average: 2.8* may be displayed. In "7" in Fig. 40, when end measurement is tapped, the free measurement ends, and the measurement result is saved. Then, after the end of measurement, the odor generation device 1 preferably performs a ventilation operation for a predetermined time (for example, about 1 minute).

### (2-3) Result display screen

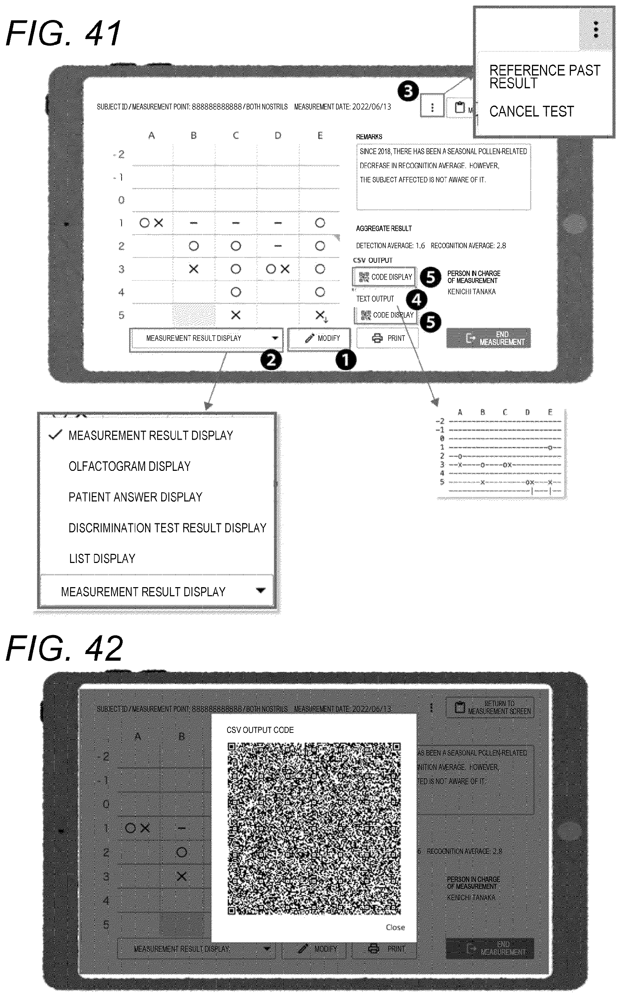

Fig. 41 is a diagram illustrating an example of an embodiment of a result display screen. Specifically, a state where the result is referenced is illustrated. In "1" in Fig. 41, the result can be modified, and the answer portion can be pulled down. In "2" in Fig. 41, by pulling down, five items (for example, the measurement result display, the olfactogram display, the patient answer display, the discrimination test result display, and the list display) are displayed, and it is possible to select, from among the items, an item whose result is desired to be displayed. Note that the measurement result may be displayed by default. In "3" in Fig. 41, when an icon represented by vertical ellipsis points is tapped, reference past result or cancel test is made selectable, and in a case where the cancel test is selected, the result is not saved. In "4" in Fig. 41, a barcode for text output is displayed, and when reading the barcode, an ASCII art text of an olfactogram is displayed as described above. In "5" in Fig. 41, when code display is tapped, data in a CSV or text format is displayed as a two-dimensional matrix barcode.

Fig. 42 is a diagram for describing a state where when the code display is tapped, a CSV output code is displayed as a two-dimensional matrix barcode. In this case, in order to highlight the output code, the background may be grayed out, and the output code may be popped up. Furthermore, the user can return to the previous screen by tapping "Close" at the lower right.

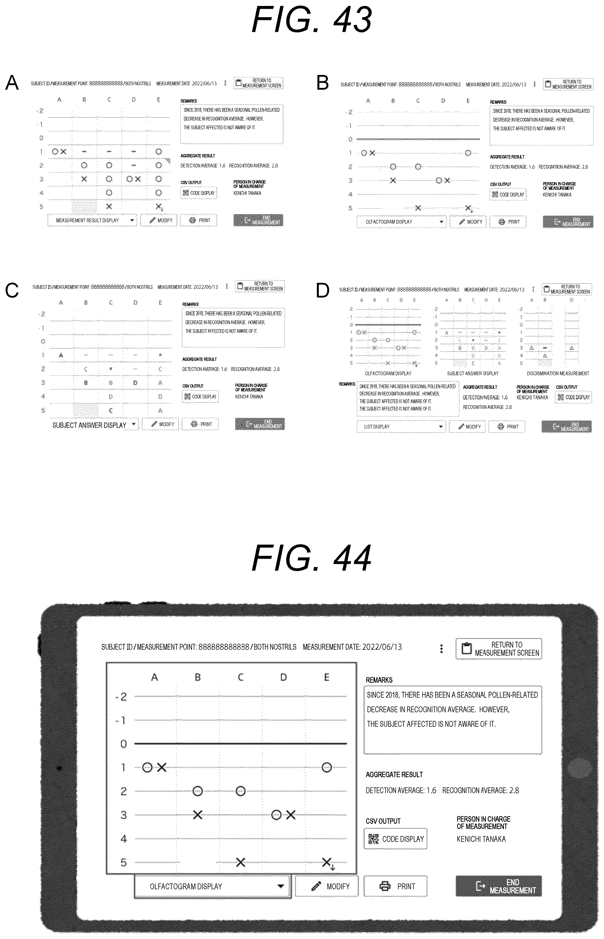

Figs. 43 is a diagram illustrating an example of an embodiment of the four measurement result displays including the measurement result display, the olfactogram display, the patient answer display, and the list display. Fig. 43A is the measurement result display in which input information displayed during measurement is displayed. Fig. 43B is the olfactogram display in which "o: detection threshold" and "×: recognition threshold" are each displayed only at an area corresponding to the threshold concentration of each odor type. Fig. 43C is the subject answer display in which details of the answer of the subject in the detection test and the recognition test are displayed. Furthermore, although not illustrated here, the result of the discrimination test is displayed in the discrimination result display. Fig. 43D is the list display in which the olfactogram display, the subject answer display, and the discrimination result display are displayed on one screen.

Fig. 44 is a diagram illustrating an example of an embodiment of the olfactogram result display. That is, the lower left tab is set to the "olfactogram display". In the olfactogram, "o: detection threshold" and "×: recognition threshold" are displayed on a line.

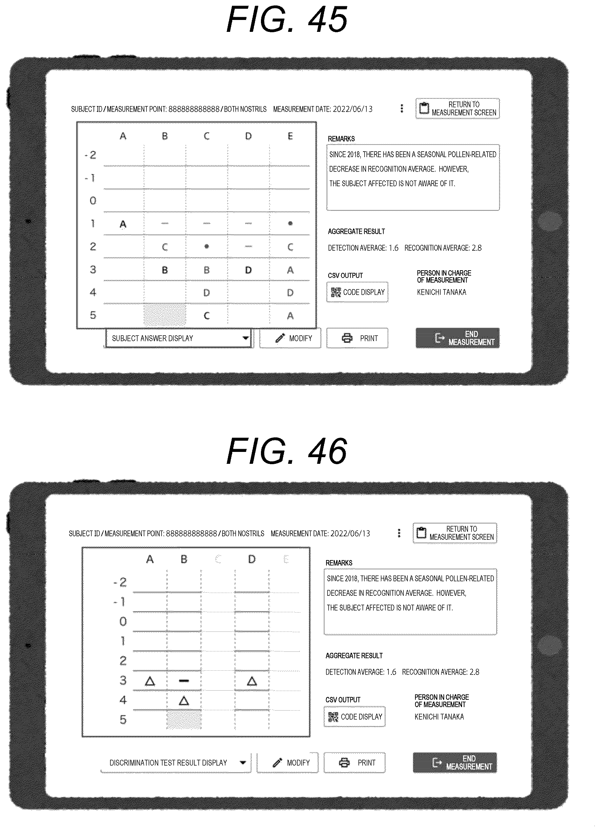

Fig. 45 is a diagram illustrating an example of an embodiment of the subject answer result display. That is, the lower left tab is set to the "subject answer display". Here, the answer result of the recognition threshold test is displayed, and, for example, in a case of a correct answer, the answer result can be displayed with a black alphabet character, and in a case of an incorrect answer, the answer result can be displayed with a red alphabet character or "·" (not sure). Furthermore, "-" indicates an odorant that has not been detected. Note that the display of the black alphabet character or "." indicates that the detection itself has been completed.

Fig. 46 is a diagram illustrating an example of an embodiment of the discrimination result display. That is, the lower left tab is set to the "discrimination result display". For example, in a case of success in discrimination, the discrimination result can be displayed as "△", and in a case of failure in discrimination, the discrimination result can be displayed as "-".

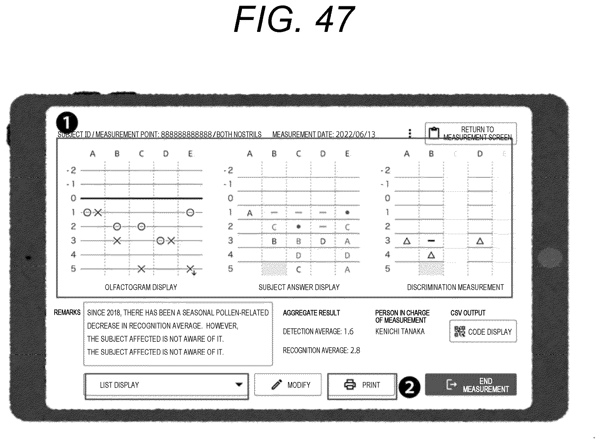

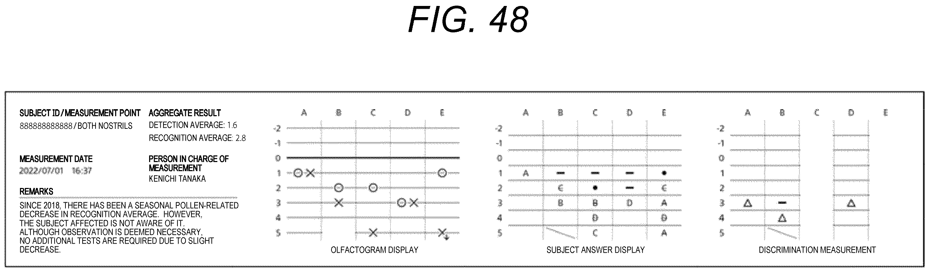

Fig. 47 is a diagram illustrating an example of an embodiment of the list display. That is, the lower left tab is set to the "list display". In "1" in Fig. 47, the olfactogram, the subject answer, and the discrimination measurement are displayed as a result list. In "2" in Fig. 47, when a "print" icon is tapped, the result of the list display can be printed out in a format as illustrated in Fig. 48.

### (2-4) Screen after end of measurement

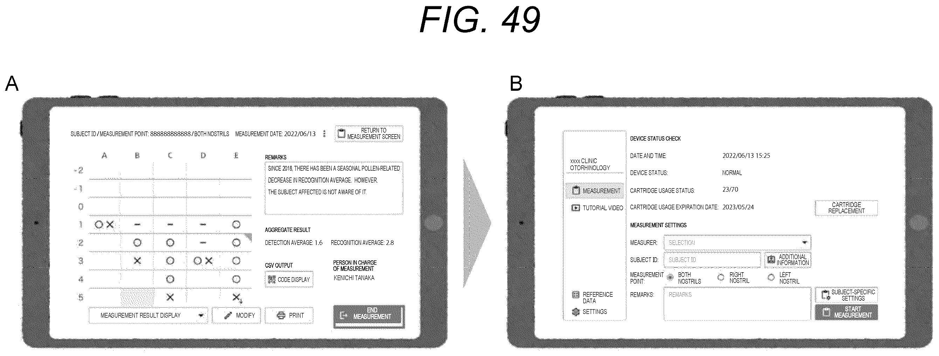

Fig. 49 is a diagram illustrating an example of an embodiment of a screen after the end of measurement. As illustrated in Fig. 49A, when end measurement is tapped, the measurement ends, and the result is saved. The save format is not particularly limited, and for example, CSV, JPG, or the like can be used. Note that the timing at which the measurement is completed is the original. Thereafter, as illustrated in Fig. 49B, the screen returns to the measurement preparation screen.

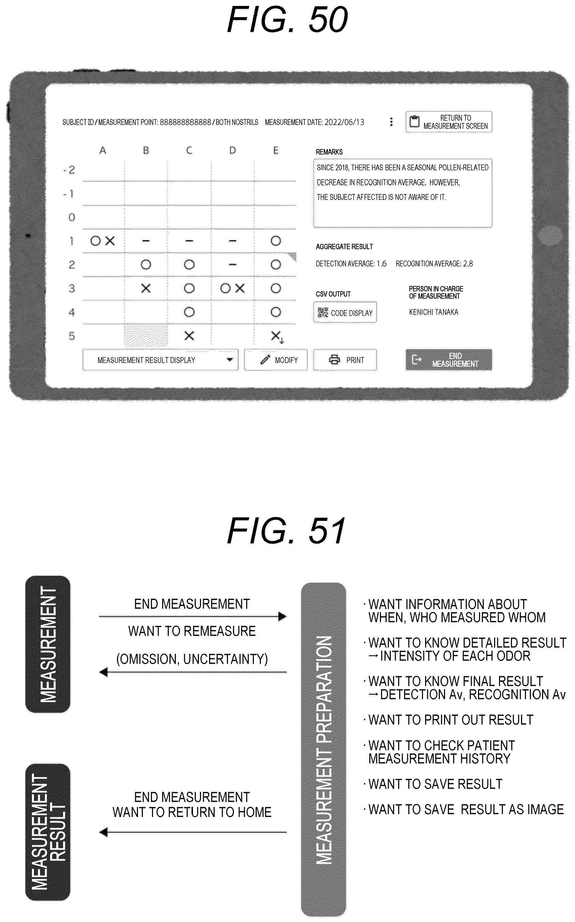

Fig. 50 is a diagram illustrating an example of the embodiment of the screen after the end of measurement. Specifically, a state where a transition is made to the result display screen after the end of measurement is illustrated. Here, a CSV file and a JPG file are saved with the same file name. Furthermore, when an "end measurement" icon is tapped, it is preferable to ask the measurer whether or not the measurer really want to end the measurement with a pop-up message. In the embodiment illustrated in Fig. 50, only when the measurement ends, a transition to the result display screen is made. That is, as described above, a button or the like for browsing the result on the way is not displayed. A state of the screen transition in this case is illustrated in Fig. 51.

### (2-5) History screen

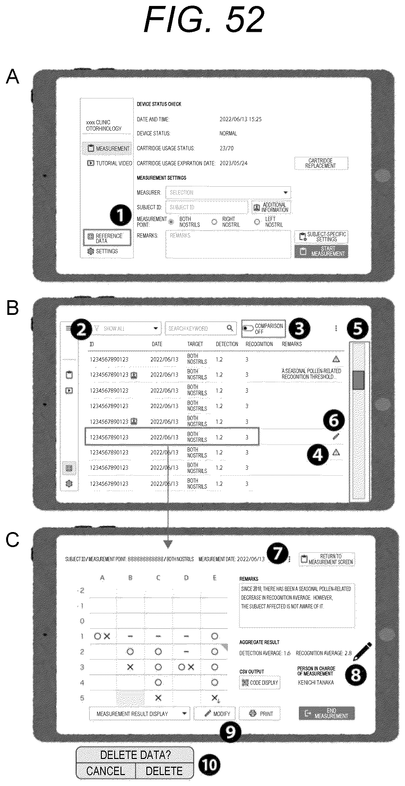

Fig. 52 is a diagram illustrating an example of an embodiment of a history screen. Specifically, on the measurement preparation screen, as illustrated in "1" in Fig. 52A, when reference data is tapped, the result history can be viewed. In "2" in Fig. 52B, the history can be filtered by ID, date, measurer, or the like. In a case where a transition is made from the free management screen tab during measurement, the ID may be displayed with the ID sorted by the ID of the measurer whose measurement is in progress. In "3" in Fig. 52B, the display of a comparison display screen can be switched to ON/OFF. "4" in Fig. 52B is an icon indicating that the result has been modified, and when this icon is tapped, the original result is displayed. In "5" in Fig. 52B, vertical scrolling up to a registered row can be made. In "6" in Fig. 52B, when a predetermined result is selected, a transition is made to Fig. 52C, and a more detailed result can be displayed.

In "7" in Fig. 52C, an icon represented by vertical ellipsis points is tapped, each condition during measurement is displayed. Therefore, the user need not perform printing or the like in order to refer to each condition. Examples of the condition include whether or not to use the three-option detection, whether or not to use the olfactory term table, an odorant presentation order, each item of the one-time measurement settings (for example, LED on/off, voice guide on/off, output time, discrimination olfactory selection, and the like), and ID information of the cartridge 10. "8" in Fig. 52C is an icon indicating that modification has been made, and the original result can be displayed by tapping this icon. In "9" in Fig. 52C, after the modification, the original can be left in another CSV, and the contents after the change can be displayed from the history. In "10" in Fig. 52C, when a delete button is pressed, a pop-up message is displayed, and when "cancel" is tapped, the screen returns to the measurement management screen. On the other hand, when "delete?" is tapped, data is deleted.

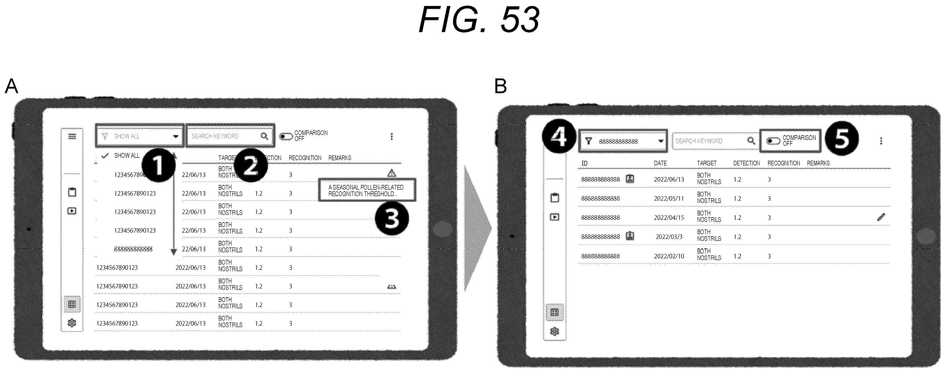

Fig. 53 is a diagram illustrating an example of the embodiment of the history screen. Specifically, a state where a filter function is operated is illustrated. In "1" in Fig. 53A, when a show all portion is pulled down, a subject ID list is displayed. The subject IDs are displayed in numerical order, and scrolling is also possible here. In "2" in Fig. 53A, an input field for search keyword is displayed, and here, for example, a search with the subject ID, a search with the ID of the cartridge 10, a text search (for example, through the remarks field or the like), and the like can be performed. A configuration where at this time, as described above, when the search keyword portion is tapped, a keyboard for inputting characters appears on the lower side of the screen, the upper side of the screen, or the like, and a keyword can be entered using the keyboard may be employed. In "3" in Fig. 53A, in a case where the number of characters in the remarks field is large, a pop-up message may be displayed by long-pressing the remarks to make the entire text readable. When a subject ID is selected, a transition is made to Fig. 53B, and a list of measurement results of the corresponding subject ID is displayed as illustrated in "4" in Fig. 53B. In "5" in Fig. 53B, when comparison is switched to ON, a comparison between a plurality of measurement results is enabled.

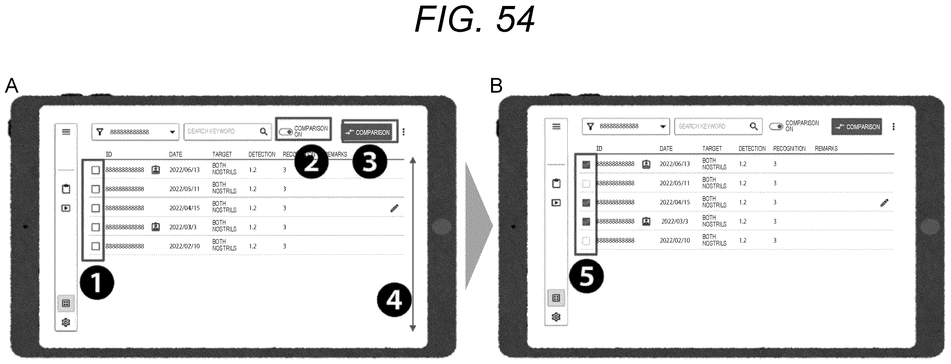

Fig. 54 is a diagram illustrating an example of the embodiment of the history screen. Specifically, a state where a plurality of comparisons is switched to ON is illustrated. In "1" in Fig. 54A, by default, a checkbox is OFF, and up to three checkboxes can be selected. In "2" in Fig. 54, for example, a state where the comparison is ON is highlighted. In "3" in Fig. 54, when the "comparison" is ON, the highlighted icon and the checkbox on the left of each ID are displayed. In "4" in Fig. 54A, it is possible to scroll through a plurality of histories. Furthermore, when a checkbox is checked, a transition is made to Fig. 54B, and the checked portion is highlighted as illustrated in "5" in Fig. 54B. In this case, for example, up to three subject IDs may be selected. Then, when the maximum number of subject IDs are selected, the other checkboxes may be grayed out to be unselectable. On the other hand, when some checkboxes are disabled and the number of enabled checkboxes becomes, for example, less than or equal to 2, the other checkboxes may become black to be selectable.

### (2-6) Comparison display screen

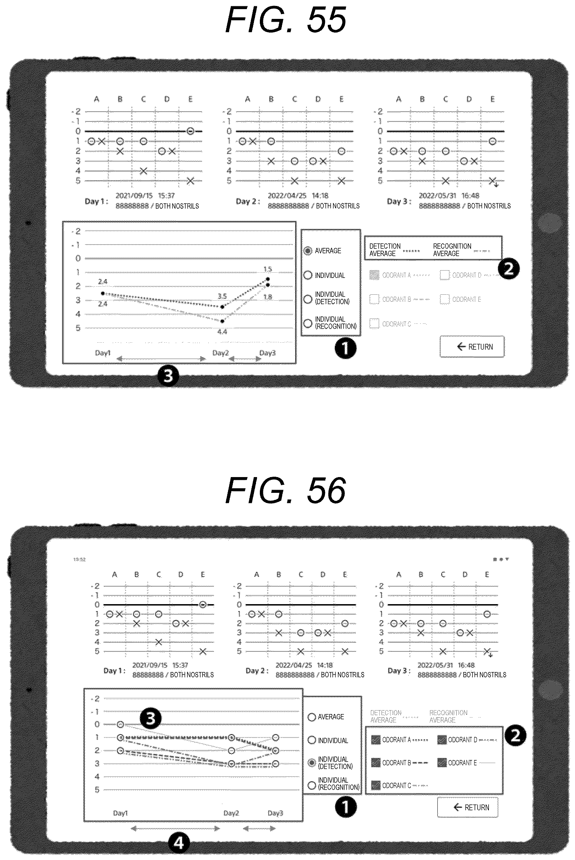

Fig. 55 is a diagram illustrating an example of an embodiment of the comparison display screen. In "1" in Fig. 55, any radio button of average, individual, individual (detection), and individual (recognition) can be switched to ON. Here, when the average is selected, a detection threshold average and a recognition threshold average are displayed in a line graph. In "2" in Fig. 55, lines corresponding to the detection average and the recognition average are displayed, and these lines are reflected in a graph on the left. In "3" in Fig. 55, the detection threshold average and the recognition threshold average are each displayed as a line graph, and intervals between Day1 and Day3 reflect intervals between the actual measurement dates as a ratio.

Fig. 56 is a diagram illustrating an example of the embodiment of the comparison display screen. Specifically, a state where the detection threshold of each odorant is visualized is illustrated. In "1" in Fig. 56, as described above, any radio button of the average, the individual, the individual (detection), and the individual (recognition) can be switched to ON. Here, when the individual (detection) is selected, a detection threshold level is displayed in a line graph according to the odorant type. In "2" in Fig. 56, the checkbox of each of the odorants A to E can be switched to ON/OFF as desired. For example, up to five odorants may be selectable, and a line corresponding to each odorant is displayed, and these lines are reflected in the graph described above. In "3" in Fig. 56, the detection can be displayed with a line having a color corresponding to each odorant type. For example, "∘" represents detection, and lines overlapping each other are slightly shifted in position and displayed. This makes all the odorant lines visible. In "4" in Fig. 56, the intervals between Day1 and Day3 reflect the intervals between the actual measurement dates as a ratio.

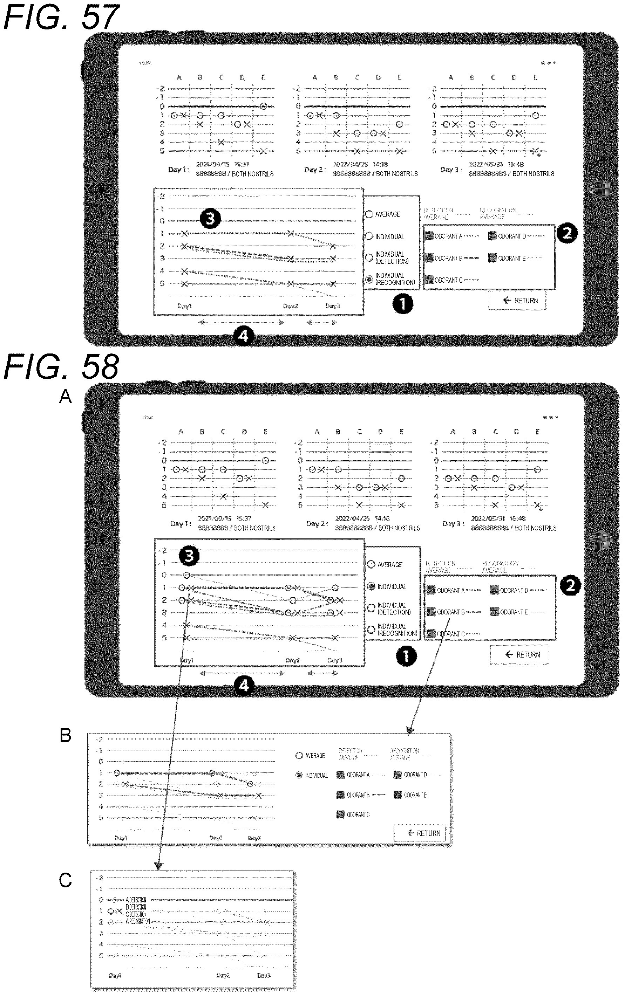

Fig. 57 is a diagram illustrating an example of the embodiment of the comparison display screen. Specifically, a state where the recognition threshold of each odorant is visualized is illustrated. In "1" in Fig. 57, as described above, any radio button of the average, the individual, the individual (detection), and the individual (recognition) can be switched to ON. Here, when the individual (recognition) is selected, a recognition threshold level is displayed in a line graph according to the odorant type. In "2" in Fig. 57, the checkbox of each of the odorants A to E can be switched to ON/OFF as desired. For example, up to five odorants may be selectable, and a line corresponding to each odorant is displayed, and these lines are reflected in the graph described above. In "3" in Fig. 57, the recognition can be displayed with a line having a color corresponding to each odorant type. For example, "×" represents recognition, and lines overlapping each other are slightly shifted in position and displayed. This makes all the odorant lines visible. In "4" in Fig. 57, the intervals between Day1 and Day3 reflect the intervals between the actual measurement dates as a ratio.

Fig. 58 is a diagram illustrating an example of the embodiment of the comparison display screen. Specifically, a state where the detection threshold and the recognition threshold of each odorant are visualized is illustrated. In "1" in Fig. 58A, as described above, any radio button of the average, the individual, the individual (detection), and the individual (recognition) can be switched to ON. Here, when the individual is selected, the detection threshold level and the recognition threshold level are displayed in a line graph according to the odorant type. In "2" in Fig. 58A, the checkbox of each of the odorants A to E can be switched to ON/OFF as desired. For example, up to five odorants may be selectable, and a line corresponding to each odorant is displayed, and these lines are reflected in the graph described above. Here, when the line portion is long-pressed, as illustrated in Fig. 58B, an odorant with a checkmark that has not been long-pressed may be grayed out, and the line being long-pressed may be highlighted. In "3" in Fig. 58A, the detection and the recognition can be each displayed with a line having a color corresponding to each odorant type. For example, "∘" represents the detection, "×" represents the recognition, and lines overlapping each other are slightly shifted in position and displayed. This makes all the odorant lines visible. Furthermore, when each plot is long-pressed, the origin of "∘" and "×" is displayed as illustrated in Fig. 58C. In "4" in Fig. 58, the intervals between Day1 and Day3 reflect the intervals between the actual measurement dates as a ratio.

### (3) Example of detection threshold test screen flow

Hereinafter, an example of a detection threshold test screen flow will be described with reference to the drawings.

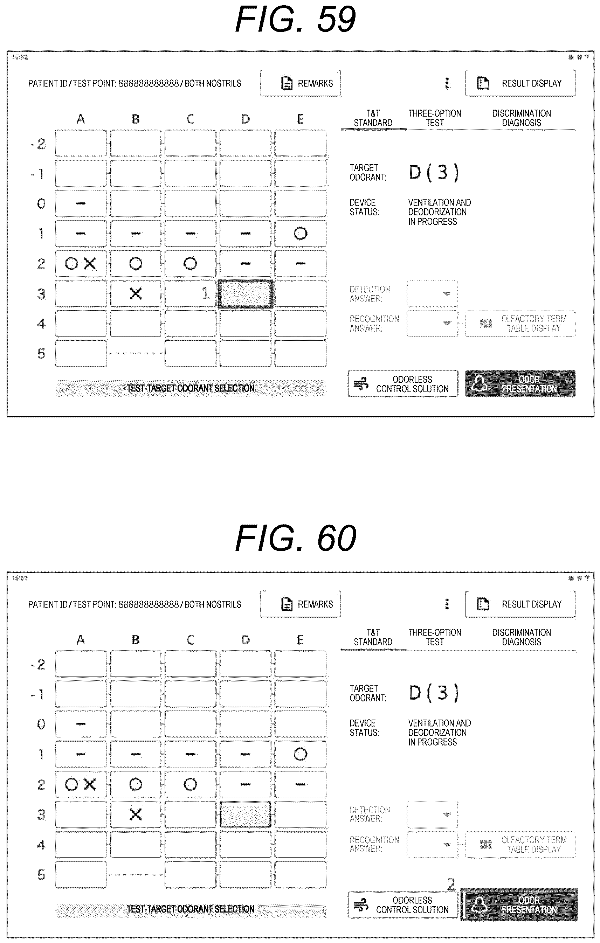

Fig. 59 is a diagram illustrating an example of an embodiment of an odorant selection screen in the detection threshold test screen. "1" in Fig. 59 illustrates, for example, a state where "D3" is selected as an odorant. As described above, the selected odorant may be highlighted in blue or the like so as to make the odorant easily visible to the user. Fig. 60 is a diagram illustrating an example of an embodiment of an odor presentation screen in the detection threshold test screen. In "2" in Fig. 60, after the odorant is selected in Fig. 59 and the odorant is highlighted, tapping the odor presentation button is enabled.

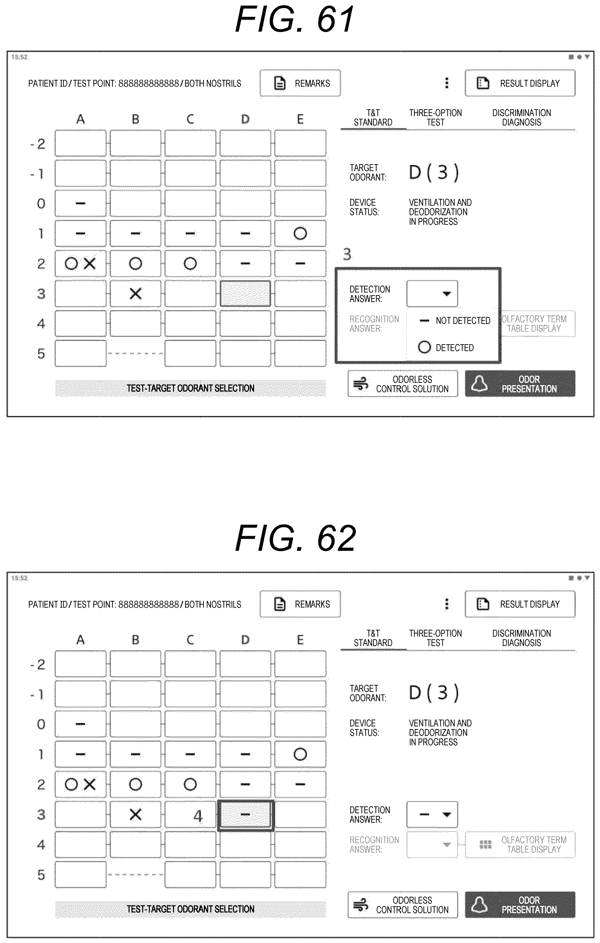

Fig. 61 is a diagram illustrating an example of an embodiment of a detection answer input screen in the detection threshold test screen. In "3" in Fig. 61, "-: not detected" or "∘: detected" can be selected as a detection answer from a pulldown, and the measurer (tester) selects either one. Fig. 62 is a diagram illustrating an example of an embodiment of a result reflection screen in the detection threshold test screen. In "4" in Fig. 62, the detection answer is "-: not detected" in "3" in Fig. 61 described above, so that "-" appears in the field of "D3" as the detection answer result. As described above, in the present embodiment, the measurement result is automatically reflected in the olfactogram.

### (4) Example of recognition threshold test screen flow

Hereinafter, an example of a recognition threshold test screen flow will be described with reference to the drawings.

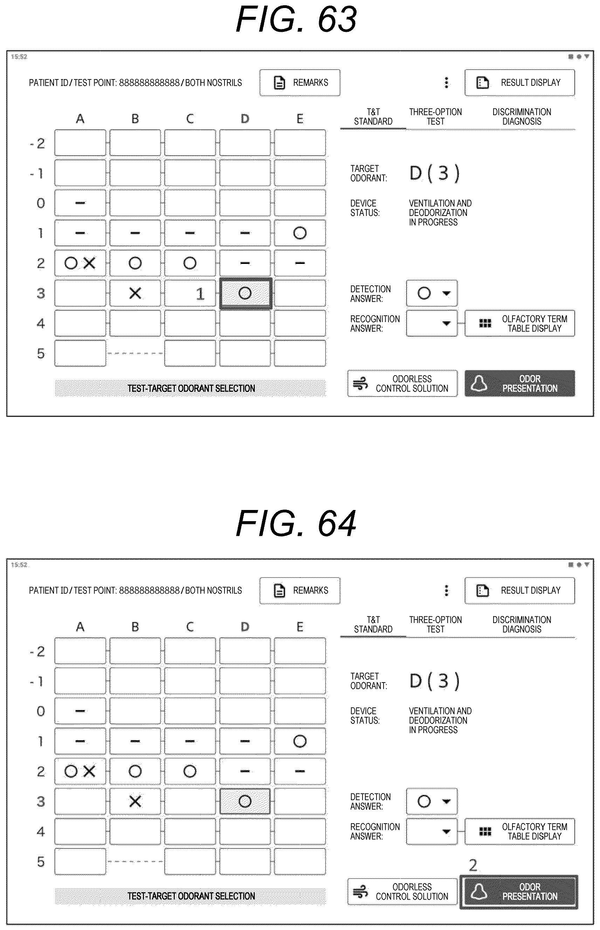

Fig. 63 is a diagram illustrating an example of an embodiment of an odorant selection screen in the recognition threshold test screen. "1" in Fig. 63 illustrates, for example, a state where "D3" is selected as an odorant. At this point, since the detection answer has already been obtained for the odorant "D3", "∘" has been input in the field of "D3". As described above, the selected odorant may be highlighted in blue or the like so as to make the odorant easily visible to the user. Fig. 64 is a diagram illustrating an example of an embodiment of an odor presentation screen in the recognition threshold test screen. In "2" in Fig. 64, after the odorant is selected by the measurer and the corresponding odorant is highlighted, tapping the odor presentation button is enabled.

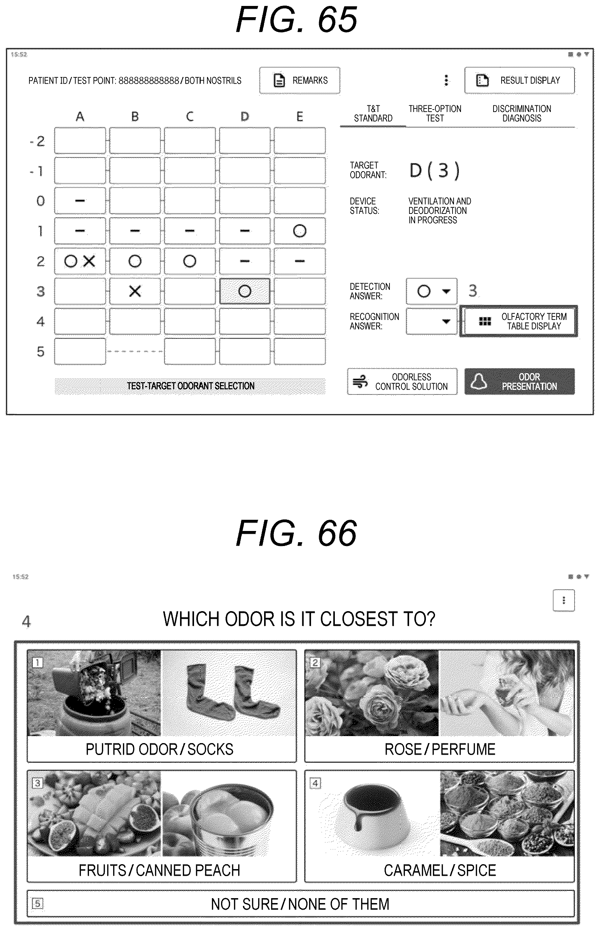

Fig. 65 is a diagram illustrating an example of an embodiment of an olfactory term table display screen in the recognition threshold test screen. In "3" in Fig. 65, tapping the olfactory term table display is enabled. Fig. 66 is a diagram illustrating an example of the embodiment of the olfactory term table display screen in the recognition threshold test screen. In "3" in Fig. 65, when the olfactory term table display is tapped, a transition is made to Fig. 66 to show a total of five options including [not sure/none of them] in addition to four options of odor expressions [putrid odor/socks, rose/perfume, fruits/canned peach, caramel/spice], and the subject selects from among the five options.

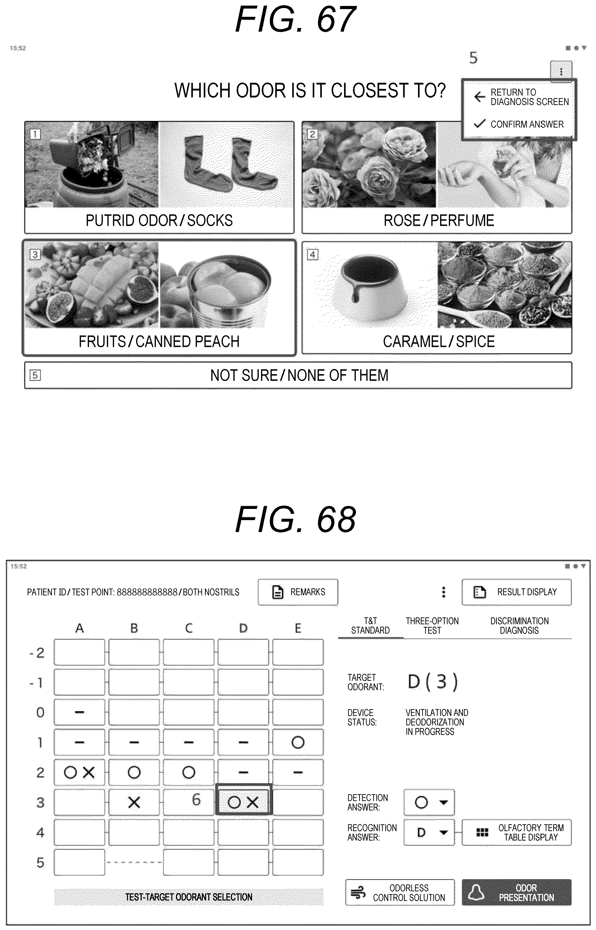

Fig. 67 is a diagram illustrating an example of an embodiment of an answer confirmation screen in the recognition threshold test screen. In Fig. 67, by default, in a case where any option is selected from among the five options without highlighting, the selected option may be highlighted. In "5" in Fig. 67, when icon represented by vertical ellipsis points is tapped, either "return to diagnosis screen" or "confirm answer" is made selectable. Fig. 68 is a diagram illustrating an example of the embodiment of the result reflection screen in the recognition threshold test screen. When the answer is confirmed in Fig. 67, a transition is made to Fig. 68, and in "6" in Fig. 68, the recognition answer is "×: recognized" on the screen in Fig. 67 described above, so that "×" appears in the field of "D3" as the recognition answer result. As described above, in the present embodiment, the measurement result is automatically reflected in the olfactogram.

### (5) Example of result screen of detection average and recognition average

Hereinafter, an example of a result screen of the detection average and the recognition average will be described with reference to the drawings.

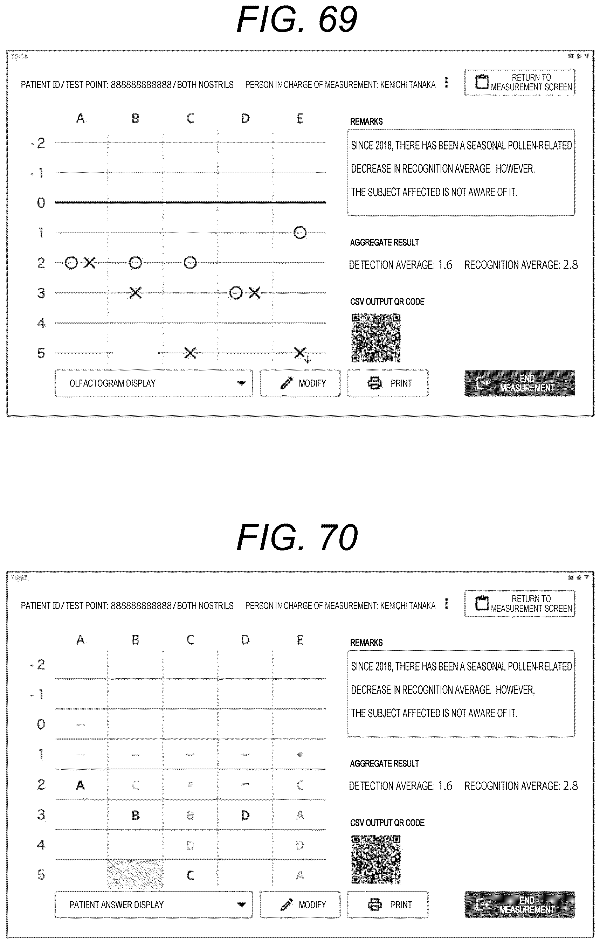

Fig. 69 is a diagram illustrating an example of an embodiment when the result screen of the detection average and the recognition average is displayed as an olfactogram. As described above, in the present embodiment, since the result of the detection average and the recognition average can be viewed in a list form from a higher perspective, it is possible to contribute to olfactory determination. Fig. 70 is a diagram illustrating an example of an embodiment when the result screen of the detection average and the recognition average is displayed as a patient answer. As described above, in the present embodiment, it is possible to confirm how the subject has answered and whether the result is correct or incorrect due to the color-coded display.

### (6) Example of three-option detection measurement flow

Hereinafter, an example of three-option detection measurement will be described with reference to the drawings.

### (6-1) Overview of three-option detection measurement

A rough description of the three-option detection measurement flow is as follows.

First, the measurer (tester) opens the free measurement screen described above and selects an odor component. Next, when the three-option detection button is pressed, the odor presentation button is automatically activated three times. Accordingly, the subject sniffs an odor three times. Then, the subject gives a verbal answer as to when the subject detects the odor in emitted odor component-containing air. The measurer inputs the answer result to the tablet terminal 301. Basically, the tablet terminal 301 is viewed only by the measurer, but may be temporarily shown to the subject.

### (6-2) Three-option detection measurement screen

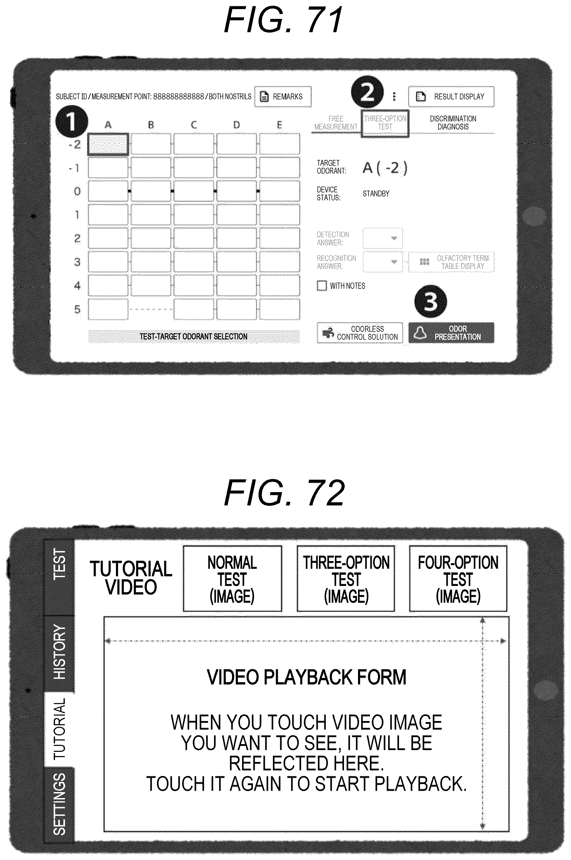

Fig. 71 is a diagram illustrating an example of an embodiment of a three-option detection measurement screen. First, in "1" in Fig. 71, an odorant is selected. Then, when three-option detection is selected in "2" in Fig. 71, the three-option detection is performed with the odorant selected in "1" in Fig. 71. In a case where the three-option detection is selected, the characters may be highlighted. Then, when the odor presentation button is selected in "3" in Fig. 71, the three options are displayed on the full screen as described later, and the presentation of the odorant is automatically started using the three options. Thereafter, after obtaining an answer from the subject, the measurer selects the answer. In "3" in Fig. 71, when the odor presentation button is tapped once, the state where the odor presentation button has been tapped is visible by means of highlighting or the like, and a transition is made to the next screen.

Figs. 72 to 84 are diagrams each illustrating an example of the embodiment of the three-option detection measurement screen. The overview of the three-option detection measurement has been described above, but the three-option detection measurement will be described below in more detail with reference to each screen. Note that the following description is on the assumption that the tablet terminal 301 is also shown to the subject.

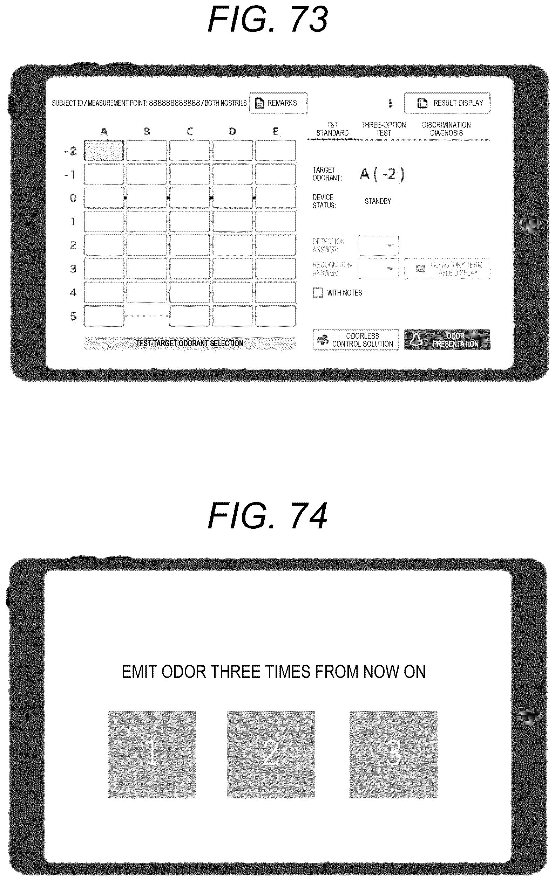

First, the measurer gives, to the subject, a verbal description of a procedure of the three-option detection measurement. Here, instead of the verbal description, the procedure may be described using a diagram or using a video as illustrated in Fig. 72. Next, as illustrated in Fig. 73, one type of odorant is selected on the basis of the initial concentration determined by the measurer. Here, the order in which the odor component-containing air is released among the three options is determined at random. Furthermore, the tablet terminal 301 is shown to the subject, and as illustrated in Fig. 74, and a message or voice guide like "Emit odor three times from now on" is given to inform the subject that the measurement is about to start".

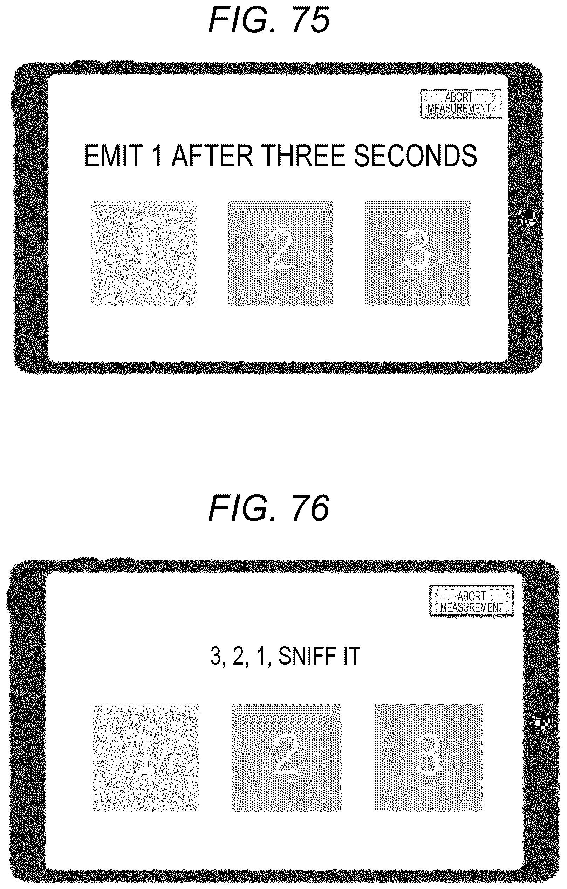

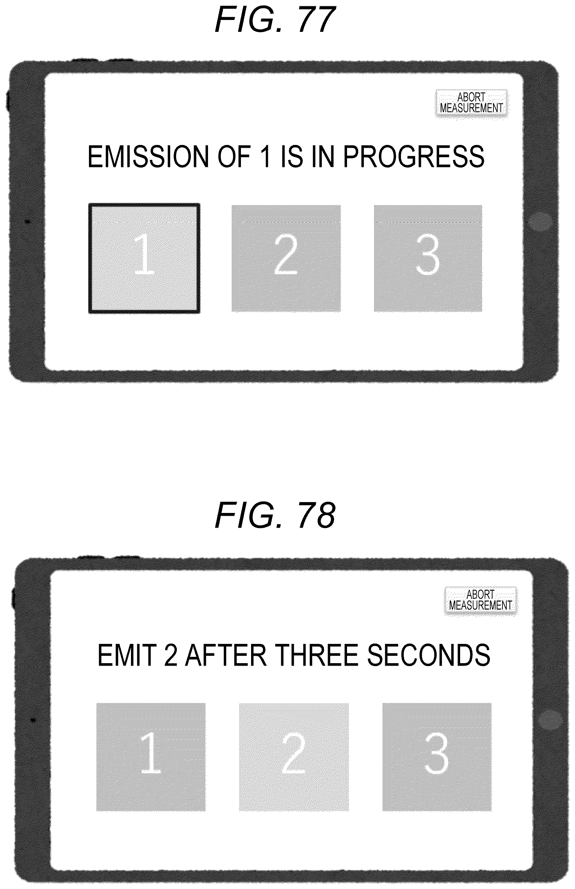

Next, the measurer taps the odor presentation button (start). Then, a three-second countdown starts. In order to make the countdown more eye-catching to the subject, as illustrated in Fig. 75, a clear message or voice guide indicating that the subject has to wait for three seconds, like "Emit 1 after three seconds", may be provided. Next, the odor component-containing air is emitted. Here, as illustrated in Fig. 76, a message or voice guide like "3, 2, 1, sniff it." may be provided. Fig. 77 is a screen displayed while the odor component-containing air is emitted for a certain period of time. During emission, for example, the number for which emission is in progress may be enclosed by a blue frame and highlighted for the sake of easy recognition.

Note that it is also possible to stop the emission of the odor component-containing air and return to the odorant selection screen by tapping a measurement abort button located at the upper right.

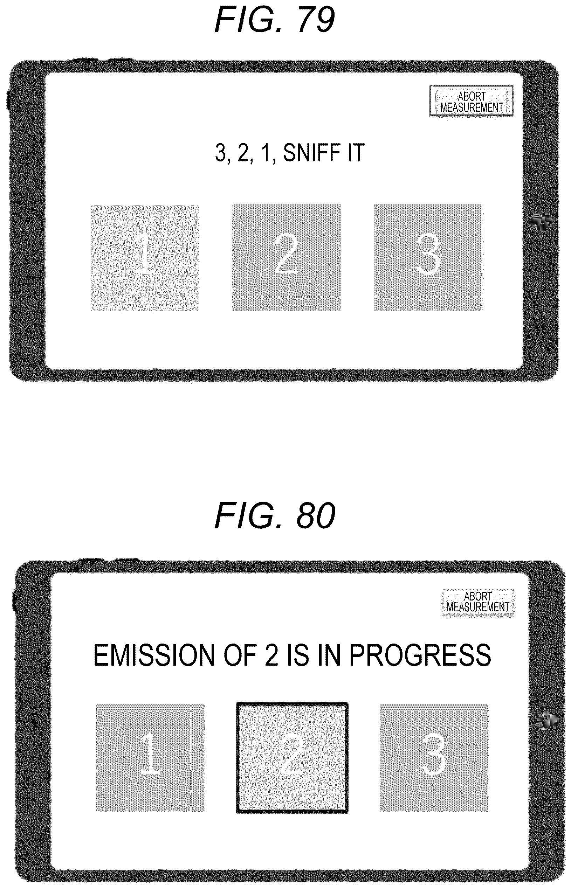

The second emission of odor component-containing air and the third emission of odor component-containing air are also performed in a manner similar to the above. That is, after the end of the first emission of odor component-containing air, the three-second countdown starts. In order to make the countdown more eye-catching to the subject, as illustrated in Fig. 78, a clear message or voice guide indicating that the subject has to wait for three seconds, like "Emit 2 after three seconds", may be provided. Next, the odor component-containing air is emitted. Here, as illustrated in Fig. 79, a message or voice guide like "3, 2, 1, sniff it." may be provided. Fig. 80 is a screen displayed while the odor component-containing air is emitted for a certain period of time. During emission, for example, the number for which emission is in progress may be enclosed by a blue frame and highlighted for the sake of easy recognition.

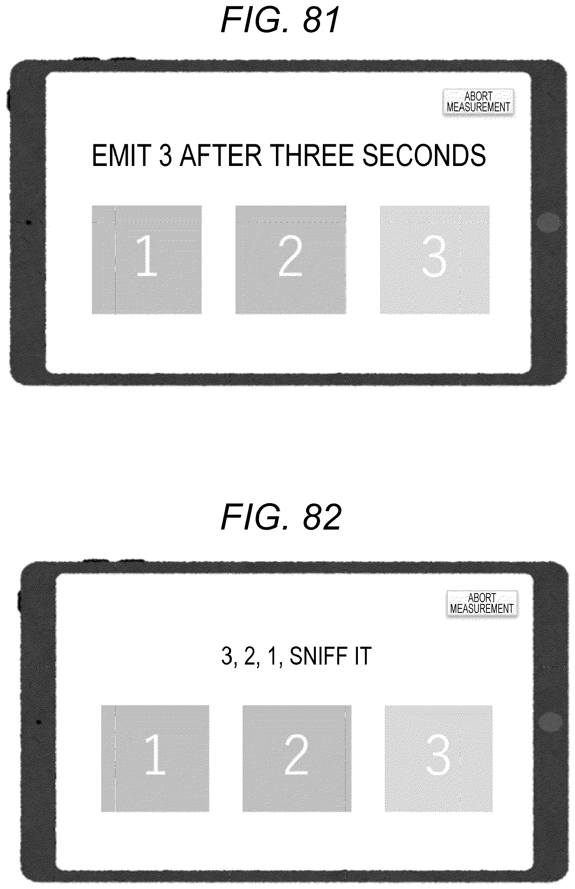

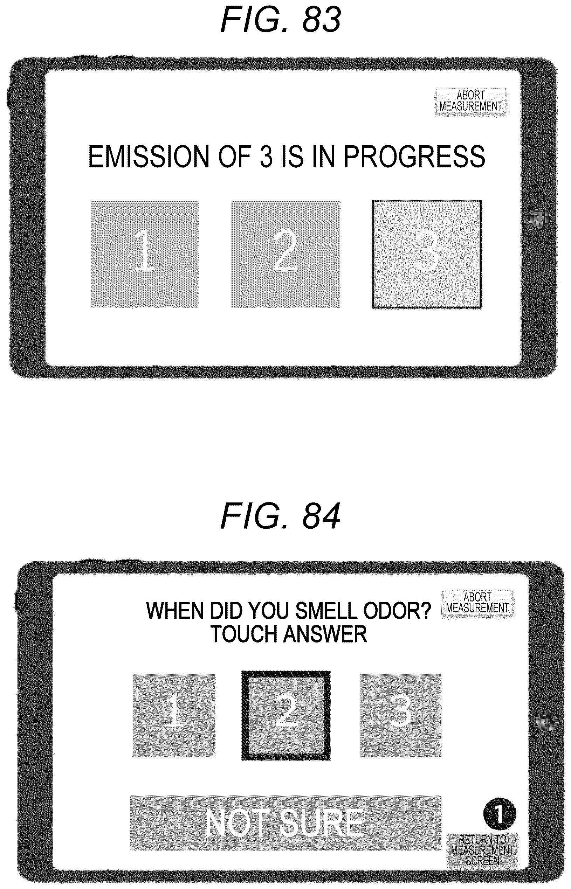

That is, after the end of the second emission of odor component-containing air, the three-second countdown starts. In order to make the countdown more eye-catching to the subject, as illustrated in Fig. 81, a clear message or voice guide indicating that the subject has to wait for three seconds, like "Emit 3 after three seconds", may be provided. Next, the odor component-containing air is emitted. Here, as illustrated in Fig. 82, a message or voice guide like "3, 2, 1, sniff it." may be provided. Fig. 83 is a screen displayed while the odor component-containing air is emitted for a certain period of time. During emission, for example, the number may be enclosed by a blue frame and highlighted for the sake of easy recognition.

Finally, Fig. 84 is shown to the subject to cause the subject to tap his/her answer as to when the subject smells the odor. As options, there are four options of [numbers 1 to 3] and [not sure]. Furthermore, tapping "1" in Fig. 84 automatically inputs the measurement result of the selected odor, the measurement result indicating whether the answer result is correct or incorrect. Here, the measurer may verbally ask the subject a question. Furthermore, the measurer himself/herself may tap the answer.

### (6-3) Behavior when odor presentation button is tapped

Hereinafter, behavior when the odor presentation button is tapped in the three-option detection threshold operation will be described. The measurer, the subject, an application screen display (hereinafter, simply referred to as "app screen"), an application logic (hereinafter, simply referred to as "app logic"), and the odor generation device 1 are correlated with each other for the behavior.

First, the measurer presses the odor presentation button. Then, the app logic causes a random function to produce a value from 1 to 3, and assigns the value to exam_number. Furthermore, an odor ID (smell_id) and an odor output time (smell_time) = set value are recognized, and odor imitation (that is, work to set smell_imitation False when exam_number is 1, and set smell_imitation True otherwise) and odor output start (that is, (emit_smell) = True is transmitted to the odor generation device 1) are performed. Upon receipt of True, the odor generation device 1 starts to output odor component-containing air. On the other hand, the app logic displays the selected odorant number on the app screen, highlights the selection frame, and disables the odor presentation button itself to make the odor presentation button blue, for example. Furthermore, at the same time, an odor cancel button is enabled.

The app logic then instructs the app screen to display a countdown. At this time, the app screen can be, for example, a screen as illustrated in Fig. 75. Furthermore, the odor generation device 1 rotates a motor simultaneously with the display of the countdown and starts the countdown. Then, after a lapse of a certain period of time, the app logic instructs the app screen to display a state where the emission of the odor component-containing air is in progress. At this time, the app screen can be, for example, a screen as illustrated in Fig. 77. Furthermore, the odor generation device 1 emits the odor component-containing air simultaneously with the display of the state where the emission is in progress. At this time, the subject takes action to sniff the emitted odor component-containing air. Then, further, after a lapse of a certain period of time, the app logic instructs the app screen to display the end of the emission of the odor component-containing air. Furthermore, at the same time, the odor generation device 1 finishes the emission of the odor component-containing air.

Next, in the app logic, the odor ID (smell_id) and the odor output time (smell_time) = set value are recognized, and the odor imitation (that is, work to set smell_imitation False when exam_number is 2, and set smell_imitation True otherwise) and the odor output start (that is, (emit_smell) = True is transmitted to the odor generation device 1) are performed. Upon receipt of True, the odor generation device 1 starts to output odor component-containing air. The app logic then instructs the app screen to display a countdown. At this time, the app screen can be, for example, a screen as illustrated in Fig. 78. Furthermore, the odor generation device 1 rotates a motor simultaneously with the display of the countdown and starts the countdown. Then, after a lapse of a certain period of time, the app logic instructs the app screen to display a state where the emission of the odor component-containing air is in progress. At this time, the app screen can be, for example, a screen as illustrated in Fig. 80. Furthermore, the odor generation device 1 emits the odor component-containing air simultaneously with the display of the state where the emission is in progress. At this time, the subject takes action to sniff the emitted odor component-containing air. Then, further, after a lapse of a certain period of time, the app logic instructs the app screen to display the end of the emission of the odor component-containing air. Furthermore, at the same time, the odor generation device 1 finishes the emission of the odor component-containing air.

Next, in the app logic, an odor ID (smell_id) and an odor output time (smell_time) = set value are recognized, and odor imitation (that is, work to set smell_imitation False when exam_number is 3, and set smell_imitation True otherwise) and odor output start (that is, (emit_smell) = True is transmitted to the odor generation device 1) are performed. Upon receipt of True, the odor generation device 1 starts to output odor component-containing air. The app logic then instructs the app screen to display a countdown. At this time, the app screen can be, for example, a screen as illustrated in Fig. 81. Furthermore, the odor generation device 1 rotates a motor simultaneously with the display of the countdown and starts the countdown. Then, after a lapse of a certain period of time, the app logic instructs the app screen to display a state where the emission of the odor component-containing air is in progress. At this time, the app screen can be, for example, a screen as illustrated in Fig. 83. Furthermore, the odor generation device 1 emits the odor component-containing air simultaneously with the display of the state where the emission is in progress. At this time, the subject takes action to sniff the emitted odor component-containing air. Then, further, after a lapse of a certain period of time, the app logic instructs the app screen to display the end of the emission of the odor component-containing air. Furthermore, at the same time, the odor generation device 1 finishes the emission of the odor component-containing air.

Next, the app logic updates the app screen as needed and displays an answer input screen as illustrated in Fig. 84. After displaying this screen, the app logic again enables the odor presentation button to be tapped. Next, when the subject inputs the number from which the subject smells an odor, the app logic enables an answer confirmation button, and the confirm answer is displayed on the app screen. Next, the measurer causes the app logic to record the measurement result by tapping the displayed confirm answer.

As to the handling of the result of the three-option detection measurement, a case where the number answered by the subject is the same as the random number from 1 to 3 (exam_number) is determined to be a correct answer. Upon receipt of the correct/incorrect answer, the measurement result of the odorant is updated. This operation is performed only for detection measurement results. Specific operations are described in the following Tables 1 and 2. Table 1 below shows result symbols, and Table 2 below shows the operation on the measurement result after the three-option detection.

**[Table 1]**

| SYMBOL | MEANING |
|---|---|
| ○ | DETECTED |
| × | RECOGNIZED |
| ○× | DETECTED AND RECOGNIZED |
| . | TEST WAS CONDUCTED, BUT NEITHER DETECTED NOR RECOGNIZED |
| | NO TEST IS REPRESENTED BY SPACE |

**[Table 2]**

| TEST RESULT | VALUE BEFORE TEST | VALUE AFTER TEST |
|---|---|---|
| CORRECT | ○× | ○× |
| | ○ | ○ |
| | × | ○× |
| | . | ○ |
| | | ○ |
| INCORRECT | ○× | × |
| | ○ | · |
| | × | × |
| | · | · |
| | | . |

The following shows behavior when the cancel button is tapped by the measurer. First, the app logic instructs the odor generation device 1 to stop the emission of the odor component-containing air. Upon receipt of the instruction, the odor generation device 1 stops the output and sets (emit_smell) False. Next, the app logic confirms whether output is possible (smell_emittable = True), and the odor generation device 1 makes a notification of the odor (smell_emittable). Here, smell_emittable = True may be confirmed by polling or the like. Then, the app logic notifies the app screen that the measurement has been successfully canceled. Next, the app logic returns the app screen to the odorant selection state without inputting a measurement result.

### (7) About olfactory term table

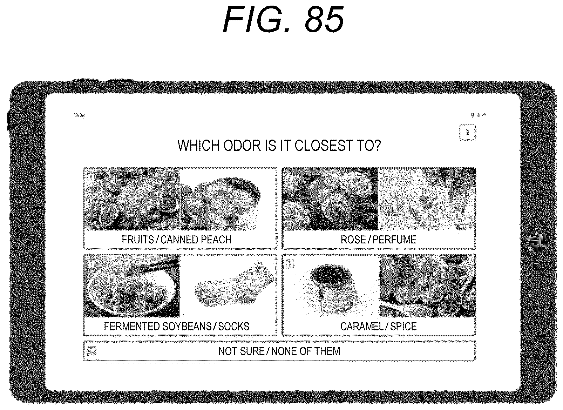

Here, a supplementary description will be given of the olfactory term table display. A combination of four types of materials determined in accordance with the odor types (A to E) and the odor concentrations (-2 to 5) is referred to as olfactory term table. The four types of materials are arranged at random. For example, in an olfactory term table illustrated in Fig. 85, rose/perfume is placed in the upper right, but is placed at random for each measurement such as the lower right, the upper left, and the lower left. The olfactory term table can be displayed as, for example, photographic images corresponding to the odorants A to E, specifically, A: rose/perfume, B: caramel/spice, C: putrid odor/stuffy socks, D: fruits/canned peach, and E: fecal odor/insect repellent. Furthermore, in the present embodiment, X: onion/garlic, Y: tatami mat/timber, and Z: popcorn/butter were prepared as a dummy term table although their images are not present in the odorant types.

Furthermore, in the olfactory term table, four options corresponding to the following Table 3 are displayed for each odorant. Their respective materials have the following combinations.

**[Table 3]**

| | **A** | **B** | **C** | **D** | **E** |
|---|---|---|---|---|---|
| **- 2** | A,B,C,D | B,C,D,E | C,D,E,X | D,E,X,Y | E,X,Y,Z |
| **- 1** | A,C,D,E | B,D,E,X | C,E,X,Y | D,X,Y,Z | E,Y,Z,A |
| **0** | A,D,E,X | B,E,X,Y | C,X,Y,Z | D,Y,Z,A | E,Z,A,B |
| **1** | A,E,X,Y | B,X,Y,Z | C,Y,Z,A | D,Z,A,B | E,A,B,C |
| **2** | A,X,Y,Z | B,Y,Z,A | C,Z,A,B | D,A,B,C | E,B,C,D |
| **3** | A,Y,Z,B | B,ZAC | C,A,B,D | D,B,C,E | E,C,D,X |
| **4** | A.Z.B.C | B,A,C,D | C,B,D,E | D,C,E,X | E,D,X,Y |
| **5** | A,B,C,D | - | C,D,E,X | D,E,X,Y | E,X,Y,Z |

As to the handling of the result of the olfactory term table, a case where the subject can select a word selected by the measurer and corresponding to the emitted odor component-containing air is determined to be a correct answer. Upon receipt of a correct/incorrect answer, the result of the odor is updated. This operation is performed only for recognition results. Specific operations are described in the following Table 4. The result symbols are similar to those in Table 1 described above.

**[Table 4]**

| TEST RESULT | VALUE BEFORE TEST | VALUE AFTER TEST |
|---|---|---|
| CORRECT | ○× | ○× |
| | ○ | ○× |
| | × | × |
| | - | × |
| | | × |
| INCORRECT | ○× | ○ |
| | ○ | ○ |
| | × | - |
| | - | - |
| | | - |

### (8) Example of discrimination test measurement flow

Hereinafter, an example of the discrimination test measurement flow will be described with reference to the drawings.

### (8-1) Overview of discrimination test measurement

A rough description of the discrimination test measurement flow is as follows.

First, the measurer (tester) opens the free measurement screen described above and taps a discrimination measurement button. Next, when the odorant is selected, the odor presentation button is automatically activated three times. Accordingly, the subject sniffs an odor three times. Then, the subject gives a verbal answer as to when the subject smells a different odor. The measurer inputs the answer result to the tablet terminal 301. Basically, the tablet terminal 301 is viewed only by the measurer, but may be temporarily shown to the subject.

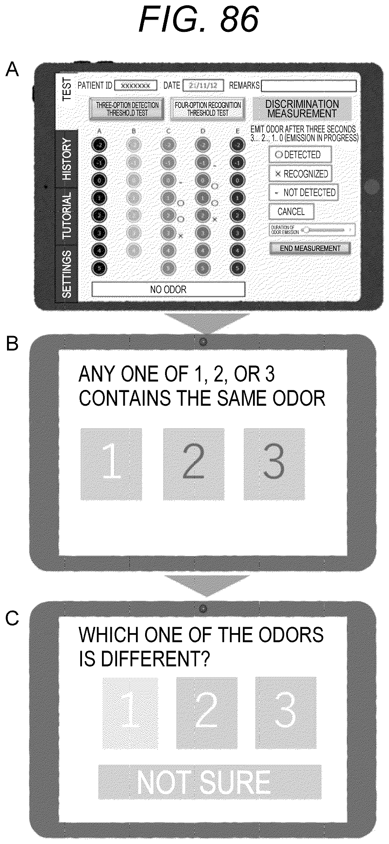

Specifically, by default, only a total of 23 types of tests including three types of A, B, and D and seven or eight concentrations are supported. Furthermore, C and E can be activated as necessary in accordance with app settings. First, as illustrated in Fig. 86A, the measurer selects a target odorant (for example, A3) and taps the discrimination measurement button. Here, the subject is informed by a message or voice guide like "any one of 1, 2, or 3 contains the same odor". Then, the target odorant × 1 time and an incorrect odorant × 2 times are emitted at random, specifically, 1: the target odorant, and 2 and 3: the incorrect odorant (predetermined odorant pair). That is, the incorrect odorant with the same intensity level is determined at random (for example, from among B3 to E3). The subject is asked to smell an odor a total of three times (target odorant × 1 time and incorrect odorant × 2 times) and answer a different odor (target odorant) as illustrated in Fig. 86C. In this case, selecting "1" is a correct answer.

### (8-2) Discrimination test measurement screen

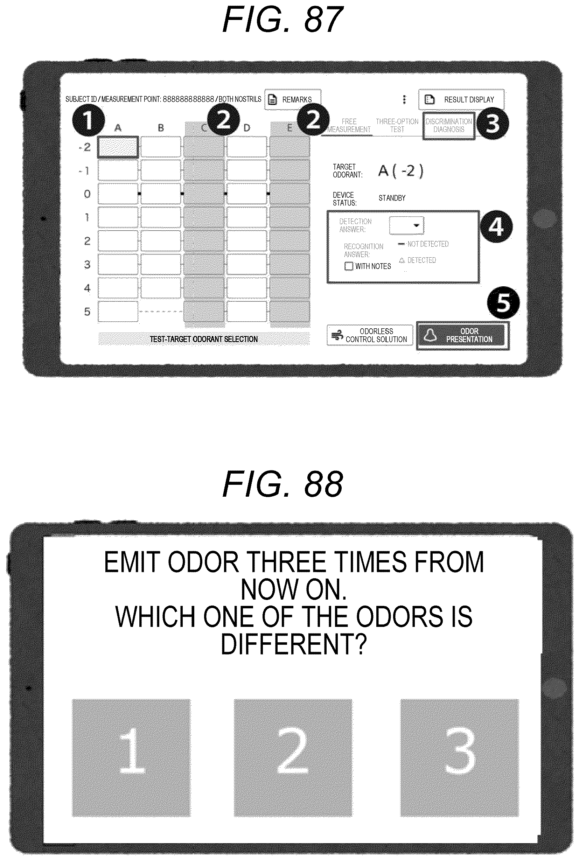

Figs. 87 to 96 are diagrams each illustrating an example of the embodiment of the discrimination test measurement screen. The overview of the discrimination test measurement has been described above, but the discrimination test measurement will be described below in more detail with reference to each screen. Note that the following description is on the assumption that the tablet terminal 301 is also shown to the subject.

First, the measurer gives, to the subject, a verbal description of a procedure of the three-option detection measurement. Here, instead of the verbal description, the procedure may be described using a diagram or using a video. Next, as illustrated in "1" in Fig. 87, the measurer selects one type of target odorant. In "2" in Fig. 87, the odors C and E are inactive. In "3" in Fig. 87, the discrimination measurement is selected. In order to make the selection eye-catching, the discrimination measurement may be highlighted. In "4" in Fig. 87, a discrimination answer can be input, and, for example, either "Δ: discriminated" or "-: not discriminated" is input. Furthermore, the tablet terminal 301 is shown to the subject, and as illustrated in Fig. 88, a message or voice guide like "Emit odor three times from now on. Which one of the odors is different?" is given to inform the subject that the measurement is about to start.

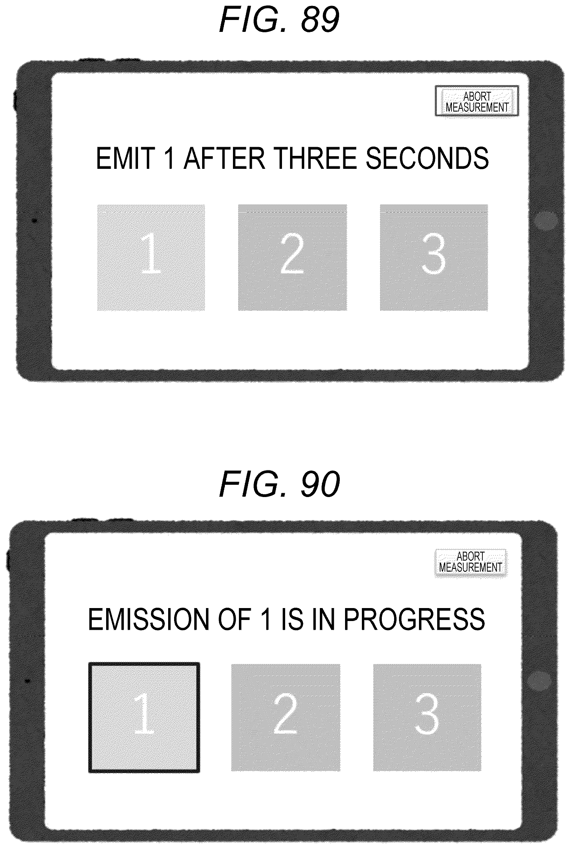

Next, the measurer taps the odor presentation button (start). Then, the three-second count starts. In order to make the countdown more eye-catching to the subject, as illustrated in Fig. 89, a clear message or voice guide indicating that the subject has to wait for three seconds, like "Emit 1 after three seconds", may be provided. Next, the odor component-containing air is emitted. Here, as illustrated in Fig. 90, a message or voice guide like "Emission of 1 is in progress" may be provided. Fig. 90 is a screen displayed while the odor component-containing air is emitted for a certain period of time. During emission, for example, the number for which emission is in progress may be enclosed by a blue frame and highlighted for the sake of easy recognition.

Note that it is also possible to stop the emission of the odor component-containing air and return to the odorant selection screen by tapping a measurement abort button located at the upper right.

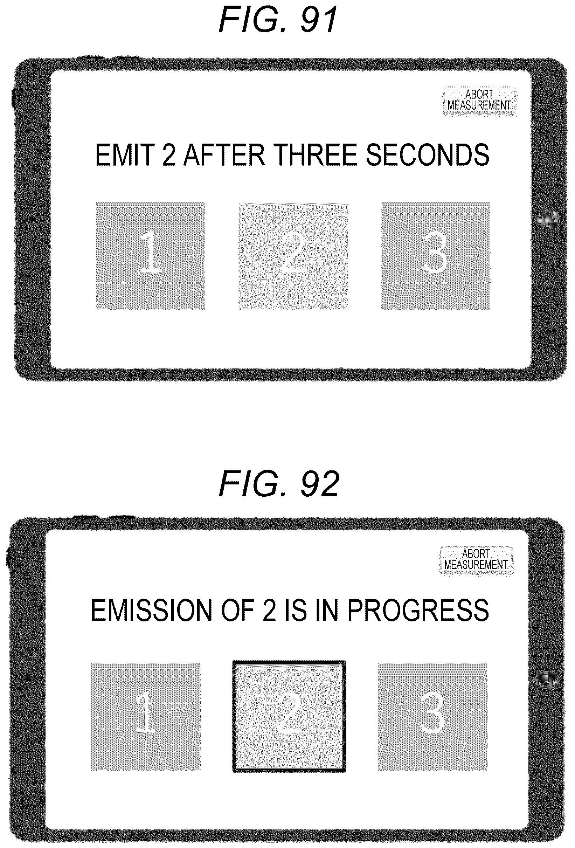

The second emission of odor component-containing air and the third emission of odor component-containing air are also performed in a manner similar to the above. That is, after the end of the first emission of odor component-containing air, the three-second countdown starts. In order to make the countdown more eye-catching to the subject, as illustrated in Fig. 91, a clear message or voice guide indicating that the subject has to wait for three seconds, like "Emit 2 after three seconds", may be provided. Next, the odor component-containing air is emitted. Fig. 92 is a screen displayed while the odor component-containing air is emitted for a certain period of time. During emission, for example, the number for which emission is in progress may be enclosed by a blue frame and highlighted for the sake of easy recognition.

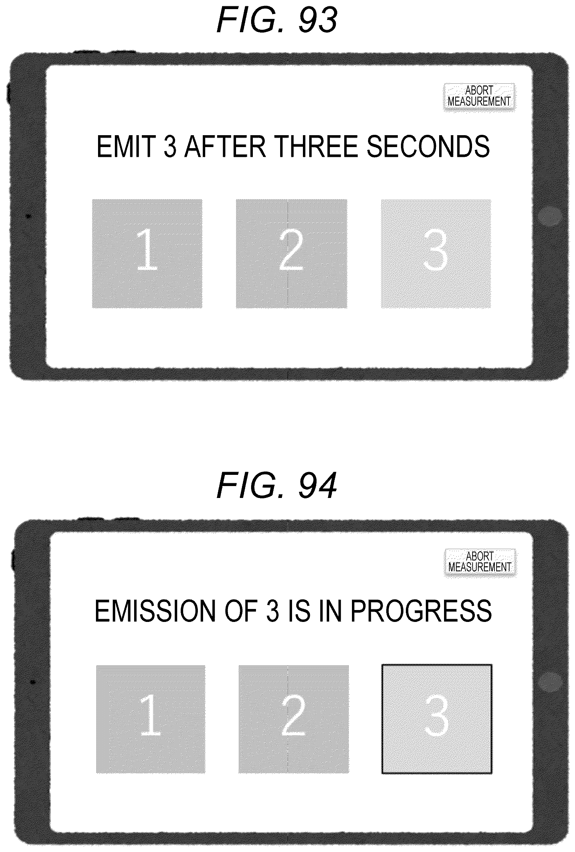

That is, after the end of the second emission of odor component-containing air, the three-second countdown starts. In order to make the countdown more eye-catching to the subject, as illustrated in Fig. 93, a clear message or voice guide indicating that the subject has to wait for three seconds, like "Emit 3 after three seconds", may be provided. Next, the odor component-containing air is emitted. Fig. 94 is a screen displayed while the odor component-containing air is emitted for a certain period of time. During emission, for example, the number for which emission is in progress may be enclosed by a blue frame and highlighted for the sake of easy recognition.

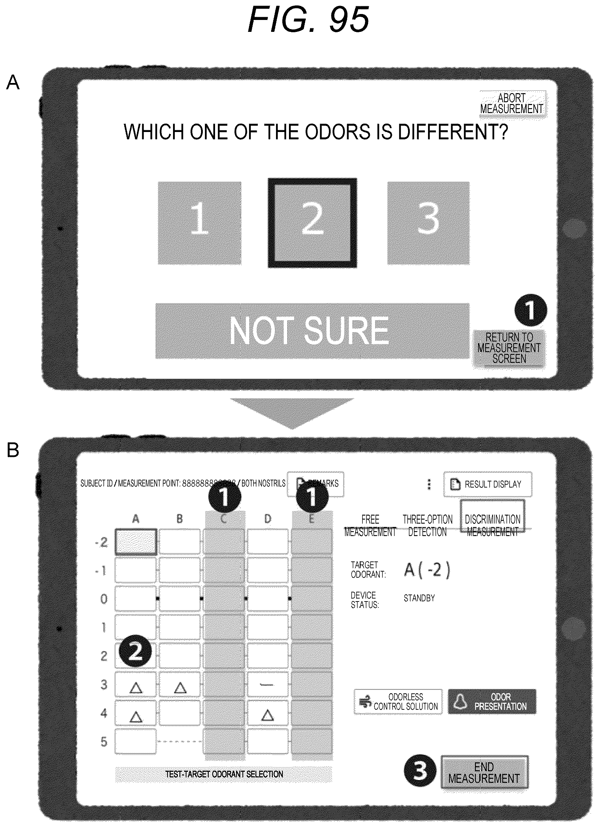

Finally, Fig. 95A is shown to the subject to cause the subject to tap his/her answer to a question "Which one of the odors is different ?". As options, there are four options of [numbers 1 to 3] and [not sure]. Furthermore, "1" in Fig. 95A becomes active when any one of the options is tapped, and the measurement result of the selected odor, indicating whether the answer value is correct or incorrect, is reflected in a table different from the table for detection and recognition. In "1" in Fig. 95B, the odors C and E are inactive by default. Note that, from the settings, when C and E are ON, C and E become active with C and E not grayed out. The result display indicated in "2" in Fig. 95B can be, for example, "Δ: discriminated", "-: not discriminated", or "(space): not yet measured". When the measurement end button in "3" in Fig. 95B is tapped, the result is saved.

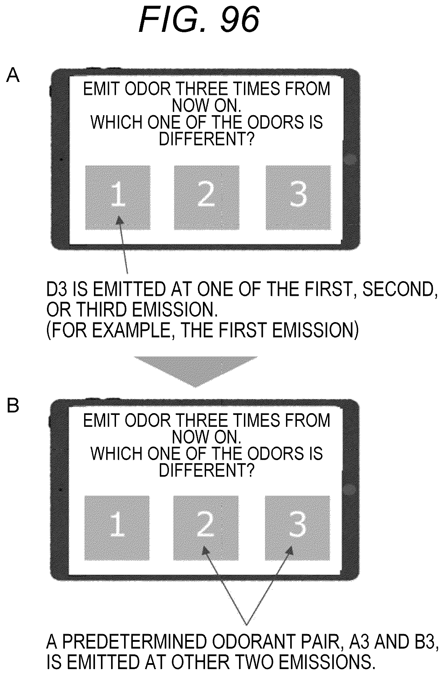

For example, in the description of the measurement flow in a case where the target odorant is D3 and the discrimination test measurement is pressed, the target odorant (D3) is emitted at any one of the first to third emissions as illustrated in Fig. 96A. On the other hand, as illustrated in Fig. 96B, at the other two emissions, a predetermined odorant pair such as A3 and B3 is emitted. Note that, in general, a bad odor is not continuously presented during the discrimination test measurement, so that an odor is selected from among A, B, and D. In a case where it is desired to make C and E subject to the discrimination test measurement, it is also possible to add C and E by changing the settings.

### (9) Odor generation device 1

Although the odor generation device 1 is as described above, the odor generation device 1 having the following configuration may be used in the present embodiment.

The odor generation device 1 according to the present embodiment illustrated in a part of Fig. 99 includes a cartridge 10, a cartridge holding portion 21, a front storage portion 22, and a back storage portion 23. Furthermore, although not illustrated, a user interface unit, a storage unit, an output unit, and the like may be included as necessary.

Hereinafter, each component will be described in detail.

### (9-1) Cartridge 10

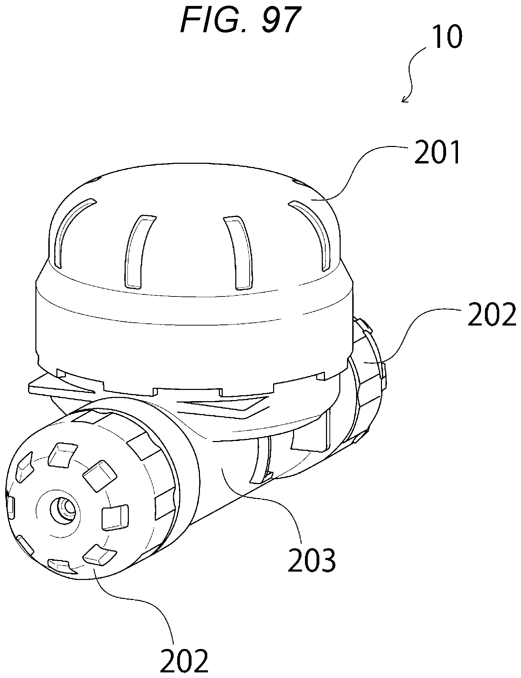

Fig. 97 is a perspective view of an embodiment of the cartridge 10. The cartridge 10 includes at least an odor holding portion 201, a ventilation portion 202, and a connection portion 203.

The odor holding portion 201 includes at least an impregnant 204 and a container portion. Furthermore, a lid portion is joined to a part of the connection portion 203 to be described later.

The impregnant 204 is stored in the container portion, and a material forming the impregnant 204 is not particularly limited as long as it can hold an odor component. An odor component held by the impregnant 204 is not particularly limited. For example, one or a combination of at least two of an odor component such as liquid fragrance or a powder fragrance as it is, an odor component obtained by dissolving or dispersing the liquid fragrance or the powder fragrance in an appropriate solvent, an essential oil, an essential oil diluted with an appropriate solvent, fruit juice, a beverage, a food, and an odor component obtained by dissolving or dispersing the food in an appropriate solvent can be freely used. Furthermore, the odor component held by the impregnant 204 is not particularly limited.

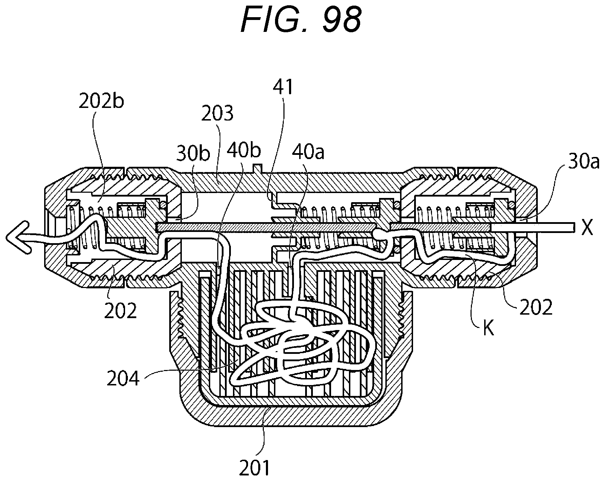

The ventilation portion 202 has at least an openable and closable ventilation opening 30. In the present embodiment, as illustrated in Fig. 98, the ventilation portion 202 is divided into two sections (a first ventilation portion 202a and a second ventilation portion 202b) by two ventilation openings 30 (30a and 30b). The ventilation opening 30 includes an inflow ventilation opening 30a for allowing air to flow into the cartridge 10 and a release ventilation opening 30b for releasing odor component-containing air.

Furthermore, an opening and closing mechanism can be connected to such ventilation openings 30 (30a and 30b). A specific structure of the opening and closing mechanism is not particularly limited as long as the ventilation openings 30 (30a and 30b) can be opened and closed, and can be freely designed. Specifically, for example, an opening and closing mechanism including an airtight lid, a shaft, and a spring may be provided.

The connection portion 203 includes at least two connection openings 40 (40a and 40b) that are formed in the lid portion joined to the connection portion 203 and allow the ventilation portion 202 and the odor holding portion 201 to communicate with each other, and a partition portion 41 disposed upstream of one of the at least two connection openings 40 (40a and 40b).

In the present embodiment, the connection portion 203 may have an opening and closing mechanism in a region where the connection portion 203 is contiguous with the ventilation portion 202. Specifically, for example, an opening and closing mechanism including an airtight lid, a shaft, and a spring may be provided.

Fig. 98 is a cross-sectional view of the cartridge 10 of the present embodiment, conceptually illustrating a state where odor component-containing air is released from the cartridge 10. In Fig. 98, K represents the flow of air. In the present embodiment, when the ventilation portion 202 is opened by causing a pusher X to press a part of the opening and closing lid from the hole of one of the ventilation portions 202 with an odor component held in the odor holding portion 201, air flows into the cartridge 10 from the outside. The air that has flowed in flows into the connection portion 203 through the ventilation portion 202, and the air that has flowed into the connecting portion 203 first flows into the odor holding portion 201, due to the partition portion 41, through the connection opening 40a through which the connection portion 203 and the odor holding portion 201 communicate with each other, and is mixed with the odor component held by the impregnant 204 to generate odor component-containing air. In this state, the odor component-containing air flows into the connection portion 203 again through the connection opening 40b through which the connection portion 203 and the odor holding portion 201 communicate with each other. Thereafter, the odor component-containing air flows into the other ventilation portion 202 through the connection portion 203, and is then released to the outside from the hole of the other ventilation portion 202.

### (9-2) Cartridge holding portion 21

The cartridge holding portion 21 holds one or more cartridges 10. Specifically, as illustrated in a part of Fig. 99, the cartridge 10 includes a retaining portion 211 that retains one or more cartridges 10 and has an emission hole 210 through which the odor component-containing air emitted from the cartridge 10 is released, and a holding portion 212 that fits with the retaining portion 211 to hold the cartridge 10. The number of the cartridges 10 to be held is not particularly limited.

The form of the retaining portion 211 and/or the holding portion 212 is not particularly limited, and can be freely designed according to the form of the cartridge 10 to be held or the like. For example, it can be formed in a roughly disk shape, a roughly rectangular parallelepiped shape, a roughly columnar shape, a roughly cubic shape, or the like.

The material forming the retaining portion 211 and/or the holding portion 212 is not particularly limited, but the use of recycled plastic or the like can contribute to the SDGs. Furthermore, forming the retaining portion 211 and the holding portion 212 separately allows only the holding portion 212 that holds the cartridge 10 to be disposed of as medical waste and allows the other retaining portion 211, the front storage portion 22, the back storage portion 23, and the like to be reused without being disposed of.

As described above, the holding portion 212 holding the plurality of cartridges 10 in advance may be handled as the cartridge unit 20. In this case, the retaining portion 211 may be provided with the emission hole 210 through which the odor component-containing air is released in a case where the holding portion 212 itself is handled as the cartridge unit 20.

### (9-3) Front storage portion 22

The front storage portion 22 includes at least a releasing portion 221 that releases the odor component-containing air to the outside. For example, as illustrated in a part of Fig. 99, the releasing portion 221 may be provided in the front storage portion 22, and in this case, may communicate with the emission hole 210 provided in the retaining portion 211.

In the present embodiment, the releasing portion 221 may include a guide portion (not illustrated) that guides an air current containing the odor component toward the nose of the user. The material forming the guide portion is not particularly limited, and examples of the material include paper (including recycled paper), wood, a bamboo sheath, plastic, and coal. The guide portion may be formed in a partially or entirely detachable form, and in this case, for example, the guide portion may be disposable for each user.

### (9-4) Back storage portion 23

As illustrated in a part of Fig. 99, the back storage portion 23 may be provided with a fitting portion 230 that fits with the holding portion 212 described above. The number of the fitting portions 230 is not particularly limited. The fitting portion 230 is preferably configured to be able to withstand, when the plurality of cartridges 10 is attached at a time, a load applied in a direction in which the cartridges 10 are detached or attached.

The back storage portion 23 includes at least a drive mechanism portion 151 and a positioning drive portion 152. The drive mechanism portion 151 and the positioning drive portion 152 are similar to those described above, so that the description thereof will be omitted here.

Note that the present technology can also adopt the following configurations.
[1] An information processing device including a processing unit that identifies information regarding an odor component to be released and controls release of the odor component on the basis of information associated with a user.
[2] The information processing device according to [1], in which the information regarding the odor component to be released includes any one or more pieces of information selected from the group consisting of a type of the odor component, a mixing ratio of the odor component, a concentration of the odor component, a release time of the odor component, and a time until start of the release of the odor component.
[3] The information processing device according to [1] or [2], in which the processing unit further identifies a concentration of the odor component to be released or a release time of the odor component on the basis of the information regarding the odor component identified and the information associated with the user.
[4] The information processing device according to [3], in which the information associated with the user includes any one or more pieces of information selected from the group consisting of biological information regarding the user, physical information regarding the user, and information regarding an answer from the user.
[5] The information processing device according to [4], in which the information regarding the answer from the user includes a result of any one or more tests selected from the group consisting of an identification test, a threshold test, and a discrimination test.
[6] The information processing device according to [5], in which the processing unit issue an instruction to output the result of the test.
[7] The information processing device according to [5] or [6], in which the result of the threshold test includes at least one result selected from the group consisting of a detection threshold, a recognition threshold, and a difference between the detection threshold and the recognition threshold.
[8] The information processing device according to any one of [4] to [7], in which the processing unit adjusts a concentration of the odor component to be released on the basis of the information regarding the answer from the user.
[9] The information processing device according to any one of [1] to [8], in which the processing unit controls the release of the odor component on the basis of environmental information that affects the release of the odor component.
[10] The information processing device according to any one of [1] to [9], further including a storage unit that stores the information associated with the user and/or the information regarding the odor component identified.
[11] The information processing device according to any one of [1] to [10], in which the processing unit determines a physical function of the user on the basis of the information associated with the user displayed in time series.
[12] An information processing system including:
   an information processing device including a processing unit that identifies information regarding an odor component to be released and controls release of the odor component on the basis of information associated with a user; and
   a display device that displays the information regarding the odor component to be released.
[13] The information processing system according to [12], in which the information regarding the odor component to be released includes a time until start of the release of the odor component.
[14] The information processing system according to [12] or [13], in which the display device displays the information associated with the user in time series.
[15] The information processing system according to any one of [12] to [14], in which the processing unit determines a physical function of the user on the basis of the information associated with the user displayed in time series.
[16] An odor generation system including:
   an odor generation device including
   a cartridge holding portion that holds a cartridge holding an odor component, and
   a releasing portion that releases the odor component to outside; and
   an information processing device including a processing unit that identifies information regarding an odor component to be released and controls release of the odor component on the basis of information associated with a user.
[17] The odor generation system according to [16], further including
   a user interface unit that displays any one or more pieces of information selected from the group consisting of the information associated with the user, the information regarding the odor component to be released, information regarding the cartridge, and information regarding measurement using the odor component.
[18] A program for executing a processing function, the processing function including identifying information regarding an odor component to be released and controlling release of the odor component on the basis of information associated with a user.

### REFERENCE SIGNS LIST

1 Odor generation device
10 Cartridge
100 Odor generation system
11, 21 Cartridge holding portion
12 Movable portion
14, 22 Front storage portion
140 Releasing portion
142 Communication hole
15, 23 Back storage portion
150 Fitting portion
151 Drive mechanism portion
152 Positioning drive portion
2 Display device
20 Cartridge unit
200 Information processing system
201 Odor holding portion
202 Ventilation portion
203 Connection portion
204 Impregnant
205 Retaining portion
2050 Hole
2052 Fitted portion
2053 Emission hole
210 Emission hole
211 Retaining portion
212 Holding portion
221 Releasing portion
230 Fitting portion
3 Information processing device
31 Processing unit
32 Storage unit
33 User interface unit
301 Tablet terminal
302 Display module
303 Button module
331 Identification portion
332 Reader
34 Output unit

## Claims

1. An information processing device comprising a processing unit that identifies information regarding an odor component to be released and controls release of the odor component on a basis of information associated with a user.

2. The information processing device according to claim 1, wherein the information regarding the odor component to be released includes any one or more pieces of information selected from the group consisting of a type of the odor component, a mixing ratio of the odor component, a concentration of the odor component, a release time of the odor component, and a time until start of the release of the odor component.

3. The information processing device according to claim 1, wherein the processing unit further identifies a concentration of the odor component to be released or a release time of the odor component on a basis of the information regarding the odor component identified and the information associated with the user.

4. The information processing device according to claim 3, wherein the information associated with the user includes any one or more pieces of information selected from the group consisting of biological information regarding the user, physical information regarding the user, and information regarding an answer from the user.

5. The information processing device according to claim 4, wherein the information regarding the answer from the user includes a result of any one or more tests selected from the group consisting of an identification test, a threshold test, and a discrimination test.

6. The information processing device according to claim 5, wherein the processing unit issue an instruction to output the result of the test.

7. The information processing device according to claim 5, wherein the result of the threshold test includes any one or more results selected from the group consisting of a detection threshold, a recognition threshold, and a difference between the detection threshold and the recognition threshold.

8. The information processing device according to claim 4, wherein the processing unit adjusts a concentration of the odor component to be released on a basis of the information regarding the answer from the user.

9. The information processing device according to claim 1, wherein the processing unit controls the release of the odor component on a basis of environmental information that affects the release of the odor component.

10. The information processing device according to claim 1, further comprising a storage unit that stores the information associated with the user and/or the information regarding the odor component identified.

11. The information processing device according to claim 1, wherein the processing unit determines a physical function of the user on a basis of the information associated with the user displayed in time series.

12. An information processing system comprising:
an information processing device including a processing unit that identifies information regarding an odor component to be released and controls release of the odor component on a basis of information associated with a user; and
a display device that displays the information regarding the odor component to be released.

13. The information processing system according to claim 12, wherein the information regarding the odor component to be released includes a time until start of the release of the odor component.

14. The information processing system according to claim 12, wherein the display device displays the information associated with the user in time series.

15. The information processing system according to claim 14, wherein the processing unit determines a physical function of the user on a basis of the information associated with the user displayed in time series.

16. An odor generation system comprising:
an odor generation device including
a cartridge holding portion that holds a cartridge holding an odor component, and
a releasing portion that releases the odor component to outside; and
an information processing device including a processing unit that identifies information regarding an odor component to be released and controls release of the odor component on a basis of information associated with a user.

17. The odor generation system according to claim 16, further comprising
a user interface unit that displays any one or more pieces of information selected from the group consisting of the information associated with the user, the information regarding the odor component to be released, information regarding the cartridge, and information regarding measurement using the odor component.

18. A program for executing a processing function, the processing function comprising identifying information regarding an odor component to be released and controlling release of the odor component on a basis of information associated with a user.
